(19) Europäisches Patentamt
European Patent Office
Office européen des brevets

(11) EP 3 569 255 A1

(12) **EUROPEAN PATENT APPLICATION**
published in accordance with Art. 153(4) EPC

(43) Date of publication:
**20.11.2019 Bulletin 2019/47**

(21) Application number: **18738938.2**

(22) Date of filing: **11.01.2018**

(51) Int Cl.:
***A61K 49/00*** *(2006.01)*

(86) International application number:
**PCT/JP2018/000532**

(87) International publication number:
**WO 2018/131663 (19.07.2018 Gazette 2018/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **11.01.2017 JP 2017002960**
**24.03.2017 JP 2017059301**

(71) Applicant: **Public University Corporation Yokohama City University**
**Yokohama-shi, Kanagawa 236-0027 (JP)**

(72) Inventors:
- **TAKAHASHI, Takuya**
  **Yokohama-shi**
  **Kanagawa 236-0004 (JP)**
- **MIYAZAKI, Tomoyuki**
  **Yokohama-shi**
  **Kanagawa 236-0004 (JP)**

(74) Representative: **Prüfer & Partner mbB**
**Patentanwälte · Rechtsanwälte**
**Sohnckestraße 12**
**81479 München (DE)**

(54) **IMAGING METHOD OF AMPA RECEPTORS IN BRAIN OF PRIMATE ORGANISM, PROGRAM, DIAGNOSTIC AGENT, COMPANION DIAGNOSTIC AGENT, DRUG, SCREENING METHOD, INPUT TERMINAL, SERVER AND SYSTEM**

(57) This imaging method of AMPA receptors in the brain of primate organisms involves a step in which a substance which is administered to the primate organism and which selectively bonds to AMPA receptors in the brain of the primate organism and has a radiolabel is transported into the brain and made to bond with AMPA receptors in the brain, and, by detecting radiation emitted from the substance bonded to the AMPA receptors in the brain, data is obtained relating to the distribution and/or expression level of the AMPA receptors in the brain.

FIG. 12

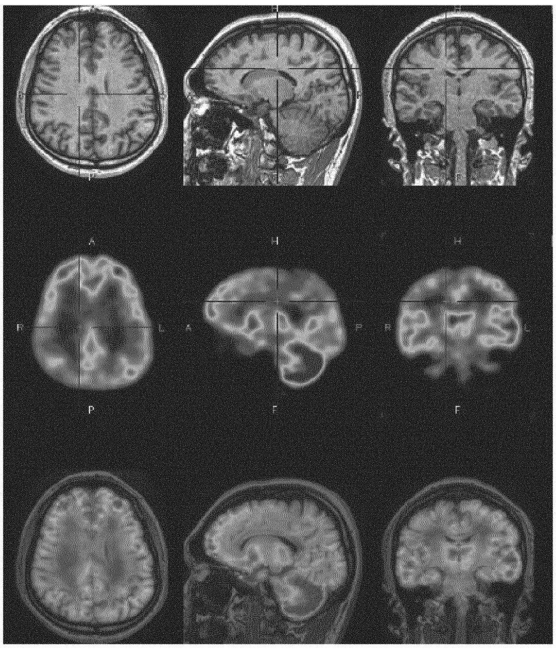

EP 3 569 255 A1

## Description

TECHNICAL FIELD

The present invention relates to a technology for imaging AMPA receptors in the brain of a primate organism.

BACKGROUND ART

It is known that AMPA receptors widely distribute in the central nervous system and involve in learning, memory, neurological degeneration, cell death, and the like. In recent years, researches related to treatment for psychiatric and neurological diseases using AMPA receptors as targets (Patent Documents 1 to 3). In order to examine the relation between the AMPA receptors and these diseases, it is required to evaluate the expression level and the distribution of AMPA receptors in the brain.

Conventionally, as technologies for analyzing AMPA receptors, the followings are known: microscopic observations on the level of synapses and spines (such as antibody staining using an electron microscope, a fluorescence observation using a two-photon microscope, a functional observation in synapses using an electrophysiological method, and a single molecule tracking method using a quantum dot method); and relatively macroscopic observations such as an immunostaining method using slices.

Among them, except in vivo imaging using a two-photon microscope, it is impossible to perform analysis in an organism. On the other hand, although in the in vivo imaging using a two-photon microscope, a microscopic observation on the level of spines and dendrites can be performed in an organism, it is impossible to perform an observation on the entire brain. Moreover, in the in vivo imaging method using a two-photon microscope, only fluorescent protein-tagged AMPA receptors can be observed, and endogenous AMPA receptors cannot be observed. The fluorescent protein-tagged AMPA receptors need to be artificially expressed by a gene transfer method, and thus in primates other than humans, a significant disadvantage is encountered in terms of cost, and moreover, it is practically impossible to perform this method on humans due to the problem of invasiveness.

Patent document 4 discloses an example where a substance having affinity to AMPA receptors is used to image AMPA receptors in the brain of a monkey organism. However, it is not actually confirmed that a probe-derived radiation detection value depending on the administered amount of unlabeled competitive substance is lowered, and it is only possible to detect radiation which is present in the brain at a certain time and is derived from a probe. In other words, it has not been demonstrated that probe-derived radiation bound to AMPA receptors is detected and that based on this detection, it is possible to actually image the AMPA receptors.

Patent Document 1: Japanese Unexamined Patent Application, Publication No. 2012-207021
Patent Document 2: Japanese Unexamined Patent Application, Publication No. 2010-202525
Patent Document 3: Japanese Unexamined Patent Application (Translation of PCT Application), Publication No. 2006-525292
Patent Document 4: Japanese Unexamined Patent Application (Translation of PCT Application), Publication No. 2016-522786

DISCLOSURE OF THE INVENTION

Problems to be Solved by the Invention

The present invention is made in view of the foregoing conditions, and an object thereof is to provide a technology for imaging AMPA receptors in the brain of a primate organism and applications thereof.

Means for Solving the Problems

(1) A method of imaging AMPA receptors in a brain of a primate organism, including: a step of delivering into the brain a substance which is administered to the primate organism, which is selectively bound to the AMPA receptors in the brain of the primate organism and is radio-labeled, binding the substance to the AMPA receptors in the brain and detecting radiation which is emitted from the substance bound to the AMPA receptors in the brain so as to acquire data on the distribution and/or the expression level of the AMPA receptors in the brain.

(2) The method described in (1), in which a required time is allowed after the delivery of the substance into the brain

such that the substance which is not bound to the AMPA receptors in the brain is urged to be discharged to the outside of the brain, and the detection is thereafter performed.

(3) The method described in (1) or (2), in which the detection is performed both when a first time elapses after the delivery of the substance into the brain and when a second time longer than the first time elapses, and based on individual detection values, the data on the distribution and/or the level of the AMPA receptors in the brain is acquired.

(4) The method described in any one of (1) to (3), in which the substance comprises a compound represented by Formula (I) or a pharmaceutically acceptable salt or solvate thereof

[Chem. 1]

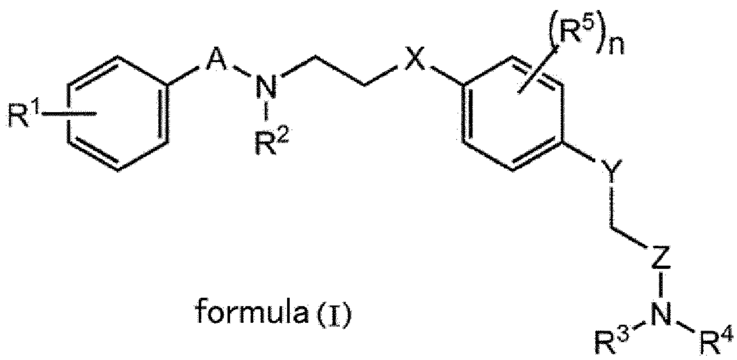

formula (I)

(in the formula,

each of A and Z independently represents CO, SO, or $SO_2$;
each of X and Y independently represents S or O;
each of $R^1$ to $R^4$ independently represents hydrogen, alkyl, alkenyl, alkynyl, or halo;
each $R^5$ independently represents alkyl, alkenyl, alkynyl, or halo;
n represents an integer of 0 to 4; and
one or more atoms are radioisotopes of the atoms).

(5) A program for instructing a computer to perform the method described in any one of (1) to (4).

(6) A diagnostic agent for a disease associated with AMPA receptors in a brain of a primate organism or a companion diagnostic agent for therapy or prophylaxis of the disease, in which a substance is selectively bound to the AMPA receptors in the brain of the primate organism and is radio-labeled is an active ingredient.

(7) A drug for therapy or prophylaxis of a disease associated with AMPA receptors in the brain of the primate organism, in which a substance which is selectively bound to the AMPA receptors in the brain of the primate organism is an active ingredient, and the drug is administered according to an administration plan based on the data obtained by the method described in any one of (1) to (4).

(8) The agent or the drug described in (6) or (7), in which the disease is a mental disease or a neurological disease.

(9) A method of screening a therapeutic or prophylactic agent for a disease associated with AMPA receptors in the brain of the primate organism, including:

a step of screening, before and after a candidate substance is administered to the primate organism, the candidate substance based on a difference in the data obtained by the method described in any one of (1) to (4).

(10) An input terminal which transmits, to a server, information on the distribution and/or the expression level of AMPA receptors in a brain of a primate organism.

(11) A server including: a database that stores data in which the distribution and/or the expression level of AMPA receptors in a brain of a primate organism is associated with the state of a disease associated with AMPA receptors in the brain of the primate organism; and

a means which compares information on the distribution and/or the expression level of AMPA receptors in a brain of an organism of a human subject that is input with the data so as to transmit information on the state of a disease of the human subject to an output terminal.

(12) A server including: a database that stores data in which the distribution and/or the expression level of AMPA receptors in a brain of a primate organism, the state of a disease associated with AMPA receptors in the brain of the primate organism, and the type, the dosage, and/or the usage of a drug that has already been administered and is intended for therapy or prophylaxis of the disease associated with AMPA receptors in the brain of the primate

organism are associated with each other; and
a means which compares information on the distribution and/or the expression level of AMPA receptors in a brain of an organism of a human subject that is input with the data and transmits, to an output terminal, information on the type, the dosage, and/or the usage of the drug that is recommended for the human subject.
(13) A system including: the input terminal described in (10) ;
the server described in (11) or (12); and
the output terminal which outputs the information transmitted from the server described in (11) or (12).

Effects of the Invention

According to the present invention, it is possible to provide a technology for imaging AMPA receptors in the brain of a primate organism and applications thereof.

BRIEF DESCRIPTION OF THE DRAWINGS

Fig. 1 is a diagram showing an example of the configuration of a system of the present invention;
Fig. 2 is a flowchart showing information processing in a checking unit and an instruction unit;
Fig. 3 is a diagram showing an example of the configuration of the system of the present invention;
Fig. 4 is a graph showing the result of an AMPA receptor binding test (in vitro autoradiography method) in vitro on K-2;
Fig. 5 is a graph showing the result of an AMPA receptor binding test (electrophysiological verification) in vitro on K-2 and K-4;
Fig. 6 is a PET image in vivo after the administration of a radio-labeled K-2 to a Wistar rat;
Fig. 7 is a PET image in vivo after the administration of the radio-labeled K-2 to a Wistar Kyoto rat (WKY rat);
Fig. 8 is a graph showing the uptake amounts of radio-labeled K-2 in the brain of the Wistar rat and the WKY rat;
Fig. 9 is a graph showing the result of a forced swimming test in the acute administration of K-2 to the rat;
Fig. 10 is a graph showing the result of forced swimming tests on K-2 and K-4;
Fig. 11 is PET imaging images of healthy persons to which [$^{11}$C] K-2 was administered;
Fig. 12 is PET imaging images of the healthy persons to which [$^{11}$C] K-2 was administered;
Fig. 13 is a graph showing the result of PET imaging on the healthy persons to which [$^{11}$C] K-2 was administered;
Fig. 14 is a graph showing the result of PET imaging on the healthy persons to which [$^{11}$C] K-2 was administered;
Fig. 15 is PET imaging images of epilepsy patients to which [$^{11}$C] K-2 was administered;
Fig. 16 is PET imaging subtraction images of the epilepsy patients to which [$^{11}$C] K-2 was administered;
Fig. 17 is a graph showing asymmetry indexes of the healthy person and the epilepsy patient to which [$^{11}$C] K-2 was administered;
Fig. 18 is a graph showing asymmetry indexes of the epilepsy patient to which [$^{11}$C] K-2 and FDG were administered; and
Fig. 19 is PET imaging images of a depressed patient to which [$^{11}$C] K-2 was administered.

PREFERRED MODE FOR CARRYING OUT THE INVENTION

Although embodiments of the present invention will be described below, the present invention is not limited to these embodiments.

(Imaging method)

In an embodiment of the present invention, a method of imaging AMPA receptors in the brain of a primate organism is provided. This method includes a step of delivering into the brain a substance which is administered to the primate organism, which is selectively bound to the AMPA receptors in the brain of the primate organism and is radio-labeled, and binding the substance to the AMPA receptors in the brain. The present inventors have first found that as in Examples which will be described later, when a substance is used which is selectively bound to AMPA receptors in the brain of a primate organism and is radio-labeled, radiation which is emitted from the substance bound to the AMPA receptors in the brain of the primate organism can be detected, and thereby have established a methodology according to the present embodiment.
In other words, in the present embodiment based on this novel discovery, a step is provided of detecting the radiation which is emitted from the substance bound to the AMPA receptors in the brain so as to acquire data on the distribution and/or the expression level of the AMPA receptors in the brain. In this way, the distribution and/or the

expression level of the AMPA receptors in the entire brain of the primate organism is grasped, and thus it is possible to image the AMPA receptors in the brain of the primate organism.

The imaging using the detection of radiation is not particularly limited, and molecular imaging, for example, positron emission tomography (PET), a multiphoton imaging method, a two-photon imaging method, a near-infrared fluorescence imaging method, autoradiography, single photon emission computed tomography (SPECT), or the like may be adopted. Among them, PET imaging is preferable.

The primate is not particularly limited, and a human or a monkey may be adopted. Between humans and monkeys, substance metabolism, the passage of a blood-brain barrier, and the amount and the distribution of AMPA receptors in the brain are considered to be slightly different. In this regard, although Examples which will be described later are based on data on humans, the present inventors have confirmed that imaging can likewise be performed on monkeys (data thereof is not shown).

Preferably, in an embodiment, a required time is allowed after the delivery of the substance described above into the brain such that the substance which is not bound to the AMPA receptors in the brain is urged to be discharged to the outside of the brain, and the detection of the radiation is thereafter performed. In this way, it is possible to more frequently detect the radiation derived from the substance which is bound to the AMPA receptors in the brain, and thus the accuracy of the imaging of the AMPA receptors is enhanced.

The required time described above is not particularly limited and may be set in advance based on the substance used and statistics (may be typically set to 10 minutes or more, 20 minutes or more, 30 minutes or more, 40 minutes or more, or 45 minutes or more after the administration of the substance) or may be set for each organism which is a target. Specifically, the required time can be (i) calculated by being applied to a mathematical analysis model, (ii) the maximum time period during which when a ratio between a region where a target protein is present and a region where the target protein is not present becomes constant, the difference therebetween is maximized or reduced or (iii) a time period during which a difference between a disease and a healthy person can be detected most clearly.

On the other hand, when the required time is excessively prolonged, the absolute amount of radiation is attenuated, and thus it can be difficult to highly accurately detect the radiation. Hence, the required time is not particularly limited and may be set to, for example, 110 minutes or less, 100 minutes or less, 90 minutes or less, 80 minutes or less, 70 minutes or less, or 60 minutes or less after the administration of the substance.

Preferably, in an embodiment, the detection is performed both when a first time elapses after the delivery of the substance into the brain and when a second time longer than the first time elapses, and based on individual detection values, the data on the distribution and/or the level of the AMPA receptors in the brain is acquired. More preferably, between the first time and the second time, the amount of radiation which is taken into each brain region and derived from the substance is detected continuously or discontinuously, the average value thereof is calculated, and based on a difference in individual average values between the brain regions, the data on the distribution and/or the level of the AMPA receptors in the brain can be acquired. During the time until the second time elapses after the first time elapses, a region in which the detection value of radiation is changed (lowered) with a relatively small range is highly likely to correspond to the AMPA receptors. Hence, the data based on the difference in the detection values is utilized, and thus the accuracy of the imaging of the AMPA receptors can be enhanced. When the first time and the second time are excessively prolonged, the absolute amount of radiation is attenuated, and thus it can be difficult to highly accurately detect the radiation.

The first time and the second time are not particularly limited, and may be set in advance based on the substance used and statistics or may be set for each organism which is a target. The first time may be typically set to 10 minutes or more, 20 minutes or more, 30 minutes or more, 40 minutes or more, or 45 minutes or more after the administration of the substance, may be set to 110 minutes or less, 100 minutes or less, 90 minutes or less, 80 minutes or less, 70 minutes or less, or 60 minutes or less, or may be determined in the same manner as the required time described above. The second time may be typically set so as to be 30 minutes or more and 150 minutes or less (specifically, 60 minutes or less) after the administration of the substance.

The substance described above is not particularly limited, and, for example, the substance is passed through a blood-brain barrier so as to be delivered into the brain after being administered parenterally, intravenously, or intraperitoneally. Hence, the substance needs to have the property of being able to pass through the blood-brain barrier, and accordingly, the substance preferably has a required low molecular weight, required lipid solubility, and required blood solubility. The substance may be a single substance or may be in a state where it is carried by a DDS (drug delivery system).

The substance may be included in a pharmaceutically acceptable carrier. The pharmaceutically acceptable carrier is not particularly limited, and examples thereof include sterile water, saltwater, saline or phosphate buffered saline (PBS), a sodium chloride injection, a Ringer's injection, an isotonic dextrose injection, a sterile water injection, dextrose, a lactated Ringer's injection, and the like.

The administered amount of substance described above may be set as necessary according to the type of substance used; the age, the weight, the health condition, the gender, and the content of the meals of a target to which

the substance is administered; the number of times the substance is administered; the route of the administration; and the like. The administration of the substance is not particularly limited.

The substance is not particularly limited, and may be, for example, a compound represented by Formula (I) below or a pharmaceutically acceptable salt or solvate thereof.

[Chem. 2]

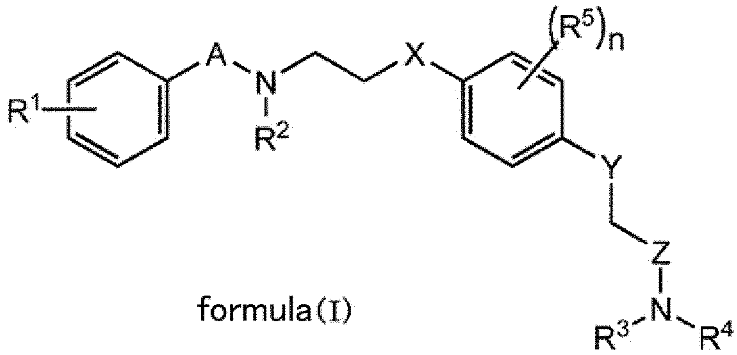

formula (I)

In the formula,
each of A and Z independently represents CO, SO, or $SO_2$;
each of X and Y independently represents S or O;
each of $R^1$ to $R^4$ independently represents hydrogen, alkyl, alkenyl, alkynyl, or halo;
each $R^5$ independently represents alkyl, alkenyl, alkynyl, or halo;
n represents an integer of 0 to 4; and
one or more atoms are radioisotopes of the atoms.

Although in the compound represented by Formula (I), the radioisotope is selected from the group consisting of $^{15}O$, $^{13}N$, $^{11}C$, $^{18}F$, and the like, there is no particular limitation. In terms of half-life, the radioisotope is preferably $^{11}C$ or $^{18}F$.

Preferably, one, two, three, or four of, preferably one of, $R^1$ to $R^4$ is a group which includes a radioisotope (for example, [$^{11}C$] alkyl (preferably $^{11}CH_3$), [$^{11}C$]alkenyl, [$^{11}C$]alkynyl, or $^{18}F$). Specifically, $R^2$ preferably represents alkyl, and furthermore, preferably, each of $R^3$ and $R^4$ represents hydrogen and each of $R^3$ and $R^4$ independently represents alkyl.

Preferably, when as the compound represented by Formula (I), A represents $SO_2$, Z represents CO, X represents S, Y represents O, $R^2$ represents alkyl, $R^1$ represents hydrogen, alkyl, or halo, and $R^1$ represents alkyl or halo, $R^1$ is located in a para-position, one of $R^3$ and $R^4$ represents hydrogen and the other represents alkyl, $R^5$ represents halo, in particular, fluoro, $R^5$ is located in both ortho-positions with respect to a Y group (that is, both meta-positions with respect to an X group), n represents 2 and one of R1 to R4 presents a group including a radioisotope (for example, [$^{11}C$]alkyl (preferably, $^{11}CH_3$), [$^{11}C$]alkenyl, [$^{11}C$]alkynyl, or $^{18}F$).

In still another embodiment, more preferably, when as the compound represented by Formula (I), A represents $SO_2$, Z represents CO, X represents S, Y represents O, $R^2$ represents alkyl, $R^1$ represents hydrogen, alkyl, or halo, and $R^1$ represents alkyl or halo, $R^1$ is located in a para-position, one of $R^3$ and $R^4$ represents hydrogen and the other represents alkyl, $R^5$ represents halo, in particular, fluoro, $R^5$ is located in both ortho-positions with respect to a Y group (that is, both meta-positions with respect to an X group), n represents 2 and one of $R^1$ to $R^4$ represents a group including a radioisotope (for example, [$^{11}C$]alkyl (preferably $^{11}CH_3$), [$^{11}C$]alkenyl, [$^{11}C$]alkynyl, or $^{18}F$).

Specific examples of the compound including a radioisotope include:

[Table 1]

| | Compound Name | Abbreviation | Structural Formula |
|---|---|---|---|
| 1' | {4-[2-(benzenesulfonyl-[$^{11}$C]methyl-amino)-ethylsulfanyl]-2,6-difluoro phenoxy}-acetamide | radio-labeled K-2 | 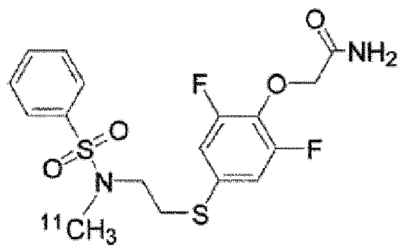 |
| 2' | 2-[4-(2-benzenesulfonylamino-ethylsulfanyl)-2,6-difluoro-phenoxyl-N-[$^{11}$C]methyl-acetamide | radio-labeled M-1 | 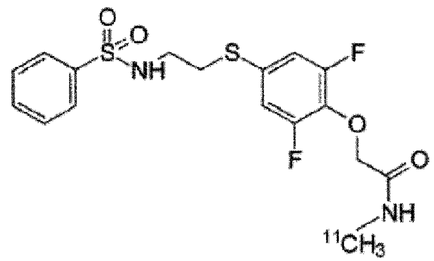 |
| 3' | 2-{2,6-difluoro-4-[2-(4-[$^{18}$F]fluoro-benzenesulfonylamino)-ethylsulfanyl]-phenoxy}-acetamide | radio-labeled M-2 | 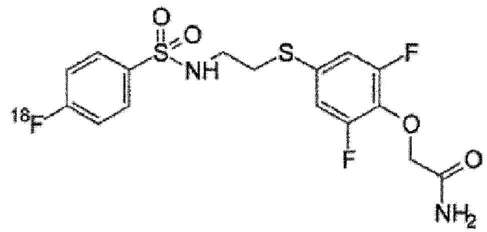 |
| 4' | 2-{2,6-difluoro-4-[2-(4-[$^{11}$C] methyl-benzenesulfonylamino)-ethylsulfanyl]-phenoxy}-acetamide | radio-labeled M-3 | 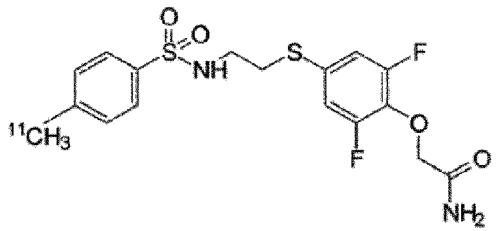 |

(Definitions)

The term "alkyl" means a monovalent group that is produced when saturated aliphatic hydrocarbon misses one hydrogen atom. An alkyl has, for example, 1 to 15 ($C_1$-$C_{15}$) carbon atoms, and typically has 1 to 10 ($C_1$-$C_{10}$), 1 to 8 ($C_1$-$C_8$), 1 to 6 ($C_1$-$C_6$), 1 to 5 ($C_1$-$C_5$), 1 to 4 ($C_1$-$C_4$), 1 to 3 ($C_1$-$C_3$), 1 to 2 ($C_1$-$C_2$), or 2 to 6 ($C_2$-$C_6$) carbon atoms. An alkyl may be a straight chain or may be branched. Examples of alkyls include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, t-butyl, pentyl, isopentyl, neopentyl, and hexyl. An alkyl may be further substituted by an adequate substituent.

The term "alkenyl" means an unsaturated aliphatic hydrocarbon group having at least one double bond. An alkenyl has, for example, 2 to 15 ($C_2$-$C_{15}$) carbon atoms, and typically has 2 to 10 ($C_2$-$C_{10}$), 2 to 8 ($C_2$-$C_8$), 2 to 6 ($C_2$-$C_6$), 2 to 5 ($C_2$-$C_5$), 2 to 4 ($C_2$-$C_4$), 2 to 3 ($C_2$-$C_3$), 3 to 6 ($C_3$-$C_6$), 3 to 8 ($C_3$-$C_8$), 4 to 6 ($C_4$-$C_6$), 4 to 7 ($C_4$-$C_7$), or 4 to 8 ($C_4$-$C_8$) carbon atoms. An alkenyl may be a straight chain or may be branched. Examples of alkenyls include, but are not limited to, specifically, vinyl ($-CH=CH_2$), allyl ($-CH_2CH=CH_2$), $-CH=CH(CH_3)$, $-CH=C(CH_3)_2$, $-C(CH_3)=CH_2$, $-C(CH_3)=CH(CH_3)$, $-C(CH_2CH_3)=CH_2$, 1,3-butadienyl ($-CH=CH-CH=CH_2$), and hepta-1,6-diene-4-yl ($-CH_2-(CH_2CH=CH_2)_2$). An alkenyl may be further substituted by an adequate substituent.

The term "alkynyl" means an unsaturated aliphatic hydrocarbon group having at least one triple bond. An alkynyl has, for example, 2 to 15 ($C_2$-$C_{15}$) carbon atoms, and typically has 2 to 10 ($C_2$-$C_{10}$), 2 to 8 ($C_2$-$C_8$), 2 to 6 ($C_2$-$C_6$), 2 to 5 ($C_2$-$C_5$), 2 to 4 ($C_2$-$C_4$), 2 to 3 ($C_2$-$C_3$), 3 to 6 ($C_3$-$C_6$), 3 to 8 ($C_3$-$C_8$), 4 to 6 ($C_4$-$C_6$), 4 to 7 ($C_4$-$C_7$), or 4 to 8 ($C_4$-$C_8$) carbon atoms. An alkynyl may be a straight chain or may be branched. Examples of alkynyl include, but are not limited to, ethynyl ($-C\equiv CH$), $-C\equiv CH(CH_3)$, $-C\equiv C(CH_2CH_3)$, $-CH_2C\equiv CH$, $-CH_2C\equiv C(CH_3)$, and-$CH_2C\equiv C(CH_2CH_3)$. An alkynyl may be further substituted with an appropriate substituent.

The term "halogen" or "halo" means fluoro (-F), chloro (-Cl), bromo (-Br), and iodine (-I).

The term "pharmaceutically acceptable salt" indicates a salt that is not harmful to mammals, particularly humans. Pharmaceutically acceptable salts can be formed using nontoxic acids or bases including inorganic acids or inorganic bases, or organic acids or organic bases. Examples of pharmaceutically acceptable salts include metal salts formed with aluminum, calcium, lithium, magnesium, potassium, sodium, zinc, and the like, and organic salts formed with lysine, N,N'-dibenzylethylenediamine, chloroprocaine, choline, diethanolamine, ethylenediamine, meglumine (N-methylglucamine), procaine, and the like. Further, pharmaceutically acceptable salts include acid-addition salts and base-addition salts.

The term "solvate" means a solvent-containing compound that is formed by association of one or a plurality of solvent molecules to the compounds of the present invention. Solvates include, for example, monosolvates, disolvates, trisolvates, and tetrasolvates. Further, solvates include hydrates.

(Producing method and intermediate)

(Synthesis Example 1)

The compound represented by Formula (I), or the pharmaceutically acceptable salt or solvate thereof, in which $R^2$ represents alkyl, alkenyl, or alkynyl can be produced, for example, by reacting a compound represented by the following Formula (II), or a pharmaceutically acceptable salt or solvate thereof

[Chem. 3]

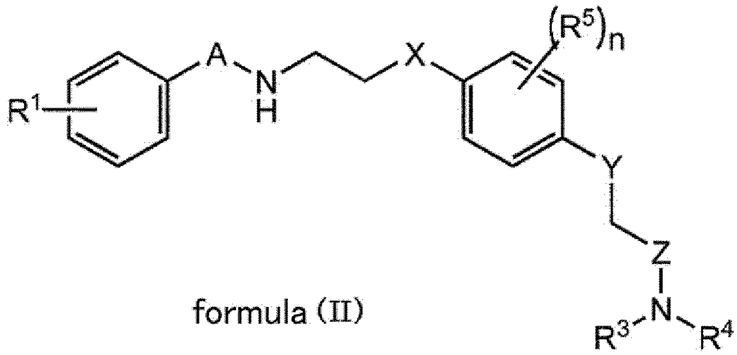

formula (II)

(in the formula, A, X, Y, Z, $R^1$, $R^3$, $R^4$, $R^5$, and n are the same as defined in the compound represented by Formula (I)) with $X^1$-$R^2$ (in the formula, $R^2$ represents alkyl, alkenyl, or alkynyl and $X^1$ represents halogen). In an embodiment, both the $R^3$ and the $R^4$ in Formula (I) and Formula (II) represent hydrogen. In an embodiment, $R^2$ represents [$^{11}$C]alkyl, [$^{11}$C]alkenyl, or [$^{11}$C]alkynyl, and $R^2$ preferably represents [$^{11}$C]alkyl, particularly $^{11}CH_3$. In an embodiment, $X^1$ represents I. As a specific examples of the compound represented by Formula (II), 2-[2,6-difluoro-4-({2-[(phenylsulfonyl)amino]ethyl} thio)phenoxy]acetamide (PEPA) is exemplified.

The reaction can be performed in a polar aprotic solvent such as dimethylformamide (DMF), tetrahydrofuran, acetonitrile, acetone, or dimethylsulfoxide. Further, the reaction is preferably performed using a base such as NaOH under a basic condition. The reaction temperature is room temperature to reflux temperature, and particularly, is preferably 60 to 100°C and more preferably 80°C. The reaction time is 1 minute to 10 minutes, and particularly 5 minutes.

The PET probe has to be produced in a short time and with a high yield since the radioisotope usually has a short half-life. The reaction is suitable for the production of the PET probe since the reaction quantitatively progresses in a short time.

The present inventors have found that the reaction of the compound represented by Formula (II) with $X^1$-$R^2$ quantitatively occurs in a NH group adjacent to the A group of the compound represented by Formula (II). Therefore, even if $R^3$ and $R^4$ represent hydrogen, only the NH group can be substituted with an N-$R^2$ group without use of a protecting group.

The compound represented by Formula (II), or the pharmaceutically acceptable salt or solvate thereof, can be used as an intermediate used for producing the compound represented by Formula (I), or the pharmaceutically acceptable salt or solvate thereof, in which $R^2$ represents alkyl, alkenyl, or alkynyl. Further, the compound represented by Formula (II), or the pharmaceutically acceptable salt or solvate thereof, can be used as an intermediate used for producing the radio-labeled compound represented by Formula (I), or the pharmaceutically acceptable salt or solvate thereof, in which $R^2$ represents [$^{11}$C]alkyl, [11C]alkenyl, or [11C]alkynyl.

(Synthesis Example 2)

The compound represented by Formula (I), or the pharmaceutically acceptable salt or solvate thereof, in which $R^1$ represents alkyl, alkenyl, or alkynyl can be produced, for example, by reacting a compound represented by the following Formula (III), or pharmaceutically acceptable salt or solvate thereof

[Chem. 4]

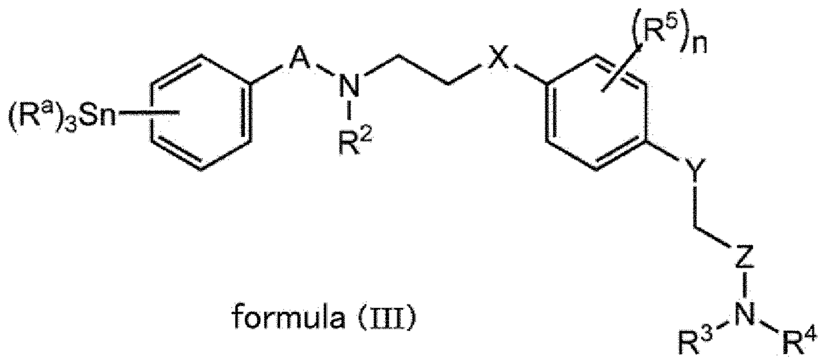

formula (III)

(in the formula, A, X, Y, Z, $R^2$, $R^3$, $R^4$, $R^5$, and n are the same as defined above, and each $R^a$ independently represents alkyl, alkenyl, or alkynyl) with $X^1$-$R^1$ (in the formula, $R^1$ is the same as defined above, and $X^1$ represents halogen). In an embodiment, all $R^a$s are n-butyl. In an embodiment, $R^1$ represents [$^{11}$C]alkyl, [$^{11}$C]alkenyl, or [$^{11}$C]alkynyl, and $R^1$ preferably represents [$^{11}$C]alkyl, particularly $^{11}CH_3$. In an embodiment, $X^1$ represents I.

Specific examples of the compound represented by Formula (III) include the following:

[Table 2]

| | Compound Name | Abbreviation | Structural Formula |
|---|---|---|---|
| 5 | 2-(2,6-difluoro-4-((2-(4-(tributylstannyl) phenylsulfonamide)ethyl)thio)phenoxy) acetamide | M-3pre | 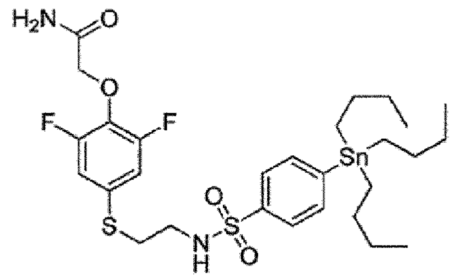 |

The reaction can be performed in the presence of a palladium catalyst, a phosphine ligand, a carbonate, and a copper halide. The palladium catalyst is, for example, tris(dibenzylideneacetone)dipalladium or the like. Further, the phosphine ligand is, for example, tri(o-tolyl)phosphine, (di-tert-butyl)methylphosphine, or the like. The carbonate is $K_2CO_3$ or the like. The copper halide is CuCl or the like. The reaction can be performed in a polar aprotic solvent such as dimethylformamide (DMF), tetrahydrofuran, acetonitrile, acetone, or dimethylsulfoxide. The reaction temperature is room temperature to reflux temperature, and particularly, is preferably 60 to 100°C, and more preferably 80°C. The reaction time is 1 minute to 10 minutes, and particularly 5 minutes.

The PET probe has to be produced in a short time and with a high yield since the radioisotope usually has a short half-life. The reaction is suitable for the production of the PET probe since the reaction quantitatively progresses in a short time.

The compound represented by Formula (III), or the pharmaceutically acceptable salt or solvate thereof, can be used as an intermediate used for producing the compound represented by Formula (I), or the pharmaceutically acceptable salt or solvate thereof, in which $R^1$ represents alkyl, alkenyl, or alkynyl. Further, the compound represented by Formula (III), or the pharmaceutically acceptable salt or solvate thereof, can be used as an intermediate used for producing the radio-labeled compound represented by Formula (I), or the pharmaceutically acceptable salt or solvate thereof, in which $R^1$ represents [$^{11}$C]alkyl, [$^{11}$C]alkenyl, or [$^{11}$C]alkynyl.

The compound represented by Formula (I), or the pharmaceutically acceptable salt or solvate thereof, can be produced by the method described in the following Examples.

(Program)

In an embodiment of the present invention, a program for instructing a computer to perform the imaging method described above is provided. Specifically, the computer uses the program so as to control an imaging device, and thereby images the AMPA receptors in the brain of the primate organism.

(Diagnostic agent, companion diagnostic agent, and drug)

In an embodiment of the present invention, a diagnostic agent for diseases associated with AMPA receptors in the brain of the primate organism or a companion diagnostic agent for the therapy or the prophylaxis of the diseases is provided. Here, the companion diagnostic agent for the therapy refers to a diagnostic agent which determines, when a disease associated with AMPA receptors in the brain is found, whether or not the therapy can be expected. Here, the companion diagnostic agent for the prophylaxis refers to a diagnostic agent which determines, when a disease associated with AMPA receptors in the brain is found, whether or not prophylaxis for estimating a future disease state (prognosis) or reducing the further progress of the disease can be expected.

By use of the diagnostic agent described above, data on the distribution and/or the expression level of the AMPA receptors in the brain which can be obtained from the target of the primate organism is compared with a correlation between the disease described above and the distribution and/or the expression level of the AMPA receptors in the brain, and thus it is possible to diagnose the target disease (specifically, whether the disease described above is present or absent, the seriousness, the possibility of seizure, and the like).

By use of the companion diagnostic agent described above, data on the distribution and/or the expression level of the AMPA receptors in the brain which can be obtained from the target of the primate organism is compared with the correlation between the disease described above and the distribution and/or the expression level of the AMPA receptors in the brain, and thus the state of the target disease can be grasped, with the result that based on this, it is possible to establish a prophylactic/therapeutic plan for the disease (the type, the combination, the dosage, the usage, and the like of a prophylactic/therapeutic drug which is administered).

For example, for a target in which the expression level of the AMPA receptors is grasped to be reduced, the administration of an AMPA receptor function activator can be recommended, and for a target in which the expression level of the AMPA receptors is grasped to have increased, the administration of an AMPA receptor antagonist can be recommended. For example, in a disease group which is clinically diagnosed to be depression in current disease classifications (such as DSM-V or ICD-10), there is a case where the expression level of the AMPA receptors is recognized to be increased, and there is a case where the expression level of the AMPA receptors is not changed or is reduced. In such cases, even when the diseases are depression in clinical diagnosis, tailor-made diagnosis/therapy such as the administration of an antagonist for increased AMPA receptor depression is performed. According to a decrease/increase of range in the expression level of the AMPA receptors and the type and the degree of distribution abnormality (when a healthy person is assumed to be normal), the type of AMPA receptor function activator/AMPA receptor antagonist (a difference in in vivo metabolic time and a difference in effective blood concentration), the administered amount, the frequency of administration, and the timing of administration (for example, when the expression level or the distribution of the AMPA receptors on the sign of the seizure of a symptom is grasped, a drug is prophylactically administered) can be set.

In other words, an embodiment of the present invention relates to drugs for the therapy or the prophylaxis of diseases associated with AMPA receptors in the brain of the primate organism, and also relates to a drug in which a substance that is selectively bound to the AMPA receptors in the brain of the primate organism is an active ingredient and which is administered according to an administration plan based on the data on the distribution and/or the expression level of the AMPA receptors in the brain that can be obtained by the imaging method described above.

The AMPA receptor antagonist which is a drug according to an embodiment (that may be used together with the companion diagnostic agent) is not particularly limited, and examples thereof include perampanel hydrate (Eisai Co., Ltd.) for epilepsy disease and talampanel (Teva Pharmaceutical Industries Ltd.).

The AMPA receptor function activator which is a drug according to an embodiment (that may be used together with the companion diagnostic agent) is not particularly limited, and may be the compound represented by Formula (I) or the pharmaceutically acceptable salt or solvate thereof.

[Chem. 5]

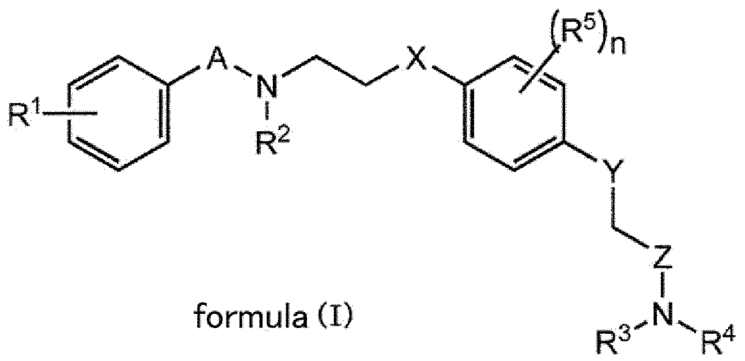

formula (I)

In the formula, each of A and Z independently represents CO, SO, or $SO_2$, and in the case of these groups, it is expected that an interaction between the groups and the AMPA receptors is exhibited. Among these, preferably, each of A and Z independently represents CO or $SO_2$, and more preferably, A represents $SO_2$ and Z represents CO. Each of X and Y independently represents S or O, and preferably, X represents S and Y represents O. Each of $R^1$ to $R^4$ independently represents hydrogen, alkyl, alkenyl, alkynyl, or halo. In an embodiment, a case where all of $R^1$ to $R^4$ are hydrogen is prevented from occurring, that is, at least one of $R^1$ to $R^4$ represents an element other than hydrogen. In an embodiment, $R^2$ represents alkyl. In another embodiment, $R^1$ represents alkyl or halo. $R^1$ can be located in any of an ortho-position, a meta-position, and a para-position. Preferably, $R^1$ is located in the para-position. In still another embodiment, one of $R^3$ and $R^4$ represents hydrogen and the other represents alkyl. Most preferably, each of $R^1$, $R^3$, and $R^4$ independently represents hydrogen, alkyl, alkenyl, alkynyl, or halo, and $R^2$ represents alkyl, alkenyl, or alkynyl. Each $R^5$ independently represents alkyl, alkenyl, alkynyl, or halo. $R^5$ preferably represents halo, and particularly preferably represents fluoro. Further preferably, $R^5$ is located in both ortho-positions with respect to a Y group (that is, both meta-positions with respect to an X group). n represents an integer of 0 to 4. Preferably, n represents 2.

In still another embodiment, as a combination of individual substituents in the compound represented by Formula (I), a combination is preferable in which each of A and Z independently represents CO, SO, or $SO_2$, each of X and Y independently represents S or O, each of $R^1$, $R^3$, and $R^4$ independently represents hydrogen, alkyl, alkenyl, alkynyl, or halo, $R^2$ represents alkyl, alkenyl or alkynyl, each $R^5$ independently represents alkyl, alkenyl, alkynyl, or halo, and n represents an integer of 0 to 4.

In still another embodiment, as a combination of individual substituents in the compound represented by Formula (I), a combination is preferable in which A represents $SO_2$, Z represents CO, X represents S, Y represents O, $R^2$ represents alkyl, and $R^1$ represents hydrogen, alkyl, or halo, and in a case where $R^1$ represents alkyl or halo, $R^1$ is located in a para-position, one of $R^3$ and $R^4$ represents hydrogen and the other represents alkyl, each $R^5$ independently represents alkyl, alkenyl, alkynyl, or halo, and n represents an integer of 0 to 4.

In still another embodiment, as a combination of individual substituents in the compound represented by Formula (I), a combination is preferable in which A represents $SO_2$, Z represents CO, X represents S, Y represents O, $R^2$ represents alkyl, $R^1$ represents hydrogen, alkyl, or halo, and in a case where $R^1$ represents alkyl or halo, $R^1$ is located in a para-position, one of $R^3$ and $R^4$ represents hydrogen and the other represents alkyl, $R^5$ represents halo, in particular, fluoro, $R^5$ is located in both ortho-positions with respect to a Y group (that is, both meta-positions with respect to an X group), and n represents 2.

In still another embodiment, as a combination of individual substituents in the compound represented by Formula (I), a combination is preferable in which A represents $SO_2$, Z represents CO, X represents S, Y represents O, $R^2$ represents alkyl, $R^1$ represents hydrogen, alkyl, or halo, and in a case where $R^1$ represents alkyl or halo, $R^1$ is located in a para-position, both of $R^3$ and $R^4$ represent hydrogen, each $R^5$ independently represents alkyl, alkenyl, alkynyl, or halo, and n represents an integer of 0 to 4.

Specific examples of the compound represented by Formula (I) include:

[Table 3]

| | Compound Name | Abbreviation | Structural Formula |
|---|---|---|---|
| 1 | {4-[2-(benzenesulfonyl-methyl-amino)-ethylsulfanyl]-2,6-difluoro-phenoxy}-acetamide | K-2 | 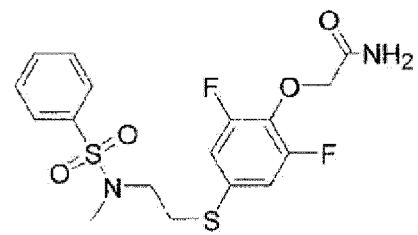 |
| 2 | 2-[4-(2-benzenesulfonylamino-ethylsulfanyl)-2,6-difluoro-phenoxy]-N-methyl-acetamide | M-1 | 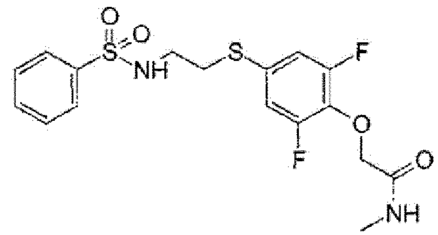 |
| 3 | 2-{2,6-difluoro-4-[2-(4-fluoro-benzenesulfonylamino)-ethylsulfanyl]-phenoxy}-acetamide | M-2 | 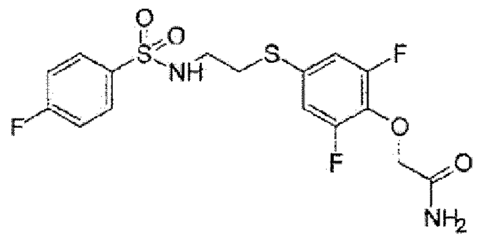 |
| 4 | 2-{2,6-difluoro-4-[2-(4-methyl-benzenesulfonylamino)-ethylsulfanyl]-phenoxy]-acetamide | M-3 | 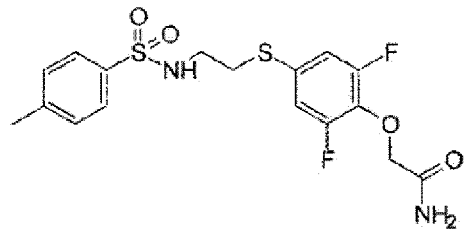 |

A production method and an intermediate are the same as described previously.

The AMPA receptor function activator/the AMPA receptor antagonist can be administered orally or parenterally. As an orally administered drug, a solid preparation such as a powder, a granule, a capsule, or a tablet, or a liquid preparation such as a syrup or an elixir can be used. As a parenterally administered drug, an injection (for a vein, a muscle, or the like), a rectally administered drug, external medicine for skin, or an inhalant can be used. These preparations are produced according to a normal method by adding pharmaceutically acceptable production aids to active ingredients. Furthermore, by a known technology, they can also be formed as sustained preparations.

The diseases associated with AMPA receptors in the brain of the primate organism are not particularly limited, and may be mental diseases and neurological diseases such as:

(1) mental diseases such as depression, major depression, bipolar depression, dysthymia, emotional disorder, recurrent depression, postnatal depression, stress disorder, depression symptom, manic disorder, anxiety, generalized anxiety disorder, anxiety syndrome, panic disorder, phobia, social phobia, social anxiety disorder, obsessive-compulsive disorder, post-traumatic stress syndrome, post-traumatic stress disorder, Tourette's syndrome, autism, fragile X syndrome, Rett syndrome, adjustment disorder, bipolar disorder, neuropathy, schizophrenia, chronic fatigue syndrome, anxiety neurosis, compulsive neurosis, scare disorder, epilepsy, hypersensitivity, attention deficit hyperactivity disorder, psychotic major depression, refractory major depression, and treatment-resistant depression;

(2) degenerative neurological disorders such as Alzheimer's disease, Alzheimer's-type senile dementia, Parkinson's disease, Huntington's chorea, multiple cerebral infarction dementia, frontotemporal dementia, Parkinson's-type frontotemporal dementia, progressive supranuclear palsy, Pick's syndrome, Niemann-Pick syndrome, corticobasal degeneration, Down syndrome, vascular dementia, Lewy body dementia, amyotrophic lateral sclerosis, motor neurogenic disease, Creutzfeldt-Jakob's disease, cerebral palsy, progressive supranuclear paralysis, and multiple sclerosis;

(3) cognitive/memory impairments associated with aging such as age-related memory disorder and senile dementia;

(4) sleep disorders such as intrinsic sleep disorder, extrinsic sleep disorder, circadian rhythm disorder, sleep-related disease, sleep disorder associated with internal medicine or psychiatric disorder, stress insomnia, insomnia, insomniac neurosis, and sleep apnea syndrome;

(5) respiratory depression caused by anesthetic, traumatic disease, or neurodegenerative disease; and

(6) traumatic brain injury, stroke, anorexia, eating disorder, anorexia nervosa, bulimia nervosa, other eating disorders, alcoholism, alcohol abuse, alcohol amnesia, alcoholism, alcohol preference, alcohol withdrawal, alcoholic psychosis, alcohol poisoning, alcoholic jealousy, alcoholic mania, alcohol-dependent mental disorder, alcohol psychosis, drug preference, drug phobia, drug mania, drug withdrawal, migraine headache, stress headache, tension headache, diabetic neuropathy, obesity, diabetes, muscle spasms, Meniere's disease, autonomic imbalance, alopecia, glaucoma, deafness, hypertension, heart disease, tachycardia, congestive heart failure, hyperpnea, bronchial asthma, apnea, sudden infant death syndrome, inflammatory disease, allergic disease, impotence, menopause, infertility, cancer, immunodeficiency syndrome due to HIV infection, encephalomyelitis, acromegaly, incontinence, metabolic syndrome, osteoporosis, peptic ulcer, irritable bowel syndrome, inflammatory bowel disease, ulcerative colitis, Crohn's disease, stress gastrointestinal disorder, neurogenic vomiting, peptic ulcer, diarrhea, constipation, and postoperative ileus.

In an embodiment of the present invention, a drug for the therapy or the prophylaxis of the disease associated with AMPA receptors in the brain of the primate organism is provided.

The disease described above is not particularly limited and may be one or more types selected from the group consisting of epilepsy, depression, schizophrenia, cerebral ischemia, Parkinson's disease, Alzheimer's disease, autism, attention-deficit hyperactivity disorder (ADHD) and multiple sclerosis.

The drug according to the present embodiment may include a pharmaceutically acceptable carrier. The pharmaceutically acceptable carrier is not particularly limited, and examples thereof include sterile water, saltwater, saline or phosphate buffered saline (PBS), a sodium chloride injection, a Ringer's injection, an isotonic dextrose injection, a sterile water injection, dextrose, a lactated Ringer's injection and the like.

(Screening method)

An embodiment of the present invention is a method of screening a therapeutic or prophylactic agent for diseases associated with AMPA receptors in the brain of the primate organism. The method includes a step of screening, before and after a candidate substance is administered to the primate organism, the candidate substance based on a difference in the data on the distribution and/or the expression level of the AMPA receptors in the brain that is obtained by the imaging method described above.

Specifically, based on an increase/decrease range in the expression level of the AMPA receptors in the brain

and variations in the type and the degree of distribution abnormality (when a healthy person is assumed to be normal) before and after the candidate substance is administered to the primate organism, the candidate substance can be screened as the AMPA receptor function activator/the AMPA receptor antagonist. In this way, it can be expected that the diseases described above which are conventionally regarded collectively are finely classified according to the expression level or the distribution of the AMPA receptors in the brain and that thus an appropriate therapeutic or prophylactic agent is produced for each classification.

In an embodiment, whether or not the screened candidate substrate actually has a therapeutic or prophylactic effect against the disease associated with AMPA receptors in the brain of the primate organism may be checked (such as in animal experiments or tests on humans), and further screening may be performed based on the fact that the candidate substrate actually has the effect.

(System)

A system according to an embodiment of the present invention includes an input terminal, a server, and an output terminal.

The input terminal transmits to the server information on the distribution and/or the expression level of the AMPA receptors in the brain of the primate organism. The information can be acquired by the imaging method of the present invention described above.

A server according to an embodiment includes a database that stores data in which the distribution and/or the expression level of the AMPA receptors in the brain of the primate organism is associated with the state of the disease associated with AMPA receptors in the brain of the primate organism. The server further includes a means which compares information on the distribution and/or the expression level of the AMPA receptors in the brain of the organism of a human subject that is input with the data within the database, generates information on the state of the disease of the human subject (information production unit), and transmits the information to the output terminal. The system which includes the server described above can accurately present the state of the disease of the human subject. The state of the disease includes, for example, whether the disease is present or absent, seriousness, and the possibility of seizure.

For example, the information production unit described above selects, from the database, data which is the same as or similar to the distribution and/or the expression level of the AMPA receptors in the brain of the human subject, and thereby can generate information on the state of the disease.

A server in another embodiment includes a database that stores data in which the distribution and/or the expression level of the AMPA receptors in the brain of the primate organism, the state of the disease associated with AMPA receptors in the brain of the primate organism, and the type, the dosage, and/or the usage of a drug that has already been administered and is intended for the therapy or the prophylaxis of the disease associated with AMPA receptors in the brain of the primate organism are associated with each other. The server further includes a means which compares information on the distribution and/or the expression level of the AMPA receptors in the brain of the organism of a human subject that is input with the data described above, generates information on the type, the dosage, and/or the usage of a drug that is recommended to the human subject (information production unit), and transmits the information to the output terminal. A system which includes the server described above can recommend the type, the dosage, and/or the usage of a drug that is appropriate for the human subject. Specific embodiments may be the same as described above on the companion diagnostic agent and the drug.

In an embodiment, in the database of the server, information on the result of the prophylaxis or the therapy of the human subject by the administration corresponding to the type, the dosage, and/or the usage of the drug that is recommended is fed back, and the information is associated with the information on the distribution and/or the expression level of the AMPA receptors in the brain of the organism of the human subject that has already been input. In this way, the database is updated, and thus the accuracy of recommendations is further enhanced.

The output terminal is an output terminal which outputs the information transmitted from the server described above.

The specific configuration of the system according to the embodiment of the present invention will be described with reference to Fig. 1. As shown in Fig. 1, the system 1000 (unillustrated) of the present invention includes an input terminal 200, a server 300, and an output terminal 500.

<1> Input terminal 200

The input terminal 200 has the function of transmitting to the server information on the distribution and/or the expression level of the AMPA receptors in the brain of the primate organism. Specifically, the input terminal 200 includes an imaging data generation unit 210, a metadata generation unit 220, a synthesis unit 250, and a transmission unit 223. In the input terminal 200, an output transmitted from a molecular imaging device 100 of PET, a multiphoton imaging method, a two-photon imaging method, a near-infrared fluorescence imaging method, autoradiography, SPECT, or the

like is input through a reception terminal (unillustrated) to the imaging data generation unit 210. The data which is input is the data which is acquired by the imaging method of the present invention. In the following description, the molecular imaging device 100 is assumed to be based on PET.

The imaging data generation unit 210 is connected to the molecular imaging device 100 outside the system 1000 so as to receive image data continuously or intermittently, and performs two-step data conversion processing so as to generate data on the distribution and/or the expression level of the AMPA receptors in the brain (hereinafter, the generated data is referred to as imaging data). The first data conversion is data conversion in which a direct output of the molecular imaging device 100 is converted into coordinates. Although it depends on the data generation system of the molecular imaging device 100, the direct output of the molecular imaging device 100 is, for example, brightness (tone) data which is subjected to helical scanning and is continuous in a scan time order. The imaging data generation unit 210 converts the continuous data in the scan time order into absolute coordinates or relative coordinates with a predetermined position in the brain being the origin point. Then, the imaging data generation unit 210 generates data on coordinates in the brain and brightness (tone). As an example, the data is expressed by a form of (Xi, Yj, Zk, Bl) (X, Y, and Z represent the three-dimensional coordinates of an arbitrary starting point, B represents the brightness (tone), and I, j, k, and I represent integers). Since this data is four-dimensional data, by software which can handle a three-dimensional space such as a three-dimensional CAD, the data can be visualized as information obtained by providing the brightness (tone) to the brain space. Specifically, the data can express the brightness (tone) in the brain as the distribution and can also be two-dimensionally cut out.

The second data conversion is data conversion in which the data on the brightness (tone) of the data obtained by the first data conversion is converted into the expression level of the AMPA receptors by a predetermined computation. The data which is used for the computation here is, for example, the name of a PET drug in the imaging method of the present invention, the administered amount thereof, the first time and the second time of the present invention, and the required time after the completion of the synthesis until the administration (time for the attenuation of radiation after the synthesis of the PET drug). Then, the brightness (tone) is converted into the expression level of the AMPA receptors from the delivery of the PET drug of the present invention into the brain, AMPA receptor-specific adsorption, a radiation attenuation curve depending on the nuclide after the synthesis, a measurement time (the first time and the second time), and the like, and thus the data on the expression level of the AMPA receptors is generated. As an example, the data is expressed by a form of (Xi, Yj, Zk, Al) (X, Y, and Z represent the three-dimensional coordinates of an arbitrary starting point, A represents the expression level of the AMPA receptors, and I, j, k, and I represent integers). This data can express the expression level of the AMPA receptors in the brain as the distribution and can also be two-dimensionally cut out.

The metadata generation unit 220 generates additional data associated with the data acquired from the molecular imaging device 100. The data is formed with, for example, (1) data on a subject (data S), (2) data on a checked part (data R), (3) data on imaging conditions (data Z), (4) date and time (data T), and (5) terminal identification number data (data N) .

(1) Data on the subject (data S)

Examples of the data on the subject (data S) include the attribute (classification) and the identification number of the subject, and in the case of a human, for example, the patient identification code, the gender, the age, the weight, the disease name, and the medication history of the human.

(2) Data on the checked part (data R)

The data on the checked part (data R) is data on a specific part in the brain which is checked in the server 300 (an information production unit 310 and a checking unit 350), and one or a plurality of specific parts may be provided. For example, the data is a code which specifies the brain part (region X) that is noted when diagnosis is performed. The region X is, for example, frontal lobe, dentate cortex, hippocampus, amygdala, putamen, cerebellum, or bridge.

(3) Data on the imaging conditions (data Z)

As an example of the data on the imaging conditions (data Z), the data is selected from the name of the molecular imaging device 100 or a previously determined device code, the name of the PET drug used in the imaging, the nuclide, the administered amount, the administration method, the date and time of the synthesis of the PET drug or the date and time of the shipment thereof (or the required time after the synthesis or for shipment until the administration), and the first time and the second time in the imaging method of the present invention.

(4) Data on the date and time (data T)

The data on the date and time (data T) is date and time data which is acquired in order to identify and record the date and time of the generation of metadata, and may be acquired from a clock memory (unillustrated) provided within the input terminal 200 or may be acquired from a clock 900 provided outside the input terminal 200. The date and time may be Coordinated Universal Time (UTC), the standard time for a given country with reference to the UTC, or the time of an internet clock.

(5) Terminal identification number data (data N)

The terminal identification number data (data N) is a unique identification number which is individually provided to each input terminal 200, and is preferably a number in which an authentication relationship with the server 300 is set in advance.

The synthesis unit 250 couples the imaging data generated in the imaging data generation unit 210 and the metadata generated in the metadata generation unit 220 so as to generate coupled data. Then, the entire data is packaged in such a form that it can be transmitted to the outside. Specifically, the frontend of the metadata is coupled to the backend of the imaging data, then a header indicating the frontend of the data is combined with the frontend of the whole and a footer indicating the completion of the data is coupled to the backend, with the result that a data package is configured. As necessary, a synchronous signal which serves as a trigger for the transmission and reception of data and an error correction code may be provided. As necessary, part or the whole of the data package may be encrypted.

The transmission unit 223 transmits the data package described above to the server 300, and an existing communication terminal can be used as it is. Since the server 300 which will be described later may be installed near the input terminal 200 or may be installed remotely, the transmission unit 223 may be a communication terminal corresponding to an intranet or the Internet.

<2> Server 300

The server 300 includes a reception unit 332 (first reception unit 332), a separation unit 333, the information production unit 310, a transmission unit 335 (first transmission unit 335), and a database 400 (first database 400).

Here, the reception unit 332 (first reception unit 332) receives the data package from the input terminal 200, and an existing communication terminal can be used as it is. Here, the header, the footer, the synchronous signal, and the like which are provided for the transmission are removed. When data is encrypted, the data is decoded. As a result of the processing described above, the coupled data in which the imaging data and the metadata are coupled is restored.

The separation unit 333 separates the imaging data and the metadata from the coupled data. Then, the separation unit 333 further separates the data S, the data R, the data Z, the data T, and the data N from the metadata. The separation unit 333 further makes a selection from the data S, the data R, the data Z, the data T, and the data N so as to transmit the selected data to the database 400 (first database 400) (here, as an example, the data R is transmitted). The separation unit 333 further feeds the imaging data and part or the whole of the metadata to the information production unit 310.

The separation unit 333 further identifies the region X based on the data R so as to separate the imaging data of the region X from the imaging data described above. The imaging data of the region X which is separated is output to the information production unit 310 (in particular, the checking unit 350).

[Information production unit 310]

The information production unit 310 includes the checking unit 350 and an instruction unit 360. Then, the matching unit 350 and the instruction unit 360 are coupled to each other in series.

In the checking unit 350, the imaging data of the region X received from the separation unit 333 is checked with reference data received from the database 400 (first database 400). Although the reference data received from the database 400 (first database 400) will be described later, the reference data is the imaging data of the region X which is prepared in advance for reference in diagnosis. An example of the reference data is, in a healthy subject or a model subject, the expression level of the AMPA receptors in the region X.

In the checking unit 350, as shown in Fig. 2, the imaging data of the region X and the reference data of the same region X are compared, and thus in which one the expression level of the AMPA receptors is higher or lower is determined. Specifically, the expression level of the AMPA receptors which is input to the checking unit 350 from the input terminal 200 and the expression level of the AMPA receptors which comes from the database 400 (first database 400) and serves as the reference data are compared so as to determine in which one the expression level of the AMPA receptors is higher or lower in two stages. In the first stage, whether or not the expression level in the imaging data is

higher than the expression level in the reference data is determined, and in the second stage, when the expression level in the imaging data is lower or equal to the expression level in the reference data, whether or not the expression level in the imaging data is lower than the expression level in the reference data is determined. By the comparisons in the two stages, whether or not the expression level in the imaging data is higher than the expression level in the reference data, whether or not the expression level in the imaging data is lower than the expression level in the reference data, and whether or not the expression level in the imaging data is equal to the expression level in the reference data can be determined in three stages.

Then, the instruction unit 360 presents instructions corresponding to the determinations in the three stages described above. Specifically, when the expression level in the imaging data is higher than the expression level in the reference data, the data A is output, when the expression level in the imaging data is lower than the expression level in the reference data, the data B is output, and when the expression level in the imaging data is equal to the expression level in the reference data, the data C is output. These pieces of data A to C are stored in the internal memory (unillustrated) of the server 300 and are read.

As examples of these pieces of data A to C, the data A is "the expression level of the AMPA receptors is higher than the reference," the data B is "the expression level of the AMPA receptors is lower than the reference," and the data C is "the expression level of the AMPA receptors is equal to the reference".

In another example, depending on in which one the expression level of the AMPA receptors is higher or lower, the result of a diagnosis of a disease or the like is presented. In another example, depending on in which one the expression level of the AMPA receptors is higher or lower, a drug to be administered, for example, a therapeutic drug, is presented. In a specific example, the data A is "the prescription of an AMPA receptor antagonist," the data B is "the prescription of an AMPA receptor promoter," and the data C is no description, "no prescription," or "follow-up is needed." As described above, within the information production unit 310, the input data is checked with the reference data, and thus the instruction corresponding to the result of the checking is determined. The determined result is output to the transmission unit 335 (first transmission unit 335) outside the information production unit 310.

The transmission unit 335 (first transmission unit 335) combines the data (referred to as instruction data) output from the information production unit 310 and part or the whole of the metadata separated in the separation unit 335 and transmits it as a data package to the output terminal 500. The metadata which is combined is at least (1) data on the subject, and can be, for example, the identification number of the subject. In the data package, the header and the footer may be included, and the synchronous signal and the error correction code may be included. As necessary, the entire data package may be encrypted. As the transmission unit (first transmission unit 335), an existing communication terminal can be used as it is, and the transmission unit may be a communication terminal corresponding to an intranet or the Internet.

[Database 400 (first database 400)]

The database 400 (first database 400) includes a selection unit 410 (first selection unit 410) and a reference data storage 450. The selection unit 410 (first selection unit 410) and the reference data storage 450 are coupled to each other in series.

The selection unit 410 (first selection unit 410) generates a request command according to the data fed from the separation unit 333 which is outside the database 400 (first database 400) and outputs it to the reference data storage 450. For example, when the first selection unit 410 receives the data R from the separation unit 333, the first selection unit 410 searches the reference data on a reference part which is described so as to generate a command that is output.

In the reference data storage 450, data is stored in which the distribution and/or the expression level of the AMPA receptors in the brain of the primate organism is associated with the state of the disease associated with AMPA receptors in the brain of the primate organism. The form of the storage may be a read-only memory (ROM) or a rewritable random-access memory (RAM). Since the data is preferably updated based on the latest medical knowledge and medical information, the random-access memory (RAM) is preferable. Since on subjects, in particular, humans, the PET imaging is assumed to be repeatedly performed according to the progress of the disease or the conditions of recovery, the random-access memory (RAM) in which imaging data for each of the subjects can be recorded or overwritten is preferable.

The data stored in the reference data storage 450 is imaging data (hereinafter referred to as reference data) which needs to be referenced at least when the state of the disease associated with AMPA receptors is diagnosed. An example of the reference data is the expression level of the AMPA receptors in a healthy subject or a model subject. Since the brain part which needs to be referenced differs depending on the individual disease, the reference data for each brain part is stored. Examples thereof include frontal lobe, dentate cortex, hippocampus, amygdala, putamen, cerebellum, bridge, and the like, and the reference data corresponds to the data R in the metadata. Hence, the reference data is stored such that, when the region X is specified as the referenced data, the reference data for the corresponding part can be output.

Then, the reference data storage 450 transmits, to the checking unit 350, the corresponding reference data

according to the request command generated in the selection unit 410 (first selection unit 410).

The reference data is not limited to a healthy subject or a model subject, and may be the expression level of the AMPA receptors in a typical example of the disease. Examples of the disease include depression, stress disorder, Alzheimer's disease, Parkinson's disease, age-related memory disorder, intrinsic sleep disorder, and the like, and the disease corresponds to the data S in the metadata. Hence, the reference data is stored such that, when the disease name is specified as data to be referenced, typical reference data for the corresponding disease can be output.

<3> Output terminal 500

The output terminal 500 has the function of receiving data from the server 300 so as to display it, and, specifically, the output terminal 500 includes a reception unit 553, a separation unit 520, an instruction display unit 540, and a metadata display unit 530.

The reception unit 553 receives the data package transmitted from the server 300 to the output terminal 500, and an existing communication terminal can be used as it is. When the data package is encrypted, the data is decoded whereas when the header, the footer, the synchronous signal, and the error correction code are provided, they are removed and a data column is generated.

The separation unit 520 separates the instruction data and the metadata from the data described above. Then, the separation unit 520 transmits the instruction data to the instruction display unit 540 and transmits the metadata to the metadata display unit 530. The instruction display unit 540 is, for example, a liquid crystal display, and displays the instruction data in a visually recognizable form such as characters or images. The metadata display unit 530 is a liquid crystal display and displays the metadata in a visually recognizable form such as characters or images. Although the instruction display unit 540 and the metadata display unit 530 are display devices (such as liquid crystal displays) which are different from each other, the displays may be produced in separate display regions or produced so as to be superimposed as a picture-in-picture (PinP) in a single liquid crystal display. In a single liquid crystal display incorporating a touch sensor, the displays of two screens may be switched by a flick operation.

The input terminal 200, the server 300 (including the information production unit 310 and the database 400), and the output terminal 500 are described above. The system 1000 (unillustrated) of the present invention includes the input terminal 200, the server 300, and the output terminal 500. Although they are connected to each other with such a communication line that they can perform transmission and reception, they do not need to be constantly connected, and as necessary, any one of them may issue a command such that they are connected at an arbitrary time. As long as the installation locations of the input terminal 200, the server 300, and the output terminal 500 are in such an environment that the connection can be established with a communication line, there is no restriction, and they may be installed remotely. A plurality of input terminals 200 and a plurality of output terminals 500 may be connected to one server 300.

Next, a preferred configuration of the system 1000 of the present invention will be likewise described with reference to Fig. 1. In addition to the most basic configuration described above, an ID generation unit 340 and a second transmission unit 323 are provided within the server 300, and a reception unit 232 and an ID authentication unit 240 are provided within the input terminal 200. It is intended that mutual authentication be established by the ID generation unit 340 within the server 300 and the ID authentication unit 240 within the input terminal 200. Specifically, in the ID generation unit 340, data from the first transmission unit 323 is input to the separation unit 333, and when the data N is generated from the metadata, the data N is transmitted to the ID generation unit 340. As described previously, the data N is the identification number of the input terminal 200. The ID generation unit 340 encodes the data N with a predetermined function and outputs it to the second transmission unit 323. Then, the second transmission unit 323 transmits the encoded data (referred to as data NN) to the input terminal 200 (the reception unit 232). The reception unit 232 of the input terminal 200 receives the data NN and outputs the data NN to the ID authentication unit 240. Next, the ID authentication unit 240 decodes it with a predetermined function and performs authentication. Specifically, the data NN is decoded such that data N is generated, and the data N is checked with the identification number (data N) included in the input terminal 200. As a result of the checking, when both of them agree with each other, an authentication relationship is assumed to be established, and thus the input terminal 200 and the server 300 continue the communication whereas when they do not agree with each other, the communication is stopped or a retry of the authentication is performed a limited number of times. Here, the function of the encoding and the function of the decoding are determined in advance. As a typical example, the encoding function is determined to be f(x), and the decoding function is determined to be $f^{-1}(x)$, which is the inverse function of the encoding function.

A further preferred configuration of the system of the present invention will next be described with reference to Fig. 3. Fig. 3 is a diagram showing a preferred form of the system 1000 of the present invention and shows the same configuration as in Fig. 1 except that a second database 600 is provided.

[Second database 600]

The second database 600 includes a second selection unit 610 and an instruction data storage 650. The second selection unit 610 and the instruction data storage 650 are coupled to each other in series.

The second selection unit 610 generates a request command according to data fed from the separation unit 333 and outputs it to the instruction data storage 650. For example, when the second selection unit 610 receives the data R from the separation unit 333, the second selection unit 610 searches instruction data on a part which is described so as to generate a command that is output.

In the instruction data storage 650, data is stored in which the distribution and/or the expression level of the AMPA receptors in the brain of the primate organism, the state of the disease associated with AMPA receptors in the brain of the primate organism, and information on the type, the dosage, and/or the usage of a drug recommended to the subject are associated with each other. As in the first database 400, the form of the storage may be a read-only memory (ROM) or a rewritable random-access memory (RAM), and the random-access memory is preferable.

The data stored in the instruction data storage 650 is at least the information on the type, the dosage and/or the usage of the drug recommended to the subject (hereinafter referred to as instruction data). An example of the instruction data is a drug that is recommended to the disease and includes the usage and dosage thereof. Since the recommended drug differs depending on the individual disease, the instruction data for each disease is stored.

Then, the instruction data storage 650 transmits, to the instruction unit 360, the corresponding instruction data according to the request command generated in the second selection unit 610. Depending on in which one the expression level of the AMPA receptors is higher or lower as determined by the checking unit 350, for example, the data A described above is "the prescription of an AMPA receptor antagonist AA," the data B is "the prescription of an AMPA receptor promoter BB," and the data C is "no prescription," and these pieces of data A to C are output to the instruction unit 360 as a set. Here, when information to be considered in the instruction is needed in addition to in which one the expression level of the AMPA receptors is higher or lower, the second selection unit can request the separation unit 333 to provide data. For example, when in the presentation of the drug, the usage and the dosage are presented, the data S is requested, and thus information on the gender, the age, the weight, and the medication history of the subject is obtained. Then, with consideration given to the information on the subject, the usage and the dosage are determined with a predetermined algorithm and are output as additional data.

The first database 400 is connected to the checking unit 350 and the second database 600 is connected to the instruction unit 360, and thus the variation of information which is presented on the diagnosis and the prescription is expanded, with the result that it is possible to present highly accurate information. Information on the result of the therapy or the prophylaxis of the human subject by the administration corresponding to the type, the dosage, and/or the usage of the drug that is recommended is constantly fed back, and thus both the reference data storage 450 and the instruction data storage 650 are updated, with the result that it is possible to enhance the accuracy of recommendations.

EXAMPLES

(Synthesis Example 1)

(Synthesis of K-1 and K-2)

2-[2,6-Difluoro-4-({2-[(phenylsulfonyl)amino]ethyl}thio)phenoxy]acetamide (K-1, PEPA) and {4-[2-(benzenesulfonyl-methyl-amino)-ethylsulfanil]-2,6-difluoro-phenoxy}-acetamide (K-2) were synthesized by the following scheme. The $^1$H NMR spectrum of each compound was recorded with Bruker Avance III 400 MHz or Varian Mercury plus-300 MHz by using TMS as an internal reference.

[Chem. 6]

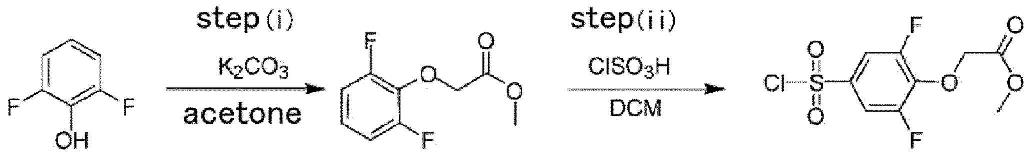


1 2 3

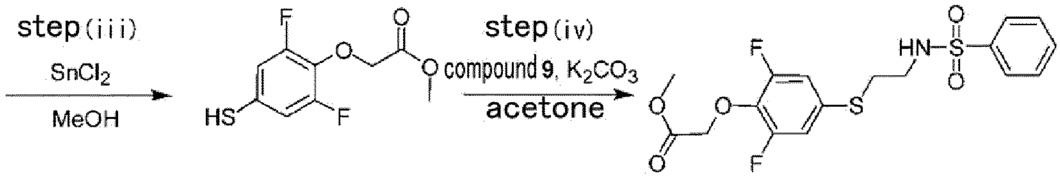


4 5

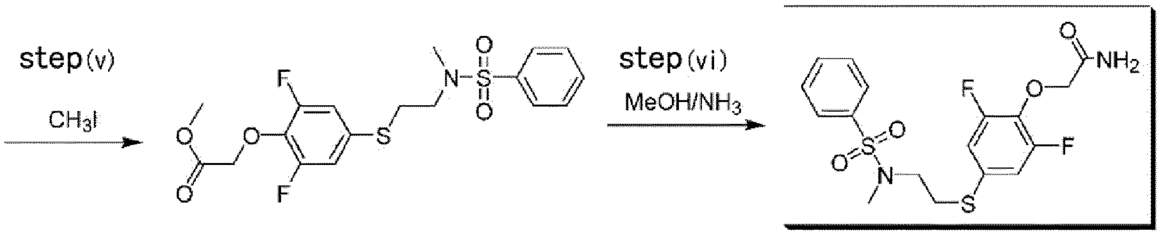


6 K-2

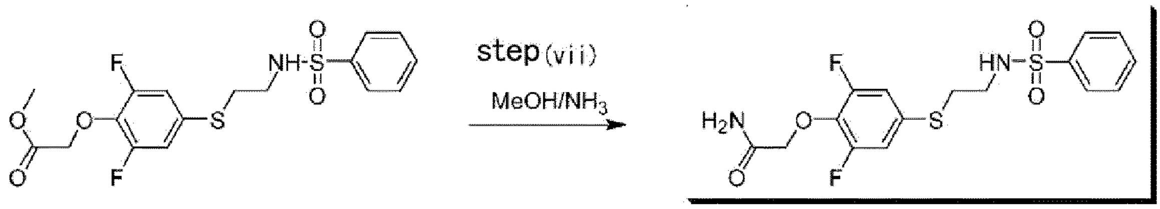


5 K-1

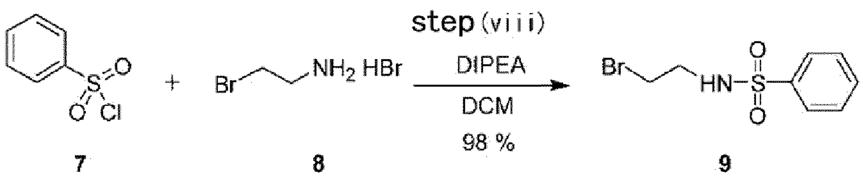


7 8 9

Step (i): Synthesis of (2,6-difluoro-phenoxy)-acetic acid methyl ester (2)

[Chem. 7]

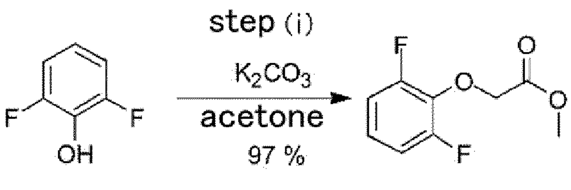


1 2

To an acetone solution (75 mL) of 2,6-difluoro-phenol (1) (5.00 g, 38.5 mmol), $K_2CO_3$ (8.40 g, 60.7 mmol) was added, and after 10 minutes, methyl bromoacetate (5.80 g, 38.5 mmol) was added to the reaction solution. The reaction solution was stirred at room temperature overnight. After the completion of the reaction, the reaction mixture solution was poured in a mixture solution of concentrated hydrochloric acid (20 mL) and ice water (200 ml), the resultant mixture was extracted with EtOAc (100 mL × 3), the organic layer was washed with water (50 mL × 3) and brine (100 mL × 2), dried with $Na_2SO_4$, and filtered. Thereafter, the resultant product was condensed under vacuum to thereby obtain a compound (2) as yellow oil (7.50 g, 97%).
$^1$H NMR (300 MHz, $CDCl_3$) : δ 3.78 (s, 3H), 4.74 (s, 2H), 6.86-6.99 (m, 3H).

Step (ii): Synthesis of (4-chlorosulfonyl-2,6-difluoro-phenoxy)-acetic acid methyl ester (3)

[Chem. 8]

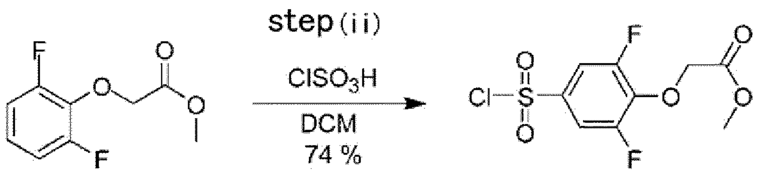


2 3

To a DCM solution of (2,6-difluoro-phenoxy)-acetic acid methyl ester (2) (5.00 g, 24.7 mmol), chlorosulfonic acid (17.2 g, 24.7 mmol) was added dropwise in an ice bath, and the reaction solution was heated to 45°C and stirred for 1.5 hours. After the completion of the reaction, the reaction mixture solution was quenched with 50 mL of ice water, the organic layer was separated and washed with water (300 mL × 3). The resultant product was dried with $Na_2SO_4$ and filtered, and then was condensed under vacuum, thereby obtaining a compound (3) as yellow oil (5.50 g, 74%).
$^1$H NMR (300 MHz, $CDCl_3$):δ 3.81 (s, 3H), 4.96 (s, 2H), 7.61 (s, 1H), 7.64 (s, 1H).

Step (iii): Synthesis of (2,6-difluoro-4-mercapto-phenoxy)-acetic acid methyl ester (4)

[Chem. 9]

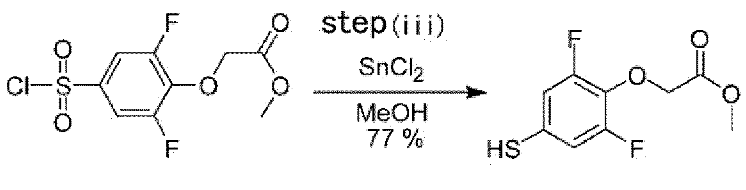


3 4

To a mixture solution of (4-chlorosulfonyl-2,6-difluoro-phenoxy)-acetic acid methyl ester (3) (5.50 g, 18.3 mmol), $SnCl_2$ (14.5 g, 64.2 mmol), and methanol (50 mL), concentrated hydrochloric acid (25 mL) was added dropwise. The reaction mixture solution was heated to reflux temperature and stirred for 2 hours. After cooling, the reaction mixture solution was poured to ice water (100 mL) and the resultant mixture was extracted with DCM (100 mL × 3). An organic layer was washed with water (100 mL × 3) and brine (100 mL × 2), was dried with $Na_2SO_4$, was filtered and was then condensed under vacuum, and thus a compound (4) was obtained as a yellow oil (3.30 g, 77%). $^1H$ NMR (300 MHz, $CDCl_3$) : δ 3.52 (s, 1H), 3.77 (s, 3H), 4.71 (s, 2H), 6.83 (s, 1H), 6.86 (s, 1H).

Step (iv): Synthesis of [4-(2-benzenesulfonylamino-ethylsulfanil)-2,6-difluoro-phenoxy]-acetic acid methyl ester (5)

[Chem. 10]

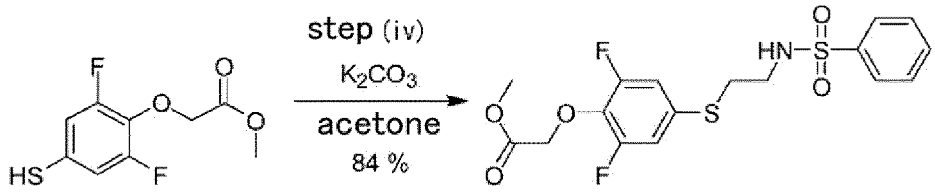


4 5

A mixture solution of (2,6-difluoro-4-mercapto-phenoxy)-acetic acid methyl ester (4) (1.10 g, 4.7 mmol), potassium carbonate (778 mg, 5.6 mmol), and acetone (15 mL) was stirred under $N_2$ at room temperature for 20 minutes. To the reaction solution, N-(2-bromo-ethyl)-benzenesulfonamide (9) (1.30 g, 4.90 mmol) was added, and the reaction solution was stirred at room temperature overnight. After the completion of the reaction, the reaction solution was poured to 30 mL of 2N HCl and the resultant product was extracted with EtOAc (50 mL × 3). The organic layer was washed with water (50 mL × 3) and brine (100 mL × 2), dried with $Na_2SO_4$, filtered, and then condensed under vacuum, thereby obtaining a residue. The residue was refined by silica gel column chromatography (PE/EA = 10/1 to 3/1, v/v) to thereby obtain a compound (5) as yellow oil (1.60 g, 84%).
$^1$HNMR (300 MHz, $CDCl_3$) : δ 2.95 (t, J = 6.6 Hz, 2H), 3.12 (q, J = 6.3 Hz, 2H), 3.78 (s, 3H), 4.72 (s, 2H), 5.20 (t, J = 6.0 Hz, 1H), 6.76-6.83 (m, 2H), 7.47-7.60 (m, 3H), 7.82-7.84 (m, 2H).

Step (v): Synthesis of {4-[2-(benzenesulfonyl-methyl-amino)-ethylsulfanil]-2,6-difluoro-phenoxy}-acetic acid methyl ester (6)

[Chem. 11]

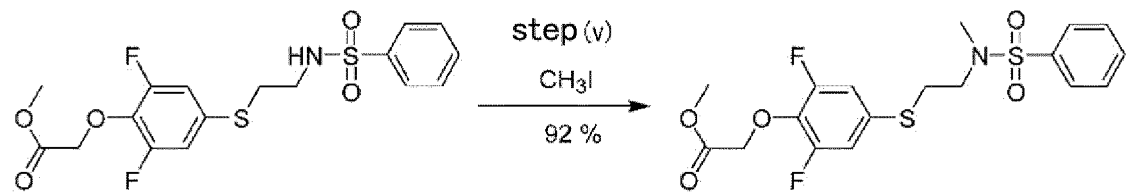


5 6

To 10 mL of a DMF mixture solution of [4-(2-benzenesulfonylamino-ethylsulfanil)-2,6-difluoro-phenoxy]-acetic acid methyl ester (5) (300 mg, 0.72 mmol) and $K_2CO_3$ (397 mg, 2.88 mmol), MeI (255 mg, 1.80 mmol) was added at 0°C. Thereafter, the reaction solution was stirred at room temperature for 1 hour. After the completion of the reaction, the reaction solution was diluted with 20 ml of water and extracted with EtOAc (30 mL × 3). The organic layer was

washed with water (30 mL × 3) and brine (20 mL × 2), dried with $Na_2SO_4$, filtered, and then condensed under vacuum, thereby obtaining a compound (6) as yellow oil (285 mg, 92%).
$^1$HNMR (300 MHz, $CDCl_3$) : δ 2.81 (s, 3H), 3.04-3.09 (m, 2H), 3.19-3.24 (m, 2H), 3.79 (s, 3H), 4.74 (s, 2H), 6.90-6.94 (m, 2H), 7.50-7.60 (m, 3H), 7.74-7.77 (m, 2H).

Step (vi): Synthesis of {4-[2-(benzenesulfonyl-methyl-amino)-ethylsulfanil]-2,6-difluoro-phenoxy}-acetamide (K-2)

[Chem. 12]

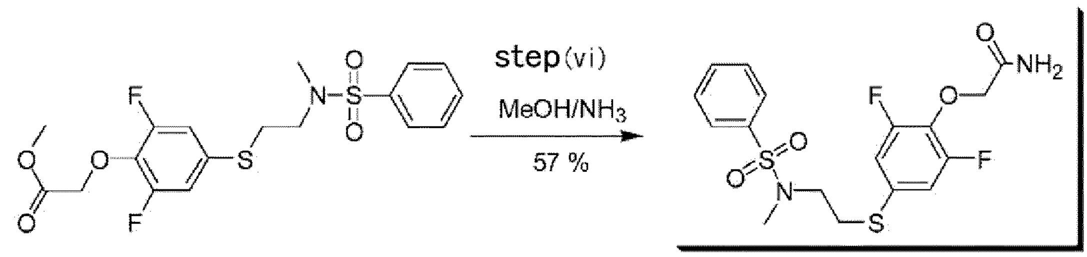


6 K-2

A mixture solution of {4-[2-(benzenesulfonyl-methyl-amino)-ethylsulfanil]-2,6-difluoro-phenoxy}-acetic acid methyl ester (6) (40.0 mg, 0.09 mmol) and 13 mL of 4N $MeOH/NH_3$ was stirred at room temperature for 18 hours. After the completion of the reaction, the reaction mixture solution was condensed under vacuum, thereby obtaining a residue. The residue was refined by preparative HPLC to thereby obtain compound (K-2) as a white solid (22.0 mg, 57%).
$^1$HNMR (300 MHz, CDCl3): δ 2.82 (s, 3H), 3.08-3.13 (m, 2H), 3.20-3.26 (m, 2H), 4.58 (s, 2H), 6.93-6.99 (m, 2H), 7.50-7.63 (m, 3H), 7.75-7.78 (m, 2H).

Synthesis of {4-[2-(benzenesulfonyl-methyl-amino)-ethylsulfanil]-2,6-difluoro-phenoxy}-N,N-dimethyl-acetamide (K-4)

[Chem. 13]

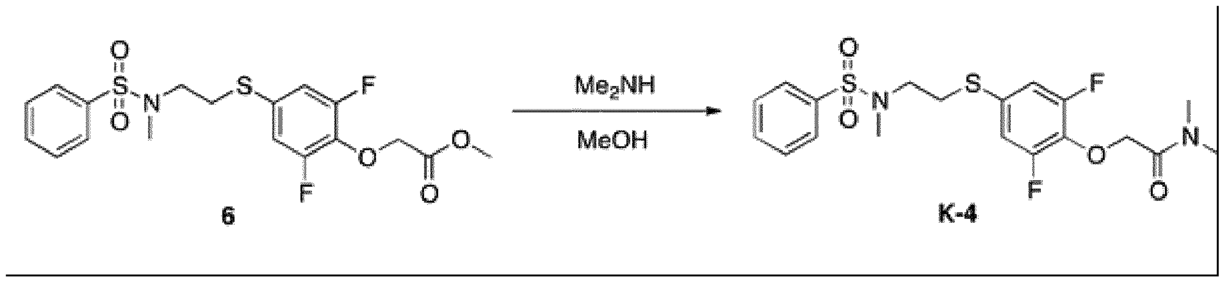


6 K-4

A mixture solution of {4-[2-(benzenesulfonyl-methyl-amino)-ethylsulfanil]-2,6-difluoro-phenoxy}-acetic acid methyl ester (6) (9 g, 27.7 mmol) and 240 mL of 5N dimethylamine/methanol was stirred at room temperature for 2 hours. After the completion of the reaction, the mixture solution was condensed under reduced pressure and was refined by silica gel chromatography to thereby obtain a compound (K-4) as a colorless oily substance (12 g, 97%).
$^1$HNMR ($CDCl_3$, 400 MHz): δ 2.82 (s, 3H), 2.98 (s, 3H), 3.05-3.09 (m, 5H), 3.19-3.22 (m, 2H), 4.80 (s, 2H), 6.89-6.92 (m, 2H), 7.53-7.55 (m, 2H), 7.58-7.60 (m, 1H), 7.75-7.77 (m, 2H). LCMS [mobile phase: analysis for 6.5 minutes with 55% water (0.05% formic acid) and 45% acetonitrile (0.05% formic acid)] purity > 95%, holding time = 3.445 minutes; MS Calcd.: 444.5; MS Found: 445.0 $[M+1]^+$.

Step (vii): Synthesis of 2-[2,6-difluoro-4-({2-[(phenylsulfonyl)amino]ethyl}thio)phenoxy]acetamide (K-1)

[Chem. 14]

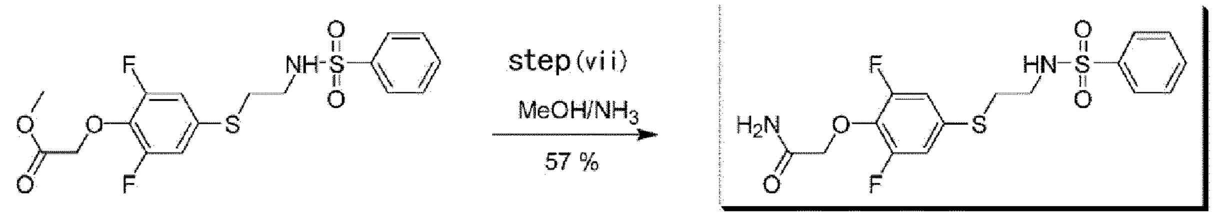


5

K-1

A mixture solution of [4-(2-benzenesulfonylamino-ethylsulfanil)-2,6-difluoro-phenoxy]-acetic acid methyl ester (5) (200 mg, 0.48 mmol) and 10 mL of 4N MeOH/$NH_3$ was stirred at room temperature for 18 hours. After the completion of the reaction, the reaction mixture solution was condensed under vacuum, thereby obtaining a residue. The residue was refined by preparative HPLC to thereby obtain a compound K-1 as a white solid (110 mg, 57%).
$^1$HNMR (300 MHz, $CDCl_3$ + $D_2O$) : δ 2.97-3.02 (m, 2H), 3.11-3.16 (m, 2H), 4.56 (s, 2H), 6.82-6.90 (m, 2H), 7.48-7.61 (m, 3H), 7.82-7.87 (m, 2H).

Synthesis of {4-[2-(benzenesulfonyl-amino)-ethylsulfanil]-2,6-difluoro-phenoxy}-N,N-dimethyl-acetamide (K-5)

[Chem. 15]

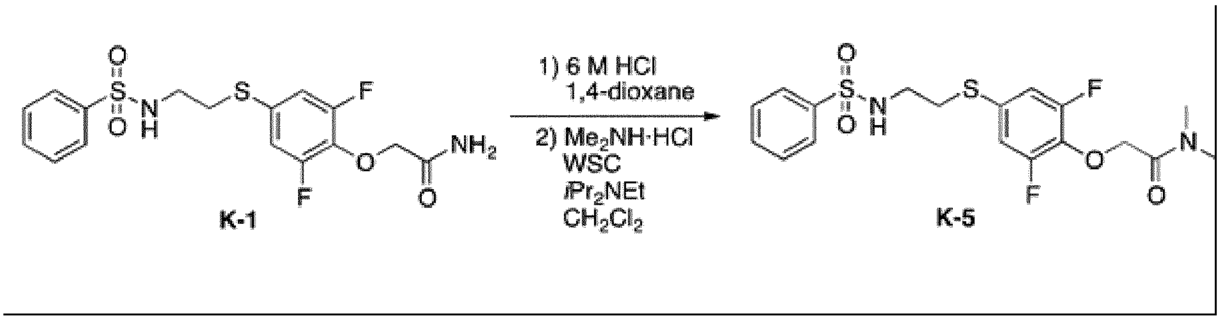


K-1

K-5

K-1 (159.4 mg, 0.396 mmol) was dissolved in 1,4-dioxane (3 mL), was subjected to addition of 6 M hydrochloric acid (1 mL), and was heated at 80°C for 2 hours. The reaction was stopped by the addition of water, the resultant product was extracted with ethyl acetate, and then a saturated sodium bicarbonate aqueous solution was added to the ethyl acetate solution, the product was extracted in a water layer, hydrochloric acid was added again so as to make the product acidic, and then the product was extracted again with ethyl acetate and was washed with saturated saline, and thereafter water was removed by the addition of anhydrous sodium sulfate. The solution was distilled under reduced pressure, a residue obtained was dissolved in a dichloromethane solution, dimethylamine · hydrochloride (94.5 mg), WSC·HCl (150.8 mg) and diisopropylethylamine (265 μL) were added to this solution, and the solution was stirred at room temperature for 2 hours. The reaction was stopped by the addition of hydrochloric acid thereto, and the resultant product was extracted with ethyl acetate. This was washed with a saturated sodium bicarbonate aqueous solution and saturated saline, and the solvent was distilled under reduced pressure. The residue was refined by silica gel chromatography to thereby obtain the target K-5 as a white solid (148.1 mg, 84%).

Step (viii): Synthesis of N-(2-bromo-ethyl)-benzenesulfonamide (9)

[Chem. 16]

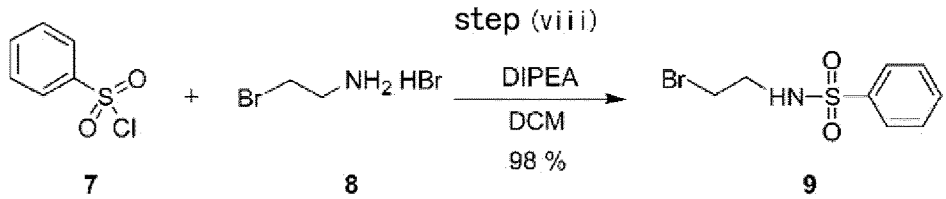


7 8 9

To a DCM (30 mL) solution of benzenesulfonyl chloride (7) (3.00 g, 17.0 mmol) and 2-bromoethylamine hydrobromide (8) (3.80 g, 18.7 mmol), DIPEA (4.80 g, 37.4 mmol) was added in an ice bath. Thereafter, the reaction solution was stirred at the same temperature for 1.5 hours. After the completion of the reaction, the reaction solution was diluted with 20 mL of water and extracted with EtOAc (30 mL × 3). The organic layer was washed with water (30 mL × 3) and brine (20 mL × 2), dried with $Na_2SO_4$, filtered, and then condensed under vacuum, thereby obtaining a compound (9) as a white solid (4.40 g, 98%).
$^1$HNMR (300 MHz, $CDCl_3$) : δ 3.36-3.39 (m, 4H), 5.09 (s, 1H), 7.50-7.63 (s, 3H), 7.87-7.89 (s, 2H).

(Synthesis Example 2)

(Synthesis of M-1, M-2, and M-3)

According to the following scheme, 2-[4-(2-benzenesulfonylamino-ethylsulfanil)-2,6-difluoro-phenoxy]-N-methyl-acetamide (M-1), 2-{2,6-difluoro-4-[2-(4-fluoro-benzenesulfonylamino)-ethylsulfanil]-phenoxy}-acetamide (M-2), and 2-{2,6-difluoro-4-[2-(4-methyl-benzenesulfonylamino)-ethylsulfanil]-phenoxy}-acetamide (M-3) were synthesized. The $^1$H NMR spectrum of each compound was recorded with Varian Mercury plus-400 MHz by using TMS as an internal reference. The following one was used as LCMS: Agilent 1200A, column: C18; column size: 4.6 * 50 minutes; mobile phase: B (ACN), A (water of 0.05% $NH_3$); gradient (B%): as described in Synthesis Example.

[Chem. 17]

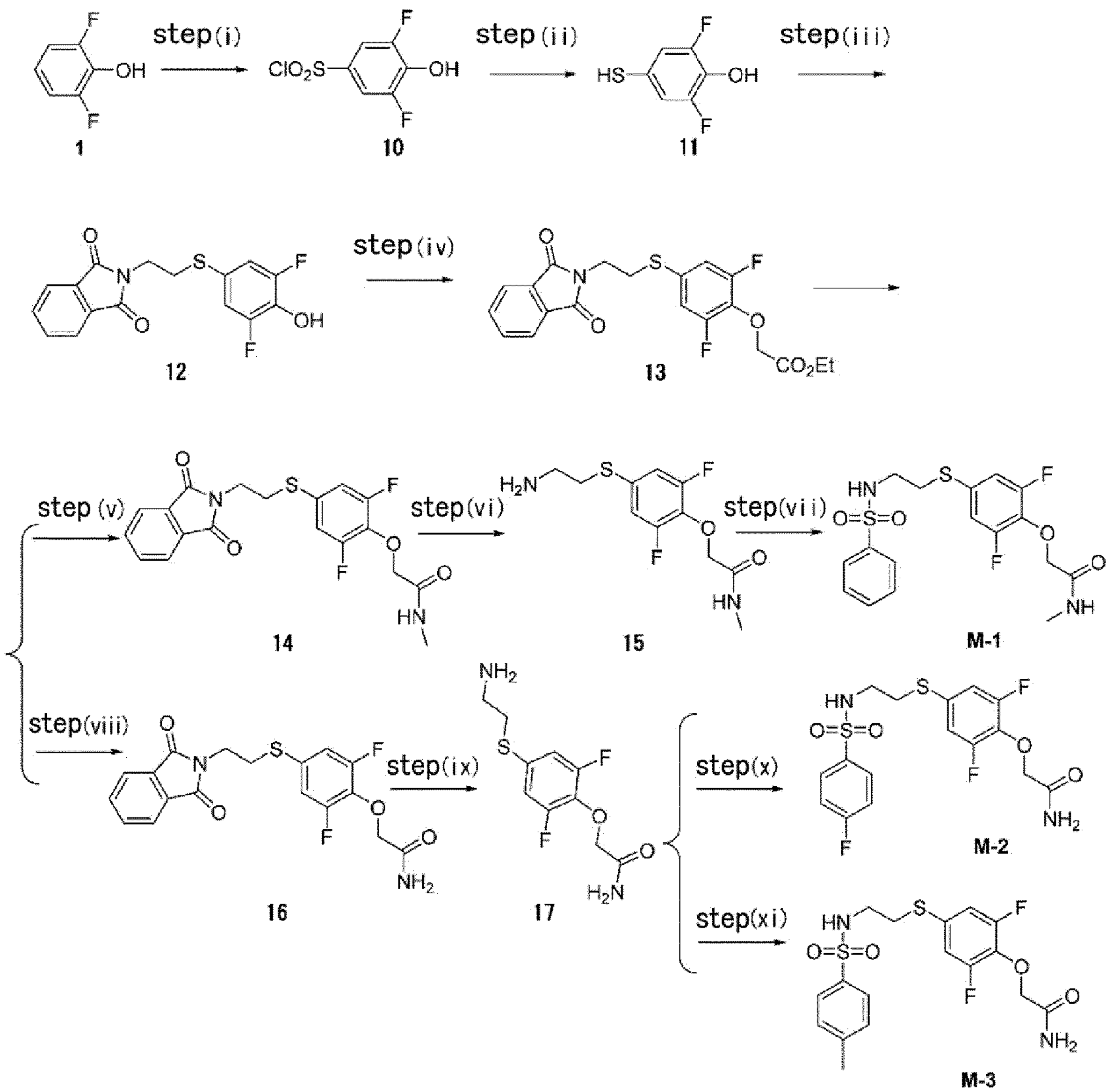


Step (i): Synthesis of 3,5-difluoro-4-hydroxy-benzenesulfonyl chloride (10)

[Chem. 18]

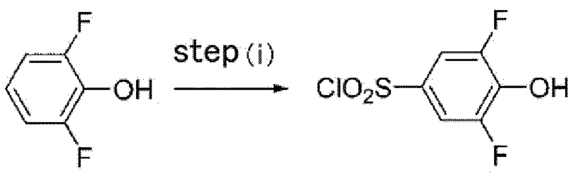


1 10

To a DCM (50 mL) solution of the compound (1) (5.0 g), chlorosulfonic acid (15 mL) was added dropwise. The reaction mixture solution was stirred at 25°C for 1 hour. The TLC (petroleum ether/EtOAc: 20/1) indicated the completion of the reaction. Thereafter, the solution was poured to crushed ice. An organic layer was separated and filtered through Celite. The filtrate was dried and distilled under vacuum to thereby obtain a compound (10) as yellow oil: 5 g (57%).

$^1$H-NMR (400 MHz, $CDCl_3$) : δ 6.30 (s, 1H), 7.66-7.68 (m, 2H) .

Step (ii): Synthesis of 2,6-difluoro-4-mercapto-phenol (11)

[Chem. 19]

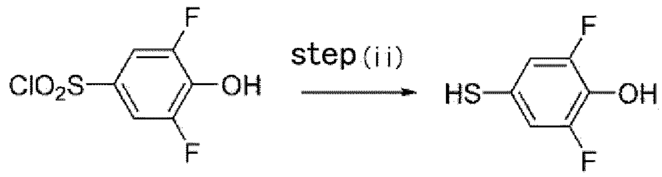


10 11

To a DCM (3 mL) solution of triphenyl phosphine (3.4 g, 13.1 mmol) and DMF (0.1 mL), a DCM (4 mL) solution of the compound (10) (1.0 g, 4.3 mmol) was added dropwise at 0°C under nitrogen. The reaction mixture solution was stirred at 25°C for 2 hours. Thereafter, 1N HCl was added to the mixture solution to adjust pH to 3 and the mixture solution was extracted with EA. The organic layer was dried with sodium sulfate to remove the solvent, thereby obtaining a crude compound (11) as yellow oil.

Step (iii): Synthesis of 2-[2-(3,5-difluoro-4-hydroxy-phenylsulfanyl)-ethyl]-isoindole-1,3-dione (12)

[Chem. 20]

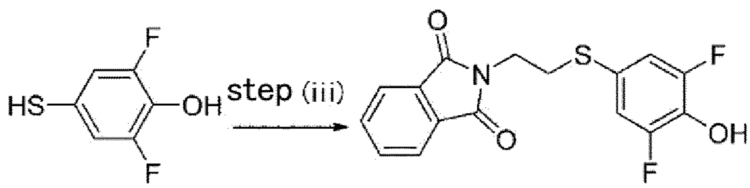


11 12

To a DMF (100 mL) solution of the crude compound (11) (14 g, 86 mmol), 2-(2-bromo-ethyl)-isoindole-1,3-dione (13.2 g, 51.8 mmol) and $K_2CO_3$ (23.8 g, 172.4 mmol) were added. The mixture solution was stirred at 25°C overnight. Thereafter, 1N HCl was added to the mixture solution to adjust pH to 3 and the mixture solution was extracted with EA. The organic layer was dried with sodium sulfate to remove the solvent, thereby obtaining a compound (12) as a yellow solid (8 g, 27%).

$^1$H-NMR (400 MHz, DMSO-d6): δ 3.20-3.23 (t, 2H), 3.75-3.79 (t, 2H), 7.08-7.10 (d, 2H), 7.84 (s, 4H).

Step (iv): Synthesis of {4-[2-(1,3-dioxo-1,3-dihydro-isoindole-2-yl)-ethylsulfanil]-2,6-difluoro-phenoxy}-ethyl acetic acid ester (13)

[Chem. 21]

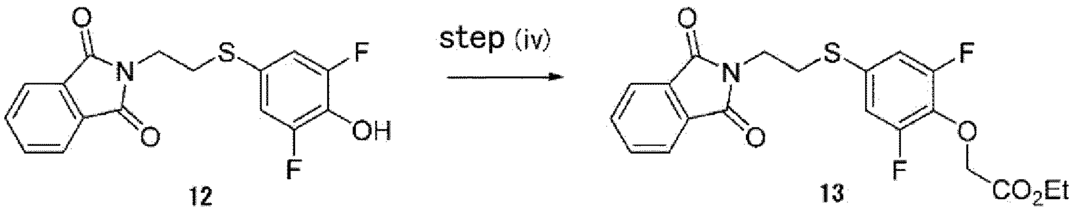


12

13

To a solution obtained by dissolving the compound (12) (5.0 g, 15 mmol) in DMF (30 mL), 3-bromo-propionic acid ethyl ester (2.5 g, 15 mmol) and $K_2CO_3$ (3.0 g, 22.5 mmol) were added. The mixture solution was stirred at 25°C overnight. Thereafter, the mixture solution was extracted with EA. The organic layer was dried with sodium sulfate to remove the solvent, thereby obtaining a compound (13) as a white solid (6 g, 97%).

$^1$H-NMR (400 MHz, $CDCl_3$) : δ 1.21-1.24 (t, 3H), 3.11-3.14 (t, 2H), 3.84-3.88 (t, 2H), 4.18-4.20 (d, 2H), 4.61 (s, 2H), 6.91-6.94 (d, 2H), 7.66-7.68 (m, 2H), 7.77-7.79 (m, 2H).

Step (v): Synthesis of 2-{4-[2-(1,3-dioxo-1,3-dihydro-isoindole-2-yl)-ethylsulfanil]-2,6-difluoro-phenoxy}-N-methyl-acetamide (14)

[Chem. 22]

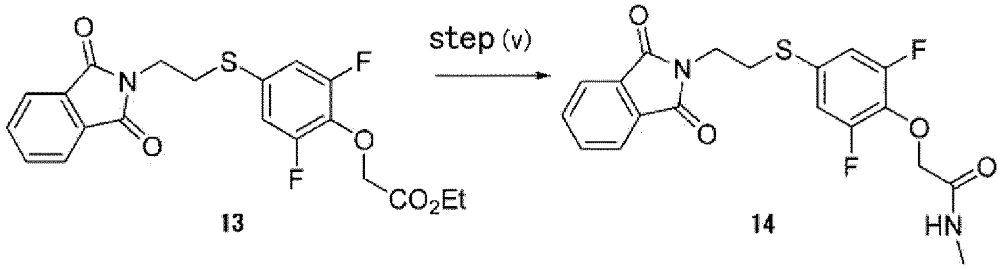


13

14

A methylamine alcohol solution (10 mL) of the compound (13) (0.5 g, 1.2 mmol) was stirred at 100°C for 30 minutes. Thereafter, the mixture solution was condensed to thereby obtain a crude compound (14) as yellow oil (1 g).

Step (vi): Synthesis of 2-[4-(2-amino-ethylsulfanil)-2,6-difluoro-phenoxy]-N-methyl-acetamide (15)

[Chem. 23]

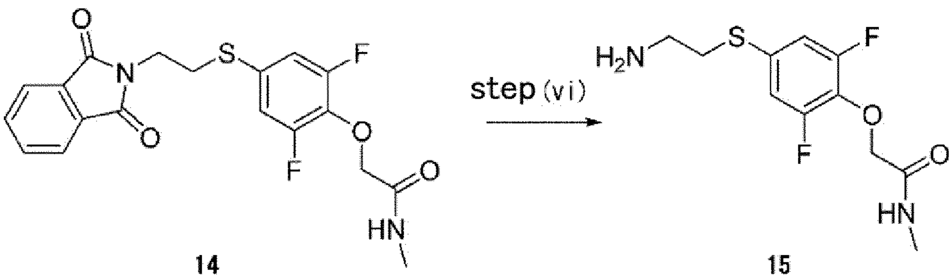


14

15

Hydrazine hydrate (0.25 g, 5 mmol) was added to an EtOH (10 mL) solution of the crude compound (14) (1 g,

2.5 mmol) at 90°C. The solution was heated to 90°C, stirred for 30 minutes, and then cooled at room temperature. The resultant product was filtered and washed with EtOH. The organic layer was dried with sodium sulfate and condensed to thereby obtain a crude compound (15) as yellow oil (0.5 g).

Step (vii): Synthesis of 2-[4-(2-benzenesulfonylamino-ethylsulfanil)-2,6-difluoro-phenoxy]-N-methyl-acetamide (M-1)

[Chem. 24]

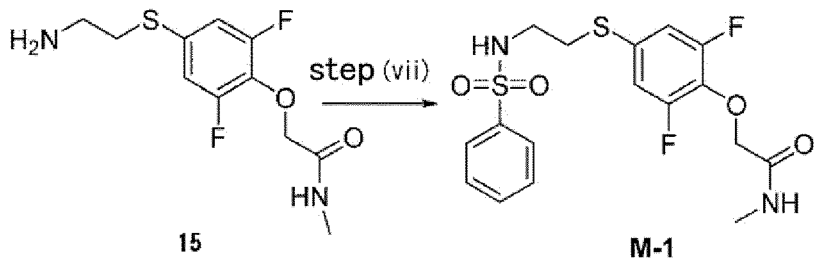


15 M-1

Benzenesulfonyl chloride (0.4 g, 2.2 mmol) and triethylamine (0.2 g, 2.2 mmol) were added to a DCM (10 mL) solution of the crude compound (15) (0.5 g, 1.8 mmol). Thereafter, the mixture solution was stirred at 25°C for 1 hour and extracted with EA. The organic layer was dried with sodium sulfate and condensed. The residue was refined by flash chromatography to thereby obtain a compound (M-1) as a white solid (20 mg).
$^1$H-NMR (400 MHz, DMSO_d6): δ 2.65-2.66 (d, 3H), 2.91-2.94 (t, 2H), 2.01-3.04 (t, 2H), 4.50 (s, 2H), 7.10-7.12 (d, 2H), 7.57-7.65 (m, 3H), 7.76-7.78 (d, 2H), 7.92-7.95 (t, 1H), 8.05 (s, 1H). MS: m/z 417 $(M+1)^+$
LCMS [mobile phase: 5% water (0.1% $NH_4OH$) and 95% $CH_3CN$ from 90% water (0.1% $NH_4OH$) and 10% $CH_3CN$, 6.0 minutes, finally 0.5 minutes under these conditions] purity 97.4%, Rt = 3.341 minutes; MS Calcd.: 416; MS Found: 417 $([M+1]^+)$.

Step (viii): Synthesis of 2-{4-[2-(1,3-dioxo-1,3-dihydro-isoindole-2-yl)-ethylsulfanil]-2,6-difluoro-phenoxy}-acetamide (16)

[Chem. 25]

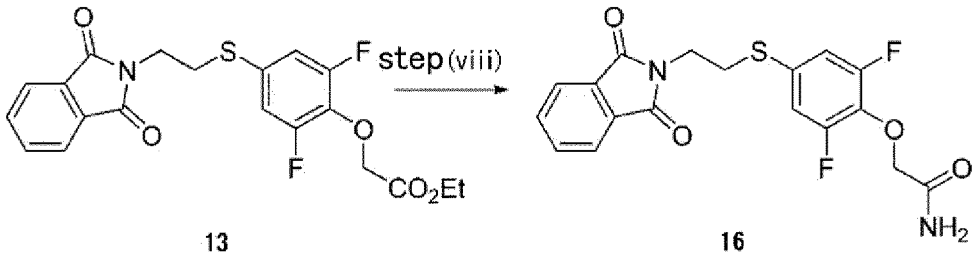


13 16

An $NH_3$/EtOH (100 mL) solution of the compound (13) (5.0 g, 11.8 mmol) was stirred at 25°C for 2 hours. Thereafter, the solution was condensed to thereby obtain a crude compound (16) as yellow oil (6.0 g).

Step (ix): Synthesis of 2-[4-(2-amino-ethylsulfanil)-2,6-difluoro-phenoxy]-acetamide (17)

[Chem. 26]

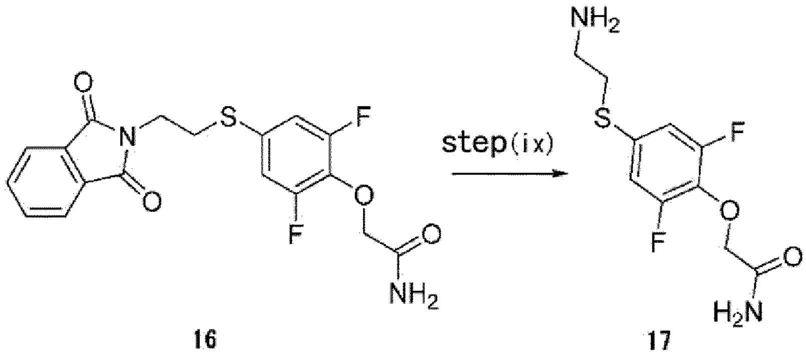


16 17

Hydrazine hydrate (1.5 g, 30 mmol) was added to an EtOH (50 mL) solution of the crude compound (16) (6.0 g, 15.3 mmol) at 90°C. The solution was heated to 90°C, stirred for 30 minutes, and then cooled at room temperature. The resultant product was filtered and washed with EtOH. The organic layer was dried with sodium sulfate and condensed to thereby obtain a crude compound (17) as yellow oil (4.0 g).

Step (x): Synthesis of 2-{2,6-difluoro-4-[2-(4-fluoro-benzenesulfonylamino)-ethylsulfanil]-phenoxy}-acetamide (M-2)

[Chem. 27]

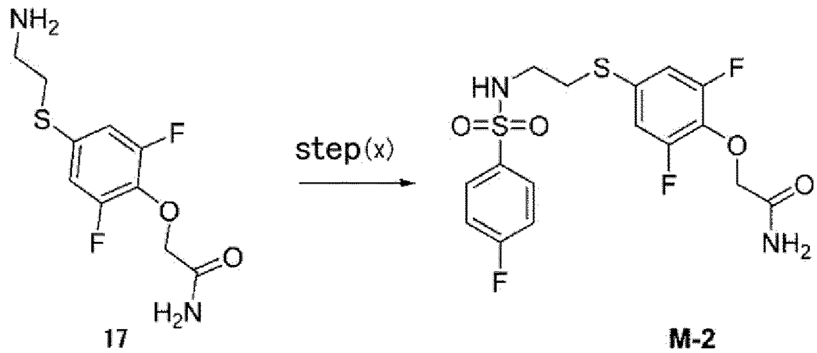


17 M-2

4-Fluoro-benzenesulfonyl chloride (0.4 g, 2.3 mmol) and triethylamine (0.2 g, 2.2 mmol) were added to a DMF (10 mL) solution of a crude compound 17 (0.5 g, 1.9 mmol). Thereafter, the mixture solution was stirred at 25°C for 1 hour and extracted with EA. The organic layer was dried with sodium sulfate and condensed. The residue was refined by flash chromatography to thereby obtain a compound (M-2) as a white solid (20 mg).
$^1$H-NMR (400 MHz, DMSO_d6): δ 2.92-2.95 (t, 2H), 3.01-3.04 (t, 2H), 4.45 (s, 2H), 7.09-7.11 (d, 2H), 7.40-7.44 (m, 3H), 7.47 (s, 1H), 7.81-7.85 (m, 2H), 7.95-7.98 (t, 1H). MS: m/z 421 $(M+1)^+$
LCMS [mobile phase: 5% water (0.1% $NH_4OH$) and 95% $CH_3CN$ from 90% water (0.1% $NH_4OH$) and 10% $CH_3CN$, 6 minutes, finally 0.5 minutes under these conditions] purity 95.1%, Rt = 3.284 minutes; MS Calcd.: 420; MS Found: 421 $([M+1]^+)$.

Step (xi): Synthesis of 2-{2,6-difluoro-4-[2-(4-methyl-benzenesulfonylamino)-ethylsulfanil]-phenoxy}-acetamide (M-3)

[Chem. 28]

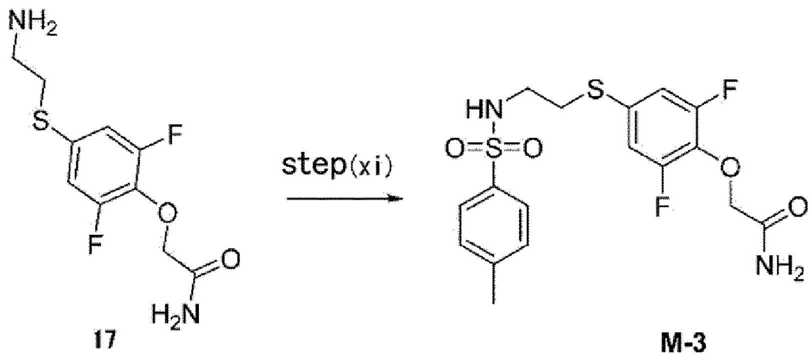


17 M-3

4-Methyl-benzenesulfonyl chloride (0.5 g, 2.3 mmol) and triethylamine (0.2 g, 2.2 mmol) were added to a DMF (10 mL) solution of the crude compound (17) (0.5 g, 1.9 mmol). Thereafter, the mixture solution was stirred at 25°C for 1 hour and extracted with EA. The organic layer was dried with sodium sulfate and condensed. The residue was refined by flash chromatography to thereby obtain a compound (M-3) as a white solid (20 mg).

$^1$H-NMR (400 MHz, DMSO-d6): $\delta$ 2.38 (s, 3H), 2.88-2.91 (t, 2H), 2.99-3.02 (t, 2H), 4.49 (s, 2H), 7.08-7.10 (d, 2H), 7.37-7.48 (m, 4H), 7.64-7.66 (d, 2H), 7.81-7.84 (t, 1H). MS: m/z 417 $(M+1)^+$

LCMS [mobile phase: 5% water (0.1% $NH_4OH$) and 95% $CH_3CN$ from 90% water (0.1% $NH_4OH$) and 10% $CH_3CN$, 6.0 minutes, finally 0.5 minutes under these conditions] purity 96.6%, Rt = 3.365 minutes; MS Calcd.: 416; MS Found: 417 $([M+1]^+)$.

(Synthesis Example 3)

(Synthesis of M-3pre)

2-(2,6-Difluoro-4-((2-(4-(tributylstannyl)phenyl sulfonamide)ethyl)thio)phenoxy)acetamide (M-3pre) was synthesized in accordance with the following scheme. The $^1$H NMR spectrum of each compound was recorded with Bruker Avance III 400 MHz and Bruker Fourier 300 MHz by using TMS as an internal reference. The following one was used as LCMS: quadrupole mass spectrometer, Agilent LC/MSD 1200 series (column: ODS 2000 (50 × 4.6 mm, 5 μm) operated in ES (+) or (-) ionization mode; T = 30°C; flow rate = 1.5 mL/min; detection wavelength: 254 nm.

[Chem. 29]

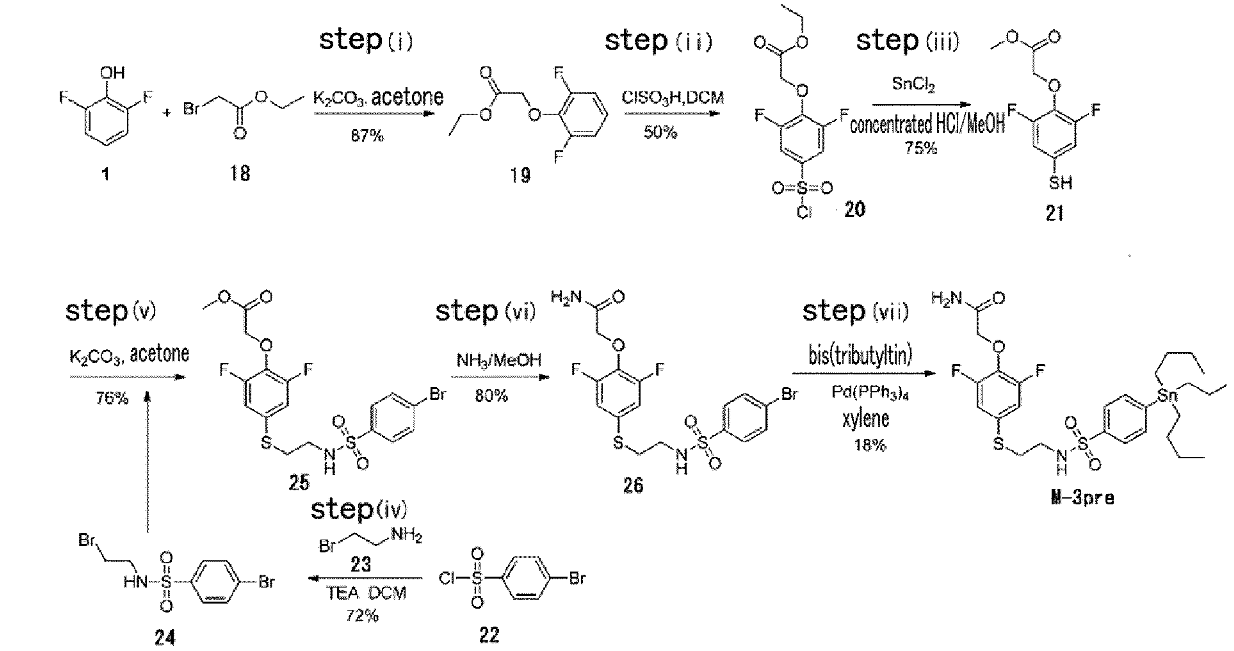


Step (i): Synthesis of ethyl 2-(2,6-difluorophenoxy)acetate (19)

[Chem. 30]

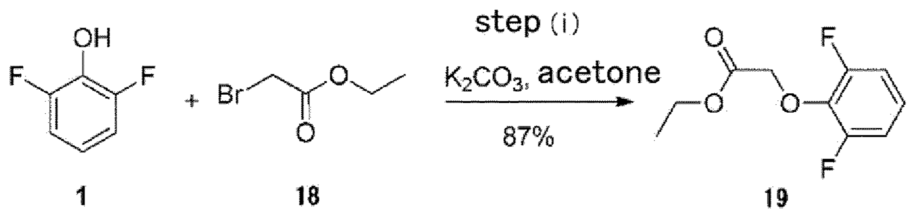


A mixture solution of the compound (1) (39.0 g, 0.30 mol), $K_2CO_3$ (62.0 g, 0.45 mol), the compound (18) (50.1 g, 0.30 mol), and acetone (200 mL) was stirred at room temperature for about 16 hours. The reaction mixture solution was poured to 3% HCl and extracted with ethyl acetate (90 mL × 3). The combined organic layer was dried with sodium sulfate anhydride, filtered, and condensed. The residue was refined by silica gel column chromatography (PE : EA = 10 : 1) to thereby obtain a compound (19) (57 g, 87%) .
$^1$H NMR ($CDCl_3$, 300 MHz) : δ 1.19 (t, J = 7.2 Hz, 3H), 4.17 (q, J = 7.2 Hz, 2H), 4.82 (s, 2H), 7.06-7.13 (m, 3H).

Step (ii): Synthesis of ethyl 2-(4-(chlorosulfonyl)-2,6-difluorophenoxy)acetate (20)

[Chem. 31]

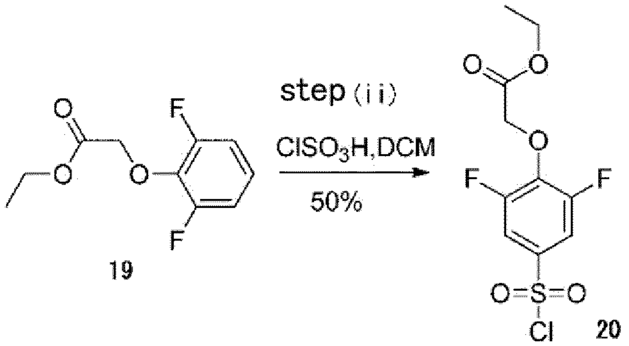


To a DCM (180 mL) solution of the compound (19) (50 g, 0.23 mol), $ClSO_3H$ (106 mL, 1.38 mol) was added at 35°C. The reaction mixture solution was heated to reflux temperature and stirred for about 1.5 hours. Thereafter, the reaction mixture solution was poured to ice. The organic layer was separated, dried, and condensed, thereby obtaining a compound 20 (37 g, 50%) .

$^1H$ NMR ($CDCl_3$, 300 MHz): $\delta$ 1.18 (t, J = 6.9 Hz, 3H), 4.16 (q, J = 6.9 Hz, 2H), 4.83 (s, 2H), 7.18-7.21 (m, 2H).

Step (iii): Synthesis of methyl 2-(2,6-difluoro-4-mercaptophenoxy)acetate (21)

[Chem. 32]

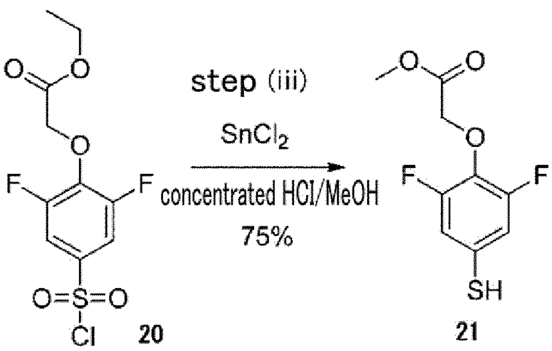


A mixture solution of the compound (20) (25.0 g, 0.08 mol), $SnCl_2$ (63.3 g, 0.28 mol), concentrated HCl (46.6 mL, 0.56 mol), and MeOH (333 mL) was heated to reflux temperature and stirred for about 1.5 hours. Thereafter, the reaction mixture solution was poured to ice and extracted with toluene. The organic layer was washed with 12% HCl three times, dried with sodium sulfate anhydride, and condensed. The residue was refined by silica gel column chromatography (PE : EA = 2 : 1) to thereby obtain a compound (21) (14 g, 75%).

$^1H$ NMR ($CDCl_3$, 300 MHz): $\delta$ 3.52 (s, 1H), 3.79 (s, 3H), 4.72 (s, 2H), 6.88 (d, J = 6.3 Hz, 2H).

Step (iv): Synthesis of 4-bromo-N-(2-bromoethyl)benzenesulfonamide (24)

[Chem. 33]

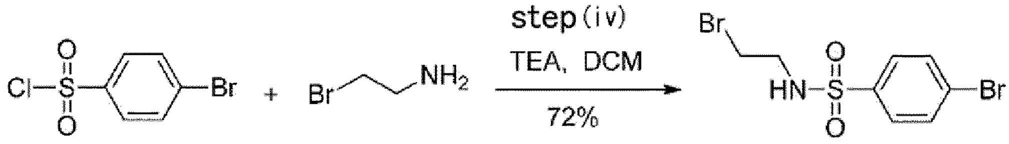


22 23 24

The compound (23) (1.35 g, 11.0 mmol) was added to a DCM (40 mL) solution of the compound (22) (2.54 g, 10.0 mmol), and subsequently, TEA (1.52 g, 15.0 mmol) was added thereto. Thereafter, the reaction mixture solution was stirred at room temperature for about 3 hours and diluted with water. The solution was extracted with DCM (80 mL × 3). The organic layer was washed with brine, dried with sodium sulfate anhydride, and condensed. The crude product was refined by silica gel column chromatography (PE : EA = 5 : 1) to thereby obtain a compound (24) (2.45 g, 72%).
$^1$H NMR (DMSO-$d_6$, 300 MHz) : δ 3.12-3.16 (m, 2H), 3.43 (t, J = 3.6 Hz, 2H), 7.69-7.73 (m, 2H), 7.79-7.82 (m, 2H), 8.13 (t, J = 3.9 Hz, 1H).

Step (v): Synthesis of methyl 2-(4-((2-(4-bromophenyl sulfonamide)ethyl)thio)-2,6-difluorophenoxy)acetate (25)

[Chem. 34]

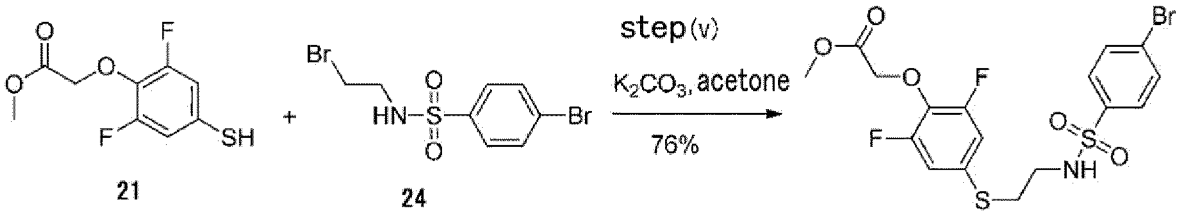


21 24 25

A mixture solution of the compound (21) (1.25 g, 5.36 mmol), $K_2CO_3$ (905 mg, 6.55 mmol), the compound (24) (1.88 g, 5.50 mmol), and acetone (50 mL) was stirred at room temperature for about 16 hours. The reaction mixture solution was poured to 3% HCl and extracted with ethyl acetate (90 mL × 3). The organic layer was dried with sodium sulfate anhydride and condensed. The crude residue was refined by silica gel column chromatography (PE : EA = 5 : 1) to thereby obtain a compound (25) (2 g, 76%) .
$^1$H NMR ($CDCl_3$, 300 MHz) : δ 2.94-2.98 (m, 2H), 3.08-3.14 (m, 2H), 3.77 (s, 3H), 4.73 (s, 2H), 5.33 (t, J = 6.0 Hz, 1H), 6.78-6.84 (m, 2H), 7.61-7.70 (m, 4H).

Step (vi): Synthesis of 2-(4-((2-(4-bromophenyl sulfonamide)ethyl)thio)-2,6-difluorophenoxy)acetamide (26)

[Chem. 35]

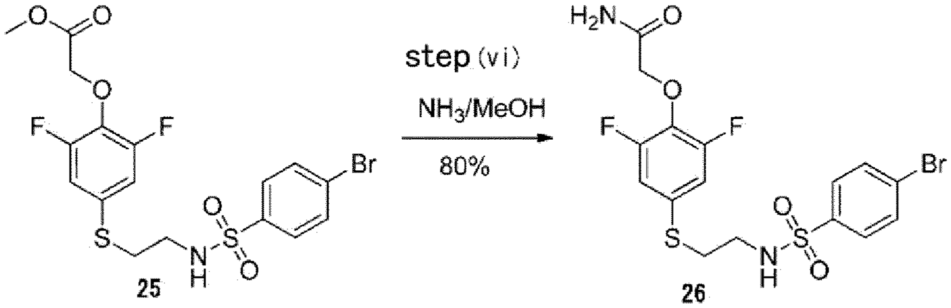


25

26

A mixture solution of the compound (25) (3.00 g, 6.06 mmol) and 2M $NH_3$/MeOH (150 mL, 300 mmol) was stirred at room temperature for about 16 hours. The obtained precipitate was recovered by filtration to thereby obtain a compound (26) (2.3 g, 80%).
$^1$H NMR (DMSO-$d_6$, 400 MHz): δ 2.93-2.96 (m, 2H), 3.00-3.03 (m, 2H), 4.48 (s, 2H), 7.10 (d, J = 9.2 Hz, 2H), 7.40-7.45 (m, 2H), 7.70 (d, J = 8.4 Hz, 2H), 7.80 (d, J = 8.4 Hz, 2H), 8.01 (brs, 1H) .

Step (vii): Synthesis of 2-(2,6-difluoro-4-((2-(4-(tributylstannyl)phenyl sulfonamide)ethyl)thio)phenoxy)acetamide (M-3pre)

[Chem. 36]

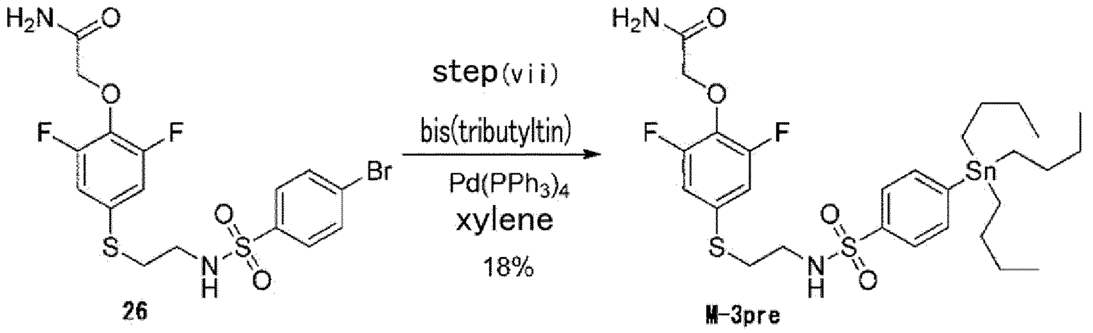


26

M-3pre

To a xylene (50 mL) solution of the compound (26) (670 mg, 1.39 mmol), bis(tributyltin) (0.87 mL, 1.81 mmol) and $Pd(PPh_3)_4$ (40 mg) were added. The reaction mixture solution was stirred under $N_2$ at 120°C for about 1 hour. Thereafter, the reaction mixture solution was condensed under vacuum, and the residue was refined by silica gel column chromatography (PE : EA = 3 : 1), thereby obtaining a compound (M-3pre) as yellow oil (180 mg, 18%).
$^1$H NMR ($CD_3OD$, 300 MHz) : δ 0.94 (t, J = 7.2 Hz, 9H), 1.12-1.17 (m, 5H), 1.29-1.39 (m, 8H), 1.52-1.60 (m, 5H), 2.98-3.06 (m, 4H), 4.55 (s, 2H), 7.01 (d, J = 9.0 Hz, 2H), 7.68 (d, J = 8.1 Hz, 2H), 7.77 (d, J = 8.1 Hz, 2H); LCMS [mobile phase: 5% water (0.02% $NH_4OAc$) and 95% $CH_3CN$ from 30% water (0.02% $NH_4OAc$) and 70% $CH_3CN$, 6 minutes, finally 0.5 minutes under these conditions] purity > 95%, Rt = 4.259 minutes; MS Calcd.: 692; MS Found: 693 ([M+H]$^+$).

(Synthesis Example 4)

(Synthesis of radio-labeled K-2)

A radio-labeled K-2 was synthesized as follows.

[Chem. 37]

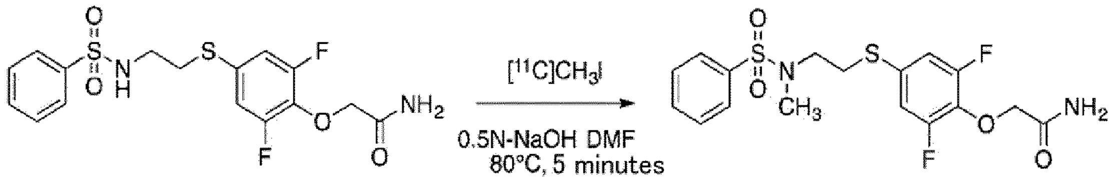


PEPA

radio-labeled K-2

1 mg of PEPA (ca 2.5 μmol) was dissolved in DMF (0.3 mL), 0.5 N-NaOH aq (7 μL) was added thereto and mixed, and then the resultant mixture was charged into a reaction container in a hot cell. After [$^{11}$C]methyl iodide was collected with the normal method, the reaction was performed at 80°C for 5 minutes. The resultant product was cooled to about room temperature, diluted with 500 μl of an LC solvent ($CH_3CN$ : $H_2O$ = 1 : 1), and then subjected to LC separation. Capcell Pak UG-80 (10X250) (Shiseido Co., Ltd., Japan) was used as a column, separation was performed at a flow rate of 5.0 ml/min, and detection was performed using UV 254 nm and RI. The RI peak portion near about 8 minutes was separated and condensed under addition of Tween 80 (final concentration: 0.8%) and 2.5 mg of ascorbic acid using an evaporator. The residue was dissolved by adding 2.5 ml of physiological saline water.

The radio-labeled K-2 and the unlabeled K-2 were compared by HPLC. The HPLC analysis was developed using Capcell Pak UG-80 (4.6X250) (Shiseido Co., Ltd., Japan) at a flow rate of 1.0 ml/min, and detection was performed using UV 254 nm and RI. The unlabeled K-2 (UV detection) and the radio-labeled K-2 (RI detection) exhibited the same peak at a retention time of 8 minutes. This indicates that both are the same substance and the radio-labeled K-2 can be produced.

It was found that the reacted methyl iodide binds to sulfonamide of 100% PEPA, and it was found that the synthesis of the radio-labeled K-2 is extremely simple and exhibits a high yield.

(Synthesis Example 5)

(Synthesis of radio-labeled M-3)

$Pd_2(dba)_3$ (1.74 mg), cuprous chloride (1.7 mg), and potassium carbonate (2.25 mg) were weighed in a 1-mL glass vial, and a DMF (300 μL) solution of $P(o\text{-}tol)_3$ (1.7 mg) was added to the mixture under a nitrogen atmosphere. The resultant mixture was stirred at room temperature for about 5 minutes, and then the solution was transferred to a labeling reaction container. [$^{11}$C]$CH_3I$ was collected under cooling, and after radioactivity was saturated, a DMF solution (300 μL) of a tributyltin compound (preM-3) (1.6 mg) of a raw material was added thereto and the reaction was performed at 80°C for about 5 minutes. The reaction mixture was allowed to pass through a PTFE filter to remove solid contents, HPLC separation was then performed, and the RI peak portion near about 7 minutes was separated, condensed, and compounded.

$Pd_2(dba)_3$: tris(dibenzylideneacetone)dipalladium
$P(o\text{-}tolyl)_3$: tri(o-tolyl)phosphine

(Reference Example 1)

(in vitro autoradiography method)

As rats, adult male SD rats (Charles River, Japan) which were 2 to 3 weeks old were used. An acute brain section which included a striatum and whose thickness was 200 μm was produced, was placed on a cell strainer and was left in an oxygenated ACSF (artificial cerebrospinal fluid) for 60 minutes.

12 ml of 100 μM PEPA (ACSF containing 1% DMSO) was prepared, and [$^{11}$C] K-2 was added thereto so as to achieve 20 nM, and the resultant solution was left for 60 minutes. After the reaction, the resultant solution was washed with the ACSF for 10 seconds three times and was finally washed with distilled water for 10 seconds. The cell strainer was cut out so as to be slightly larger than the brain section after the reaction and was exposed to an imaging plate for 2 hours. The imaging plate after the exposure was imaged with Typhoon (GE Healthcare Japan) (resolution of 25 pixels).

(Biological example)

(Preparation and administration of AMPA receptor-binding compound)

In a forced swimming test, AMPA receptor-binding compounds K-2 and K-4 were dissolved in 100% DMSO (Nacalai Tesque, Inc. Japan) so as to have a concentration of 2.1 mM, were diluted with saline immediately before being administered (15% DMSO, 320 μM) and were intravenously administered to the rat with an administered amount of 5 μl/weight (g) such that the administered amount fell within a range of 1 mg/kg to 1.5 mg/kg.

(Animal experiment)

In all animal experiments, the deliberation and the approval of the animal experiment committee in Yokohama City University were received (approval number: F-A-15-051). As rats, adult male Wistar rats which were 6 to 10 weeks old and Wistar Kyoto rats (WKY rats) which were depression model rats (Charles River, Japan) were used. In order to acquire a route for the repeated administration of K-2, a jugular vein cannula indwelling surgery was performed a week before the experiment. The rat was made to fall sleep with isoflurane (DS Pharma Animal Health, Japan), and thereafter, while anesthesia was maintained at an isoflurane concentration of 1.5% (air 2 L/minute), the jugular vein cannula indwelling surgery was performed. The skin of a neck was incised about 3 cm such that subcutaneous fat was opened, and thus a jugular vein was exposed. A needle to which a cannula tube was fitted was made to penetrate the jugular vein, then the cannula tube was removed from the needle, and the tip of the cannula tube was inserted 2.5 cm into the jugular vein so as to be fixed near an insertion portion with sutures. The opposite side of the cannula tube was exposed to the outside of the body through the back of the rat, a plug was fitted so as to prevent backflow, and the opened skin was sutured. After the completion of the surgery, the rat was returned to a home cage.

(In vivo PET imaging using rat)

PET imaging was performed with microPET (Focus 220; Siemens Medical Solution). PET imaging experiment using the rat: After the rat was made to fall asleep in an anesthesia box filled with isoflurane (DS Pharma Animal Health, Japan), anesthesia was maintained at an isoflurane concentration of 1.5% (air 2 L/minute), and then an intravenous line was acquired from a tail vein with a 24G Surflo indwelling needle (Terumo Corporation, Japan). The rat was fixed to a PET shooting base, then radiation imaging for attenuation correction was performed before imaging and thereafter a radio-labeled K-2 (about 40 MBq) was administered. During the PET imaging, the body temperature was maintained at 37 ± 0.5°C with a feedback type heating plate (BWT-100A; Bio Research Center, Japan). After the imaging, the intravenous line was removed, the administration of isoflurane was stopped, and then the rat was removed from the PET base and was returned to the home cage. The rat was raised in a room in which the imaging was performed during one week after the imaging, and then the rat was returned to a normal rat group raising room. A summation image was constructed, show-through was removed with a 0.5 mm Hanning filter and a dynamic image was reconstructed. The reconstructed image was analyzed with PMOD image analysis software (PMOD technologies) by integrating VOIs including a plurality of regions of hippocampus, medial prefrontal cortex, nucleus accumbens, striatum, thalamus, cerebellum and the like with one formed on a template MRI image. A calculation value used in quantitative determination was %SUV (% of standardized uptake value) and was obtained by the following Formula;

$$\%SUV = \text{amount of radiation of each tissue surrounded by VOI (kBq/cc)/administered amount of radiation (MBq)} \times \text{weight (kg)} \times 100$$

(Forced swimming test using rat)

The forced swimming test was performed in a pre-administration experiment on the day before the administration of the drug, in an acute administration experiment on the first day of the administration of the drug and in a chronic administration experiment on the seventh day of the administration of the drug. In the forced swimming test (the pre-administration experiment, the acute administration experiment, and the chronic administration experiment), the rat was left for one hour so as to be habituated to a room where the experiment was performed. In the pre-administration experiment, immediately after the habituation, the forced swimming test was performed. In the acute administration experiment, 15% DMSO or K-2 and K-4 dissolved in 15% DMSO were administered intravenously 30 minutes before

the start of the forced swimming test. In the chronic administration experiment, 15% DMSO or K-2 and K-4 dissolved in 15% DMSO were administered intravenously for 7 days, and the forced swimming test was performed 30 minutes after the administration on the seventh day. The forced swimming test was performed with a cylindrical cage whose diameter was 20 cm and whose swimming height was 50 cm by injecting tap water having a temperature of 26±0.5°C up to about a water level of 40 cm. The rat was input into the cylindrical cage, and how the rat swam was shot for 10 minutes with a digital video camera (HC-V750, Panasonic, Japan). After the shooting, water droplets were wiped off the rat with a paper towel, and the rat was returned to the home cage. In the analysis, in the acute administration experiment and the chronic administration experiment, a time during which the rat did not move the four limbs in 2 to 10 minutes of a video shot (the time of 0 to 2 minutes was regarded as an environmental adaptation time) was measured as an immobile time. The immobile time was compared between the rat to which 15% DMSO was administered and the rats to which K-2 and K-4 dissolved in 15% DMSO were administered.

(Experiment result)

(Result of AMPA receptor binding test)

It was confirmed by the in vitro autoradiography method that K-2 indicated binding affinity to the AMPA receptors, and the Kd value thereof was 47.9 nM (Fig. 4). It was confirmed by the electrophysiological verification that K-2 and K-4 indicated binding affinity to the AMPA receptors (Fig. 5).

(PET imaging on rat using AMPA receptor-labeled compound K-2)

A radio-labeled K-2 ([$^{11}$C] K-2) was administered to the Wistar rat and the WKY rat, and PET imaging was performed in vivo (Figs. 6 and 7). Then, on these images, analysis was performed with the PMOD image analysis software. Consequently, in the medial prefrontal cortex, the striatum, the cerebral cortex and the thalamus of the WKY rat, a significantly lower uptake of the radio-labeled K-2 in the brain was indicated (Fig. 8), and it was suggested that in these parts, the accumulated amount of AMPA receptor was lowered. It was found from the result thereof that in the WKY rat which was the depression model rat, the expression level of the AMPA receptors was lowered in specific brain regions.

(Forced swimming test on rat using AMPA receptor-binding compound K-2)

In the acute administration experiment, it was not found that there was no difference in the immobile time between the rat to which 15% DMSO was administered and the rat to which K-2 was administered (Fig. 9). In the chronic administration experiment, it was found that in the rat to which K-2 was administered, the immobile time was significantly lowered, and the effect thereof was significantly greater in 1.5 mg/kg than in 1 mg/kg (Fig. 10). Likewise, it was found that in the rat to which K-4 was administered, the immobile time was significantly lowered, and by comparison in the administration of 1 mg/kg, K-4 significantly lowers the immobile time instead of K-2 (Fig. 10). As a result thereof, it was suggested that the chronic administration of K-2 and K-4 in the WKY rat which was the depression model rat indicated an antidepressant effect. Since the relationship was confirmed between the compounds such as K-2 an K-4 indicating binding affinity to the AMPA receptors and depression which was known to be related to the AMPA receptors (by Jingli Zhang et al., Rev. Neurosci. 2013; 24 (5): 499-505, and by Simon E Ward et al., British Journal of Pharmacology (2010), 160, 181-190), it is suggested that the antidepressant effect described above is caused by the activation of the AMPA receptor function. Hence, it is suggested that K-2 and K-4 and compounds similar thereto in the present invention are useful for the therapy of not only depression but also diseases associated with AMPA receptors. Thus, it is suggested that based on the result of the imaging of AMPA receptors in the brain of a mammal organism, the diagnosis of a disease associated with AMPA receptors in the brain, the companion diagnosis for the therapy or the prophylaxis of the disease, the establishment of the administration plan of a drug for the therapy or the prophylaxis of the disease and the screening of the therapeutic or prophylactic agent for the disease are possible.

(Example 1)

(PET imaging in humans)

PET imaging was performed on two human subjects. The human subject had a lying posture on an imaging stand, and an intravenous line was acquired in a forearm or the back of a hand with a 22 gauge Surflo needle. Absorption correction CT imaging was performed for about one minute, and then [$^{11}$C] K-2 of about 370 MBq was administered, as a PET drug, intravenously over one minute. Thereafter, imaging was performed for 120 minutes. For the imaging, a

PET/CT device "Aquiduo" (Toshiba Medical Systems Corporation) was used. In list data which was collected, dynamic images were reconstructed by an OS-EM method.

The reconstructed image was analyzed with the PMOD image analysis software (PMOD technologies) by integrating VOIs including a plurality of regions of frontal lobe, dentate cortex, hippocampus, amygdala, putamen, cerebellum, bridge and the like with one formed on a template MRI image. Fig. 11 is an image (referred to as a first-half image) for 30 minutes until the elapse of 30 minutes since the administration of the PET drug and an image (referred to as a second-half image) for 60 minutes until the elapse of 120 minutes (an example of the second time) since the elapse of 60 minutes (an example of the first time) after the administration of the PET drug. In both the human subjects, although in the first-half image, a slight difference in the accumulation of [$^{11}$C] K-2 was found between the left and right of hippocampus, it was difficult to clearly recognize it whereas in the second-half image (right side), a difference in the accumulation was found between the left and right of hippocampus such that high accumulation was recognized in the right hippocampus of the human subject A and that high accumulation was recognized in the left hippocampus of the human subject B. This result indicates that between 30 minutes after the administration of the PET drug and 60 minutes after the administration of the PET drug, nonspecific accumulation caused by the PET drug which was not bound to the AMPA receptors in the brain was remarkably washed out. Hence, it is found that when [$^{11}$C] K-2 was used as the PET drug, though there is no particular limitation, it is preferable to perform detection and discovery at least 30 minutes after the administration, in particular, about 60 minutes after the administration.

The obtained image is an image in which an AMPA receptor-binding brain region can be clearly distinguished from an AMPA receptor-non-binding region and which has a high resolution and a high contrast, and thus the image is said to be appropriate for the diagnosis of a disease associated with the binding of AMPA receptors. In the first-half image until the elapse of 30 minutes since the administration of the PET drug, both the PET drug which was bound to the AMPA receptors in the brain and the PET drug which was not bound to the AMPA receptors in the brain were detected in a mixed manner, and thus although the entire brightness was high, the resolution was low (coarse), with the result that the image is said to be an image in which there is a room for improvement in order to clearly determine the AMPA receptor-binding brain region. It is theoretically possible to continuously acquire images until the elapse of 120 minutes since the administration of the PET drug. However, the continuous images are addition images which include the low-resolution first-half image and the high-resolution second-half image, in which furthermore, since the first-half image having a large amount of radiation more contributes to the brightness of the images, as a whole, low-resolution images are provided and in which thus skills are somewhat needed for diagnosis. Since in an example of the method of setting the first time for obtaining a high-resolution and multi-tone image, the first time is a time at which the PET drug that is not bound to the AMPA receptors in the brain is remarkably washed out or thereafter, the first time can be after a time at which at least the total detected amount of radiation in the entire brain is the maximum value. A difference between the first-half image and the second-half image was recognized not only on humans but also on monkeys (data thereof is not shown).

(Example 2)

(PET imaging in humans)

PET imaging was performed on healthy men (Y5, Y14, and Y16) in their twenties. The man had a lying posture on an imaging stand, and an intravenous line was acquired in a forearm or the back of a hand with a 22 gauge Surflo needle. Absorption correction CT imaging was performed for about one minute, and then [$^{11}$C] K-2 of about 370 MBq was administered intravenously over one minute. Thereafter, imaging was performed for 120 minutes. For the imaging, the PET/CT device "Aquiduo" (Toshiba Medical Systems Corporation) was used. In list data which was collected, dynamic images were reconstructed by the OS-EM method.

The reconstructed image was analyzed with the PMOD image analysis software (PMOD technologies) by integrating VOIs including a plurality of regions of frontal lobe, dentate cortex, hippocampus, amygdala, putamen, cerebellum, bridge and the like with one formed on a template MRI image. As a calculation value used in quantitative determination, %SUV (% of standardized uptake value) was used. With consideration given to the result of Example 1, as the %SUV of the three healthy persons, the average value for 70 minutes from the elapse of 50 minutes since the administration of the PET drug (an example of the first time) to the elapse of 120 minutes since the administration (an example of the second time) was calculated so as to be used for the analysis.

As shown in Figs. 12 and 13, it is found that an individual difference is small on the difference of detection values between brain regions. It is found from Fig. 12 in particular that the uptake into the brain is high, that the difference between brain regions is clear and that almost no signal is present in white matter where the AMPA receptors are not present. In other words, it has been found that the imaging of the AMPA receptors in the brain of a human organism can be performed appropriately.

Moreover, with consideration given to the result of Figs. 6 and 7 indicating a correlation between a disease

associated with AMPA receptors in the brain of a mammal and the expression level and the localization of the AMPA receptors in the mammal organism, it was suggested that a substance which is selectively bound to the AMPA receptors in the brain of the primate organism and is radio-labeled can be used as an active ingredient of a diagnostic agent for the disease associated with AMPA receptors in the brain of the primate organism or a companion diagnostic agent for the therapy or the prophylaxis of the disease. Likewise, it was suggested that in the administration plan of a drug for the therapy or the prophylaxis of the disease associated with AMPA receptors in the brain of the primate organism and the method of screening a therapeutic or prophylactic agent, the result of the imaging method of the present invention can be used.

(Example 3)

In Example 2, as the %SUV of six healthy persons, the average value for 10 minutes (referred to as a first half) from the elapse of 50 minutes since the administration of the PET drug (an example of the first time) to the elapse of 60 minutes since the administration (an example of the second time) and the average value for 30 minutes (referred to as a second half) from the elapse of 60 minutes since the administration of the PET drug (an example of the first time) to the elapse of 90 minutes since the administration (an example of the second time) were calculated so as to be used for the analysis. The result thereof is shown in Fig. 14. The vertical axis of Fig. 14 represents a relative value of the %SUV of each of the brain regions when the %SUV of occipital lobe is assumed to be 1. The occipital lobe has a relatively large amount of blood flow ingredient so as to be suitable as the reference part.

Since in the second half, the amount of radiation is significantly attenuated, large variations in the values of the healthy persons were produced, and thus the standard deviation was expanded. When the variations in the values are expanded, it is more likely that it is difficult to detect a significant difference in statistical analysis. Hence, it is found that when [$^{11}$C] K-2 of about 370 MBq was used as the PET drug, though there is no particular limitation, it is preferable to perform detection and discovery within 60 minutes after the administration.

(Example 4)

(AMPA-PET of epilepsy patient where epilepsy focus is confined to medial temporal lobe-PET imaging in medial temporal lobe epilepsy patient)

PET imaging was performed on medial temporal lobe epilepsy patients of three examples. The patient had a lying posture on an imaging stand, and an intravenous line was acquired in a forearm or the back of a hand with a 22 gauge Surflo needle. Absorption correction CT imaging was performed for about one minute, and then [$^{11}$C] K-2 of about 370 MBq was administered intravenously over one minute. Thereafter, imaging was performed for 90 minutes. For the imaging, the PET/CT device "Aquiduo" (Toshiba Medical Systems Corporation) was used. In list data which was collected, dynamic images were reconstructed by the OS-EM method.

The reconstructed image was analyzed with the PMOD image analysis software (PMOD technologies) by integrating a PET image on a standardized MRI image. As the reconstructed image, a SUVR (standardized uptake value ratio) image was used. The SUVR image was produced by dividing an addition average image for 20 minutes until the elapse of 30 to 50 minutes since the administration of the PET drug by the average value of the amount of radiation for the same time which was accumulated in corpus callosum that was a reference region.

As shown in Fig. 15, high accumulation of [$^{11}$C] K-2 was recognized in medial temporal lobe on a focus side (white arrows). It is found from the result thereof that the SUVR image from the elapse of 30 minutes (an example of the first time) to the elapse of 50 minutes (an example of the second time) since the administration of the PET drug was used and that thus the AMPA receptors were accumulated locally in the focus part of the medial temporal lobe.

(Example 5)

(Subtraction image analysis on epilepsy patient)

PET imaging was performed on medial temporal lobe epilepsy patients of three examples. The patient had a lying posture on an imaging stand, and an intravenous line was acquired in a forearm or the back of a hand with a 22 gauge Surflo needle. Absorption correction CT imaging was performed for about one minute, and then [$^{11}$C] K-2 of about 370 MBq was administered intravenously over one minute. Thereafter, imaging was performed for 90 minutes. For the imaging, the PET/CT device "Aquiduo" (Toshiba Medical Systems Corporation) was used. In list data which was collected, dynamic images were reconstructed by the OS-EM method.

The reconstructed image was analyzed with the PMOD image analysis software (PMOD technologies) by integrating a PET image on a standardized MRI image. As the reconstructed image, a subtraction image was used so

as to produce a reconstructed image as follows. An average image (image including a large number of specific bonds to the AMPA receptors) for 20 minutes from the elapse of 30 minutes (an example of the first time) to the elapse of 50 minutes (an example of the second time) since the administration of the PET drug and an average image (image including a large amount of blood flow ingredient) for 1 minute from the elapse of 1.5 minutes to the elapse of 2.5 minutes were calculated. In each time period, by division by the average value of the amount of radiation accumulated in the entire brain, the SUVR image in which the entire brain was the reference region was calculated. From the SUVR image for 20 minutes from the elapse of 30 minutes to the elapse of 50 minutes since the administration of the PET drug, the SUVR image for 1 minute from the elapse of 1.5 minutes to the elapse of 2.5 minutes was subtracted, and thus a difference was calculated, with the result that the subtraction image was produced.

As shown in Fig. 16, high accumulation of [$^{11}$C] K-2 was recognized in medial temporal lobe on the focus side (white arrows). It is found from the result thereof that the subtraction image was used and that thus the AMPA receptors were accumulated locally in the focus part of the medial temporal lobe.

(Example 6)

(Asymmetry Index comparison on healthy person and medial temporal lobe epilepsy patient)

PET imaging was performed on healthy persons of six examples and medial temporal lobe epilepsy patients of three examples. The healthy person or the patient had a lying posture on an imaging stand, and an intravenous line was acquired in a forearm or the back of a hand with a 22 gauge Surflo needle. Absorption correction CT imaging was performed for about one minute, and then [$^{11}$C] K-2 of about 370 MBq was administered intravenously over one minute. Thereafter, imaging was performed for 90 minutes. For the imaging, the PET/CT device "Aquiduo" (Toshiba Medical Systems Corporation) was used. In list data which was collected, dynamic images were reconstructed by the OS-EM method.

The reconstructed image was analyzed with the PMOD image analysis software (PMOD technologies) by integrating VOIs including a plurality of regions of frontal lobe, temporal lobe, occipital lobe, parietal lobe, hippocampus, amygdala, putamen, corpus callosum, cerebellum, bridge and the like with one formed on a standardized MRI image. As the reconstructed image, a subtraction image was used so as to produce a reconstructed image as follows. An average image for 20 minutes from the elapse of 30 minutes (an example of the first time) to the elapse of 50 minutes (an example of the second time) since the administration of the PET drug and an average image (image including a large amount of blood flow ingredient) for 1 minute from the elapse of 1.5 minutes to the elapse of 2.5 minutes were calculated. In each time period, by division by the average value of the amount of radiation accumulated in the entire brain, the SUVR image in which the entire brain was the reference region was calculated. From the SUVR image for 20 minutes from the elapse of 30 minutes to the elapse of 50 minutes since the administration of the PET drug, the SUVR image for 1 minute from the elapse of 1.5 minutes to the elapse of 2.5 minutes was subtracted, and thus a difference was calculated, with the result that the subtraction image was produced. In the subtraction images of the healthy person and the medial temporal lobe epilepsy patient, the VOIs of left and right temporal lobes were used so as to calculate image values, and thus an Asymmetry index (AI) was determined. The AI was calculated by a calculation Formula below.

healthy person; AI = 200 × (left temporal lobe VOI value - right temporal lobe VOI value) / (left temporal lobe VOI value + right temporal lobe VOI value)

medial temporal lobe epilepsy patient; AI = 200 × (subtraction image value surrounded by VOI of focus side temporal lobe - subtraction image value surrounded by VOI of non-focus side temporal lobe) / (subtraction image value surrounded by VOI of focus side temporal lobe + subtraction image value surrounded by VOI of non-focus side temporal lobe)

As shown in Fig. 17, when the AIs in the healthy person and the medial temporal lobe epilepsy patient are compared, in the medial temporal lobe epilepsy patient, a significantly high AI is indicated. With the AIs, it is found that in the medial temporal lobe epilepsy patient, the AMPA receptors are highly accumulated in the medial temporal lobe on the focus side.

(Example 7)

(Comparison between K-2 imaging and FDG imaging on medial temporal lobe epilepsy patient)

PET imaging was performed on medial temporal lobe epilepsy patients of three examples with 18F-FDG (fluorodeoxyglucose). In a front room, an intravenous line was acquired in a forearm or the back of a hand with a 22 gauge Surflo needle.

18F-FDG of about 185 MBq was administered intravenously over one minute. Thereafter, the patient was left for 60 minutes, and then imaging was performed for 20 minutes (after absorption correction CT imaging was performed for about one minute). In list data which was collected, dynamic images were reconstructed by the OS-EM method.

The reconstructed image was analyzed with the PMOD image analysis software (PMOD technologies) by integrating VOIs including a plurality of regions of frontal lobe, temporal lobe, occipital lobe, parietal lobe, hippocampus, amygdala, putamen, corpus callosum, cerebellum, bridge and the like with one formed on a standardized MRI image. As the reconstructed image, a SUVR (standardized uptake value ratio) image was used. The SUVR image was produced by dividing an addition average image for all the time after the administration of the PET drug by the average value of the amount of radiation which was accumulated in corpus callosum that was the reference region. An Asymmetry index (AI) was determined from the subtraction images of [$^{11}$C] K-2 in the medial temporal lobe epilepsy patients (images acquired in Example 6 on the medial temporal lobe epilepsy patients of three examples) and the SUVR image of FDG. The AI was calculated by a calculation Formula below.

FDG image; AI = 200 × (amount of radiation surrounded by VOI of focus side temporal lobe (MBq/cc) - amount of radiation surrounded by VOI of non-focus side temporal lobe (MBq/cc)) / (amount of radiation surrounded by VOI of focus side temporal lobe (MBq/cc) + amount of radiation surrounded by VOI of non-focus side temporal lobe (MBq/cc)).

[$^{11}$C] K-2 subtraction image; AI = 200 × (subtraction image value surrounded by VOI of focus side temporal lobe - subtraction image value surrounded by VOI of non-focus side temporal lobe) / (subtraction image value surrounded by VOI of focus side temporal lobe + subtraction image value surrounded by VOI of non-focus side temporal lobe)

As shown in Fig. 18, when the AIs calculated from the subtraction images of [$^{11}$C] K-2 in the medial temporal lobe epilepsy patients and the SUVR image of FDG are compared, in the AI calculated from the subtraction images of [$^{11}$C] K-2, a significantly high value is indicated. In other words, it is found that the subtraction images of [$^{11}$C] K-2 were used to measure the AI and that thus high-sensitive detection can be performed as the focus part as compared with the FDG.

(Example 8)

(AMPA-PET comparison between depressed state of depressed patient and remission period)

PET imaging was performed on a depressed patient and the same patient in a remission period. The patient had a lying posture on an imaging stand, and an intravenous line was acquired in a forearm or the back of a hand with a 22 gauge Surflo needle. Absorption correction CT imaging was performed for about one minute, and then [$^{11}$C] K-2 of about 370 MBq was administered intravenously over one minute. Thereafter, imaging was performed for 60 minutes. For the imaging, the PET/CT device "Aquiduo" (Toshiba Medical Systems Corporation) was used. In list data which was collected, dynamic images were reconstructed by the OS-EM method.

The reconstructed image was analyzed with the PMOD image analysis software (PMOD technologies) by integrating VOIs including a plurality of regions of frontal lobe, temporal lobe, occipital lobe, parietal lobe, hippocampus, amygdala, putamen, corpus callosum, cerebellum, bridge and the like with one formed on a standardized MRI image. As the reconstructed image, a SUVR (standardized uptake value ratio) image was used. The SUVR image was produced by dividing an addition average image for 20 minutes until the elapse of 30 to 50 minutes since the administration of the PET drug by the average value of the amount of radiation for the same time which was accumulated in corpus callosum that was the reference region.

As shown in Fig. 19, in a plurality of brain regions of the depressed patient, low accumulation of [$^{11}$C] K-2 was recognized. In the result thereof, with the SUVR images after the elapse of 30 to 50 minutes since the administration of the PET drug, it was possible to clarify that the density of the AMPA receptors in the depressed patient was lowered. On the other hand, in the depressed patient in the remission period, as compared with the depressed patient, in almost all brain regions, high accumulation of [$^{11}$C] K-2 was recognized, and thus it was found that the density of the AMPA receptors was recovered to the same level as a healthy person. Consequently, with the SUVR images after the elapse of 30 minutes (an example of the first time) to 50 minutes (an example of the second time) since the administration of

the PET drug, changes in the clinical symptoms of the depressed patient were able to be detected from a difference in the density of the AMPA receptors.

Examples 1 to 8 described above indicate that on primates, in particular, on humans, the imaging of the AMPA receptors in the brain of the organism is performed, that the images thereof are used and that thus it is possible to diagnose diseases associated with AMPA receptors in the brain and to perform companion diagnosis for the therapy or the prophylaxis of the diseases. In particular, Example 8 indicates that the therapeutic effect of the disease and a recovery status until healing can be found from the imaging images, and thus it can be said that it is possible to establish the administration plan of a drug for the therapy or the prophylaxis. It can also be said that it is possible to screen the therapeutic or prophylactic agent for the disease described above. It is also indicated that as a PET drug, as compared with FDG, the compound of the present invention, in particular, a compound in which an example thereof is K-2 and in which $R^2$ is alkyl is preferable.

(Description of Abbreviations)

AMPA: α-amino-3-hydroxy-5-methyl-4-isoxazole-propionic acid
DIPEA: diisopropylethylamine
DCM: dichloromethane
EA, EtOAc: ethyl acetate
FDG: fluorodeoxyglucose
PE: petroleum ether
PEPA: 2-[2,6-difluoro-4-({2-[(phenylsulfonyl)amino]ethyl}thio)phenoxy]acetamide
PET: positron emission tomography
TEA: tetraethylammonium
TMS: tetramethylsilane
MeOH: methanol
EtOH: ethanol
DMF: N,N-dimethylformamide
MeI: methyl iodide
WSC-HCl: water-soluble carbodiimide hydrochloride
DMSO: dimethyl sulfoxide
ACSF: artificial cerebrospinal fluid

EXPLANATION OF REFERENCE NUMERALS

100: molecular imaging device
200: input terminal
210: imaging data generation unit
220: metadata generation unit
300: server
310: information production unit
350: checking unit
360: instruction unit
400: database (first database)
500: output terminal
600: second database
900: clock
1000: system

## Claims

1. A method of imaging AMPA receptors in a brain of a primate organism, the method comprising: a step of delivering into the brain a substance which is administered to the primate organism, the substance being selectively bound to the AMPA receptors in the brain of the primate organism and radiolabeled, binding the substance to the AMPA

receptors in the brain and detecting radiation which is emitted from the substance bound to the AMPA receptors in the brain so as to acquire data on a distribution and/or an expression level of the AMPA receptors in the brain.

**2.** The method according to claim 1, wherein a required time is allowed after the delivery of the substance into the brain such that the substance which is not bound to the AMPA receptors in the brain is urged to be discharged to an outside of the brain, and the detection is thereafter performed.

**3.** The method according to claim 1 or 2, wherein the detection is performed both when a first period of time elapses after the delivery of the substance into the brain and when a second period of time longer than the first period of time elapses, and
based on individual detection values, the data on the distribution and/or the level of the AMPA receptors in the brain is acquired.

**4.** The method according to any one of claims 1 to 3, wherein the substance comprises a compound represented by Formula (I) or a pharmaceutically acceptable salt or solvate thereof:

[Chem. 1]

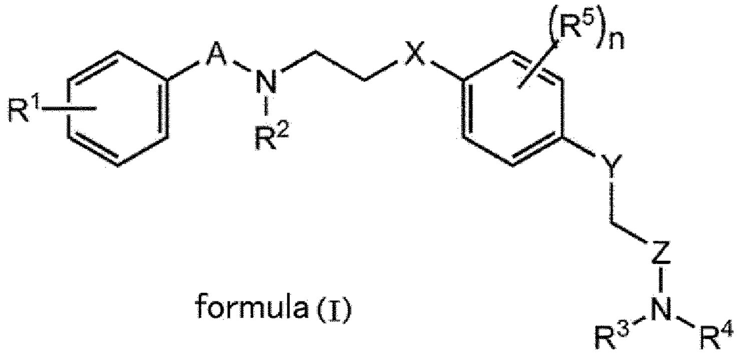

formula (I)

(in the formula,

each of A and Z independently represents CO, SO, or $SO_2$;
each of X and Y independently represents S or O;
each of $R^1$ to $R^4$ independently represents hydrogen, alkyl, alkenyl, alkynyl, or halo;
each $R^5$ independently represents alkyl, alkenyl, alkynyl, or halo;
n represents an integer of 0 to 4; and
one or more atoms are radioisotopes of the atoms.)

**5.** A program for instructing a computer to perform the method according to any one of claims 1 to 4.

**6.** A diagnostic agent for a disease associated with AMPA receptors in a brain of a primate organism or a companion diagnostic agent for therapy or prophylaxis of the disease, wherein a substance which is selectively bound to the AMPA receptors in the brain of the primate organism and is radiolabeled is an active ingredient.

**7.** A drug for therapy or prophylaxis of a disease associated with AMPA receptors in a brain of a primate organism, wherein a substance which is selectively bound to the AMPA receptors in the brain of the primate organism is an active ingredient, and the drug is administered according to an administration plan based on the data obtained by the method according to any one of claims 1 to 4.

**8.** The agent or the drug according to claim 6 or 7, wherein the disease is a mental disease or a neurological disease.

**9.** A method of screening a therapeutic or prophylactic agent for a disease associated with AMPA receptors in a brain of a primate organism, the method comprising:
a step of screening, before and after a candidate substance is administered to the primate organism, the candidate substance based on a difference in the data obtained by the method according to any one of claims 1 to 4.

**10.** An input terminal which transmits to a server information on a distribution and/or an expression level of AMPA receptors in a brain of a primate organism.

**11.** A server comprising: a database that stores data in which a distribution and/or an expression level of AMPA receptors in a brain of a primate organism is associated with a state of a disease associated with AMPA receptors in the brain of the primate organism; and
a means which compares information having been input on a distribution and/or an expression level of AMPA receptors in a brain of an organism of a human subject with the data so as to transmit information on a state of the disease of the human subject to an output terminal.

**12.** A server comprising: a database that stores data in which a distribution and/or an expression level of AMPA receptors in a brain of a primate organism, a state of a disease associated with AMPA receptors in the brain of the primate organism and a type, a dosage, and/or usage of a drug that has already been administered and is intended for therapy or prophylaxis of the disease associated with AMPA receptors in the brain of the primate organism are associated with each other; and
a means which compares information having been input on a distribution and/or an expression level of AMPA receptors in a brain of an organism of a human subject with the data and transmits, to an output terminal, information on a type, a dosage, and/or a usage of a drug that is recommended to the human subject.

**13.** A system comprising: the input terminal according to claim 10;
the server according to claim 11 or 12; and
the output terminal which outputs the information transmitted from the server according to claim 11 or 12.

FIG. 1

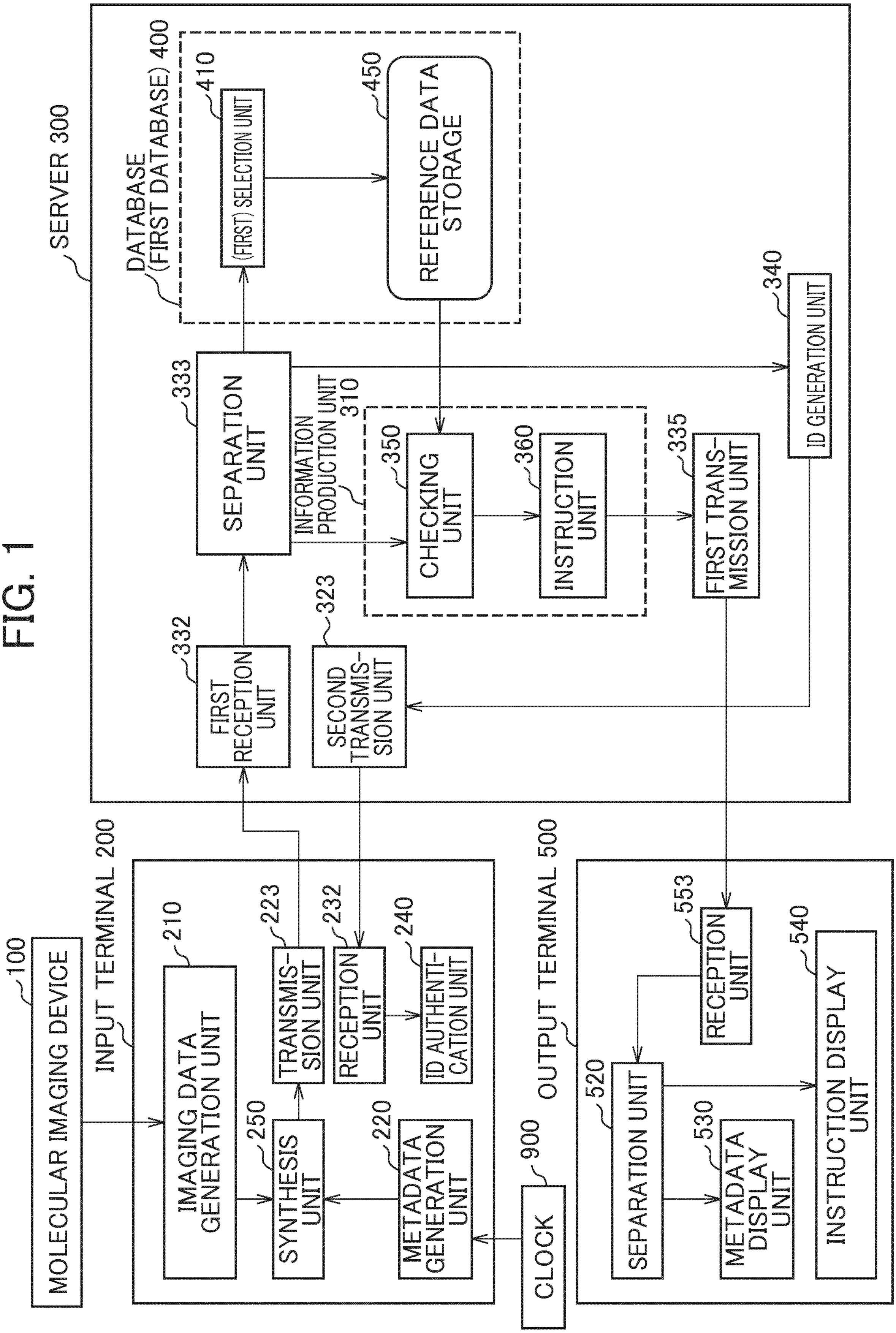
SERVER 300
DATABASE (FIRST DATABASE) 400
410
(FIRST) SELECTION UNIT
450
REFERENCE DATA STORAGE
333
SEPARATION UNIT
INFORMATION PRODUCTION UNIT 310
350
CHECKING UNIT
360
INSTRUCTION UNIT
335
FIRST TRANS-MISSION UNIT
340
ID GENERATION UNIT
332
FIRST RECEPTION UNIT
323
SECOND TRANSMIS-SION UNIT
100
MOLECULAR IMAGING DEVICE
INPUT TERMINAL 200
210
IMAGING DATA GENERATION UNIT
223
TRANSMIS-SION UNIT
232
RECEPTION UNIT
240
ID AUTHENTI-CATION UNIT
250
SYNTHESIS UNIT
220
METADATA GENERATION UNIT
900
CLOCK
OUTPUT TERMINAL 500
553
RECEPTION UNIT
540
INSTRUCTION DISPLAY UNIT
520
SEPARATION UNIT
530
METADATA DISPLAY UNIT

FIG. 2

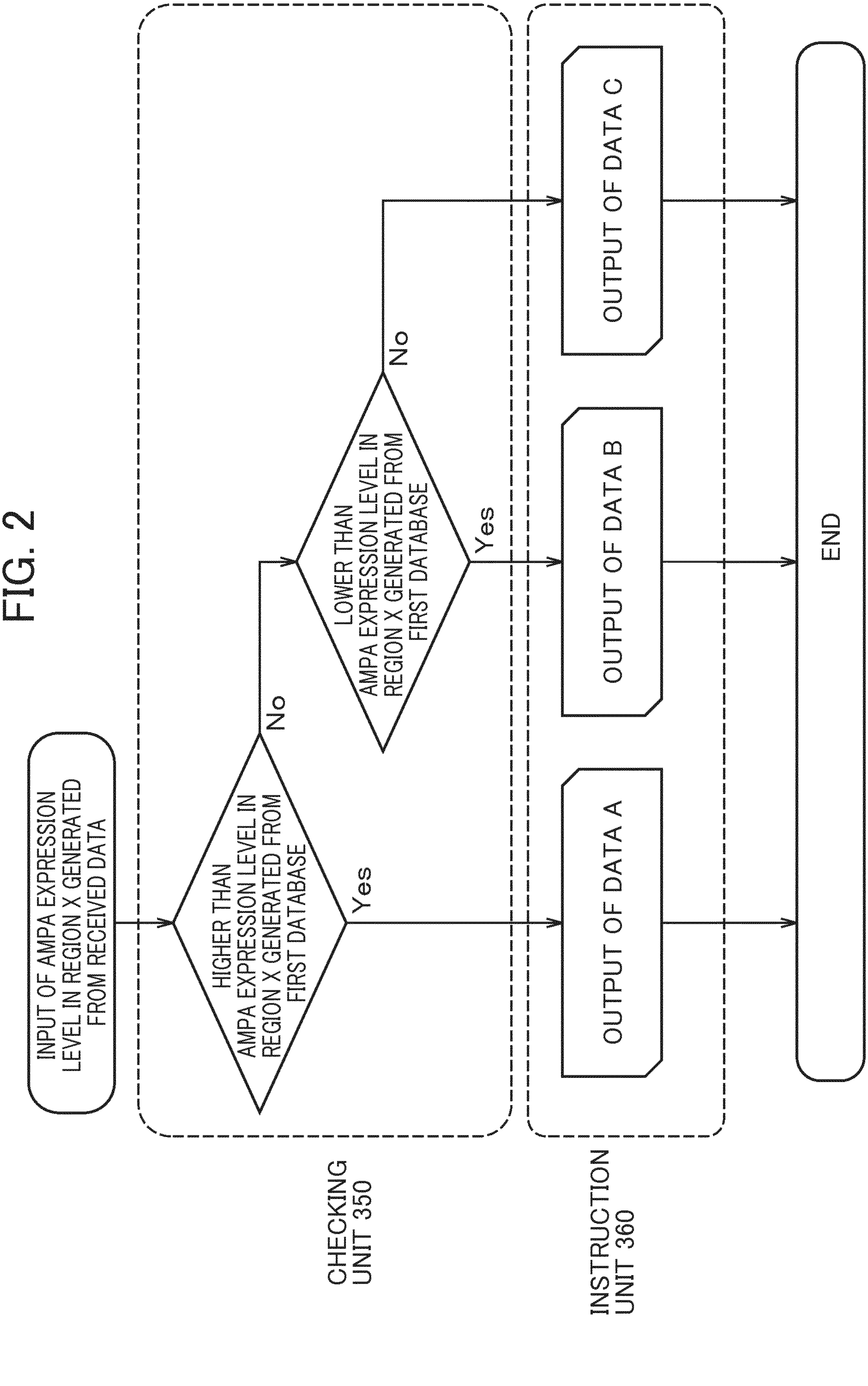
INPUT OF AMPA EXPRESSION LEVEL IN REGION X GENERATED FROM RECEIVED DATA
HIGHER THAN AMPA EXPRESSION LEVEL IN REGION X GENERATED FROM FIRST DATABASE
Yes
No
LOWER THAN AMPA EXPRESSION LEVEL IN REGION X GENERATED FROM FIRST DATABASE
Yes
No
OUTPUT OF DATA A
OUTPUT OF DATA B
OUTPUT OF DATA C
END
CHECKING UNIT 350
INSTRUCTION UNIT 360

FIG. 3

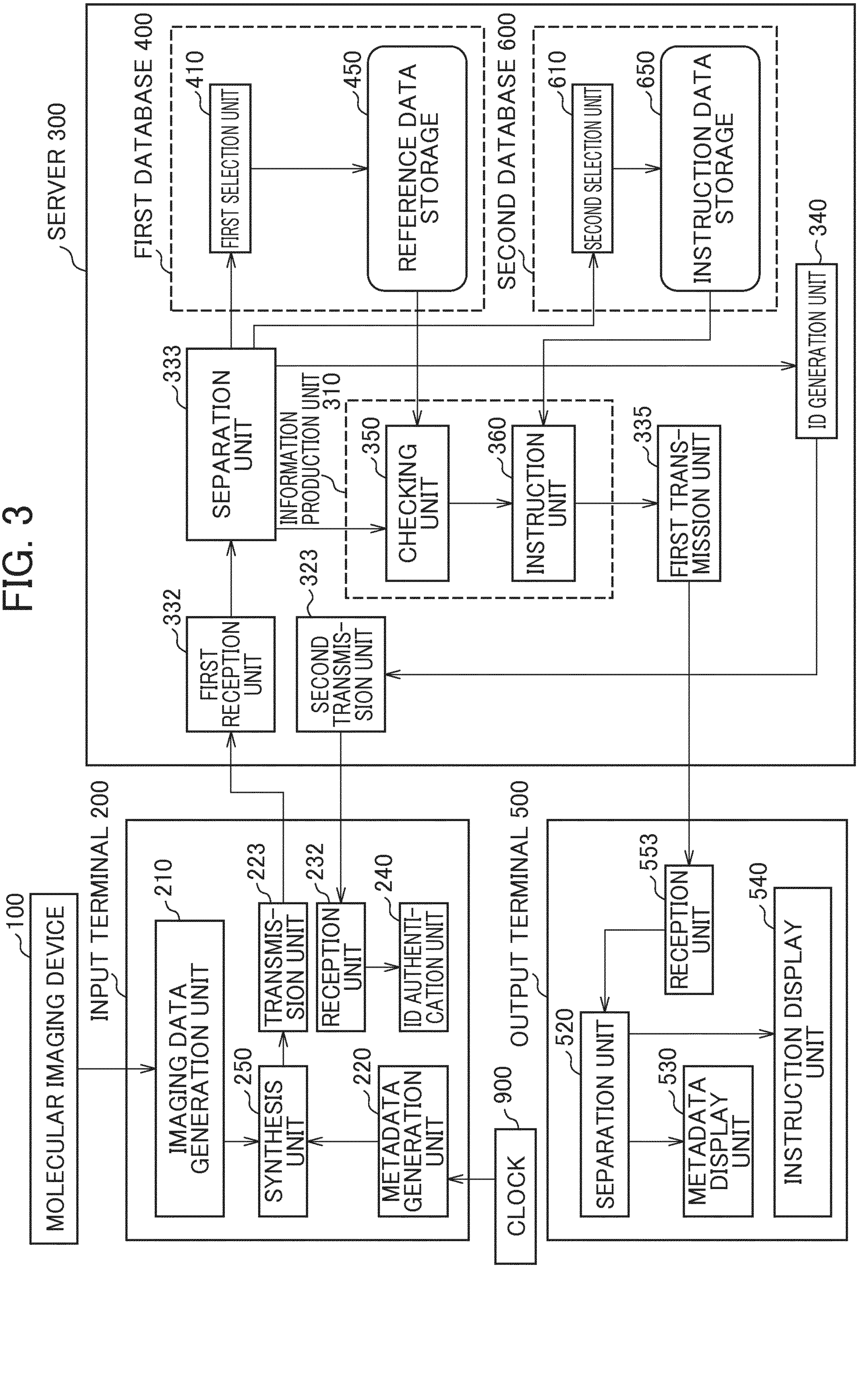
SERVER 300
FIRST DATABASE 400
410
FIRST SELECTION UNIT
450
REFERENCE DATA STORAGE
SECOND DATABASE 600
610
SECOND SELECTION UNIT
650
INSTRUCTION DATA STORAGE
340
ID GENERATION UNIT
333
SEPARATION UNIT
INFORMATION PRODUCTION UNIT 310
350
CHECKING UNIT
360
INSTRUCTION UNIT
335
FIRST TRANS-MISSION UNIT
332
FIRST RECEPTION UNIT
323
SECOND TRANSMIS-SION UNIT
100
MOLECULAR IMAGING DEVICE
INPUT TERMINAL 200
210
IMAGING DATA GENERATION UNIT
223
TRANSMIS-SION UNIT
232
RECEPTION UNIT
240
ID AUTHENTI-CATION UNIT
250
SYNTHESIS UNIT
220
METADATA GENERATION UNIT
900
CLOCK
OUTPUT TERMINAL 500
553
RECEPTION UNIT
540
INSTRUCTION DISPLAY UNIT
520
SEPARATION UNIT
530
METADATA DISPLAY UNIT

FIG. 4

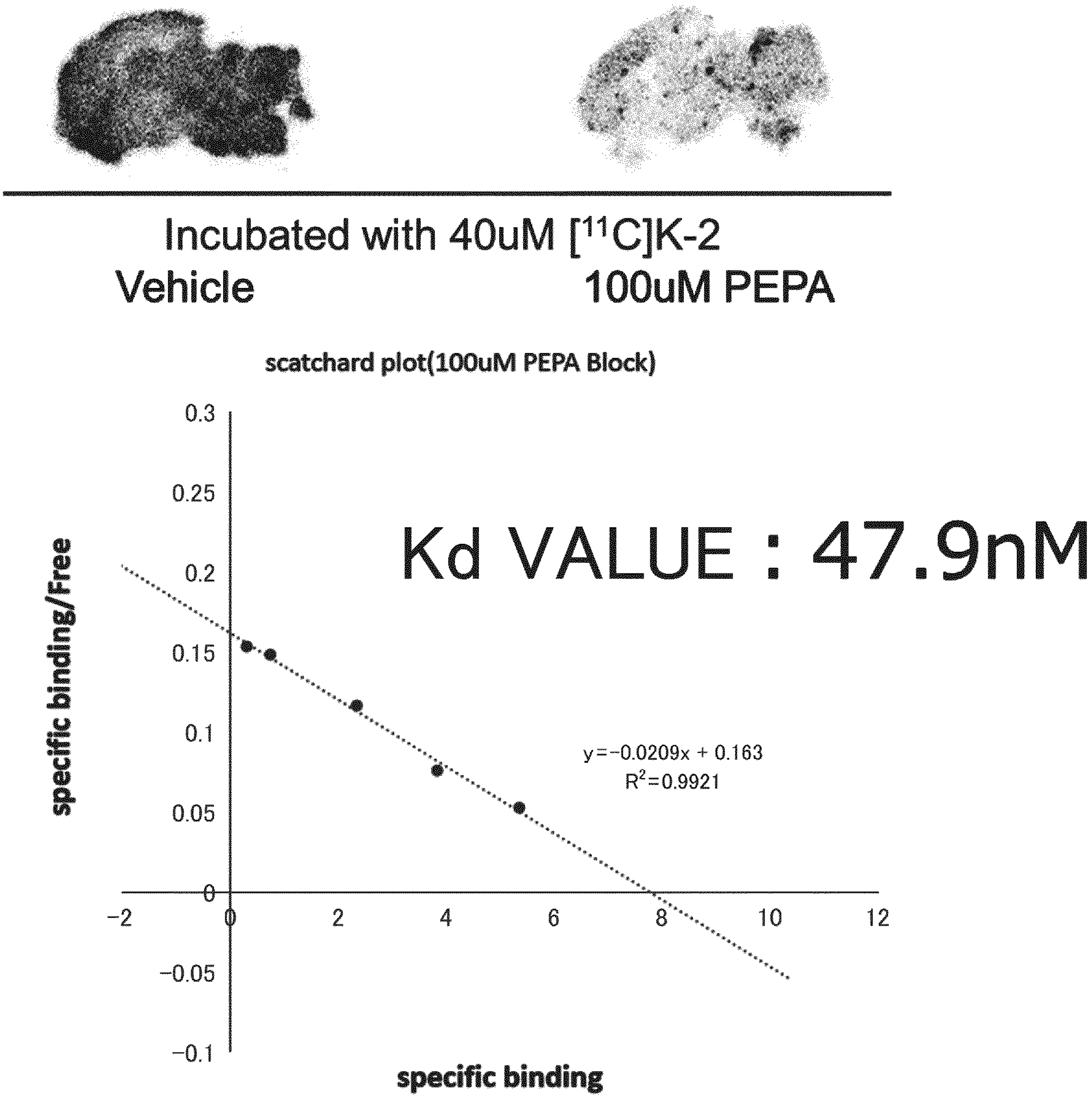
Incubated with 40uM [11C]K-2
Vehicle
100uM PEPA
scatchard plot(100uM PEPA Block)
Kd VALUE : 47.9nM
y=−0.0209x + 0.163
$R^2$=0.9921
specific binding/Free
specific binding
0.3
0.25
0.2
0.15
0.1
0.05
0
−0.05
−0.1
−2
0
2
4
6
8
10
12

# FIG. 5

K-2

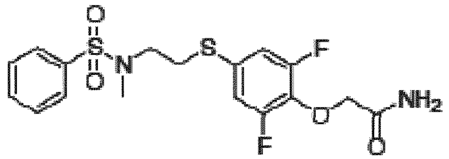

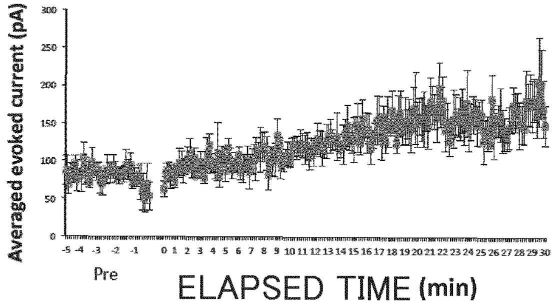
Averaged evoked current (pA)
Pre
ELAPSED TIME (min)

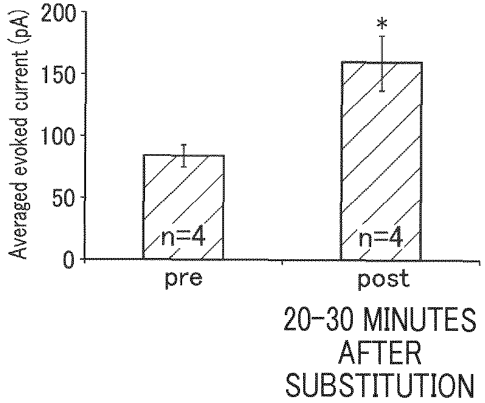
Averaged evoked current (pA)
n=4
n=4
pre
post
20-30 MINUTES AFTER SUBSTITUTION
* p = 0.0019
paired t-test

K-4

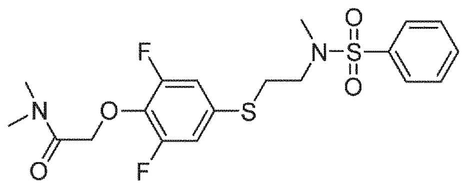

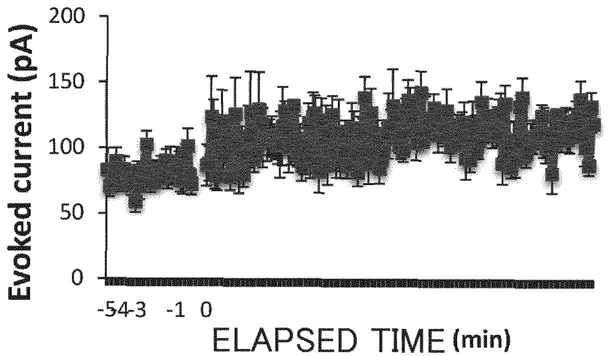
Evoked current (pA)
ELAPSED TIME (min)

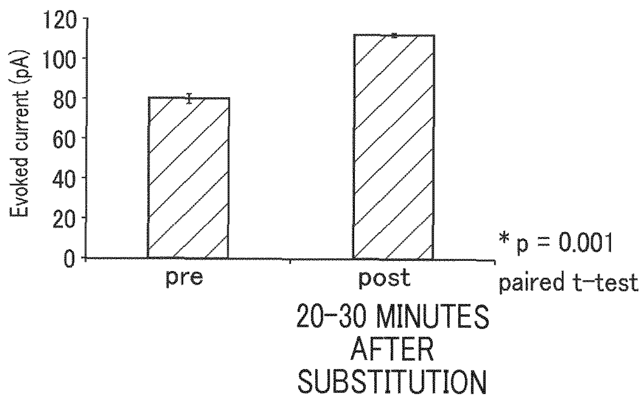
Evoked current (pA)
pre
post
20-30 MINUTES AFTER SUBSTITUTION
* p = 0.001
paired t-test

FIG. 6

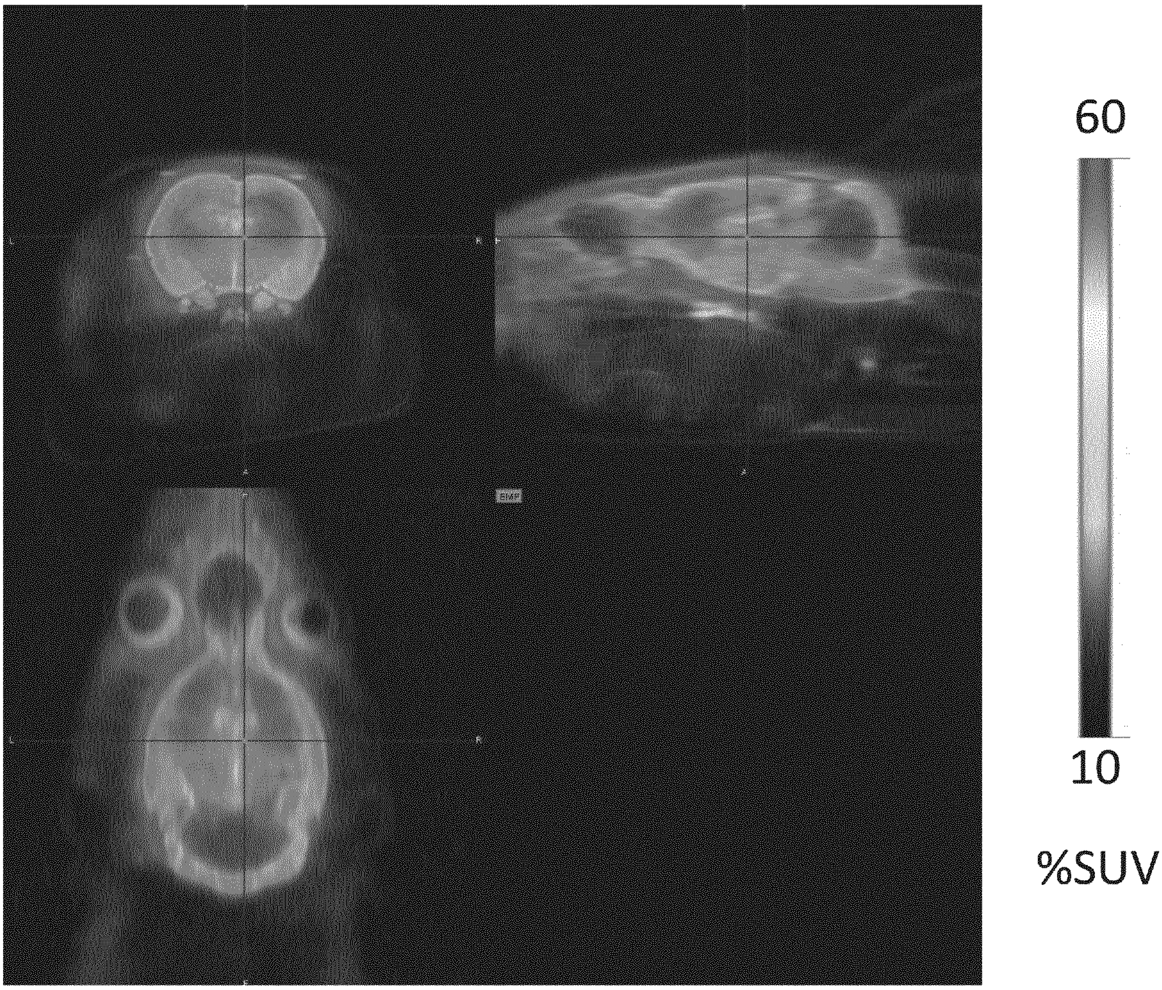

FIG. 7

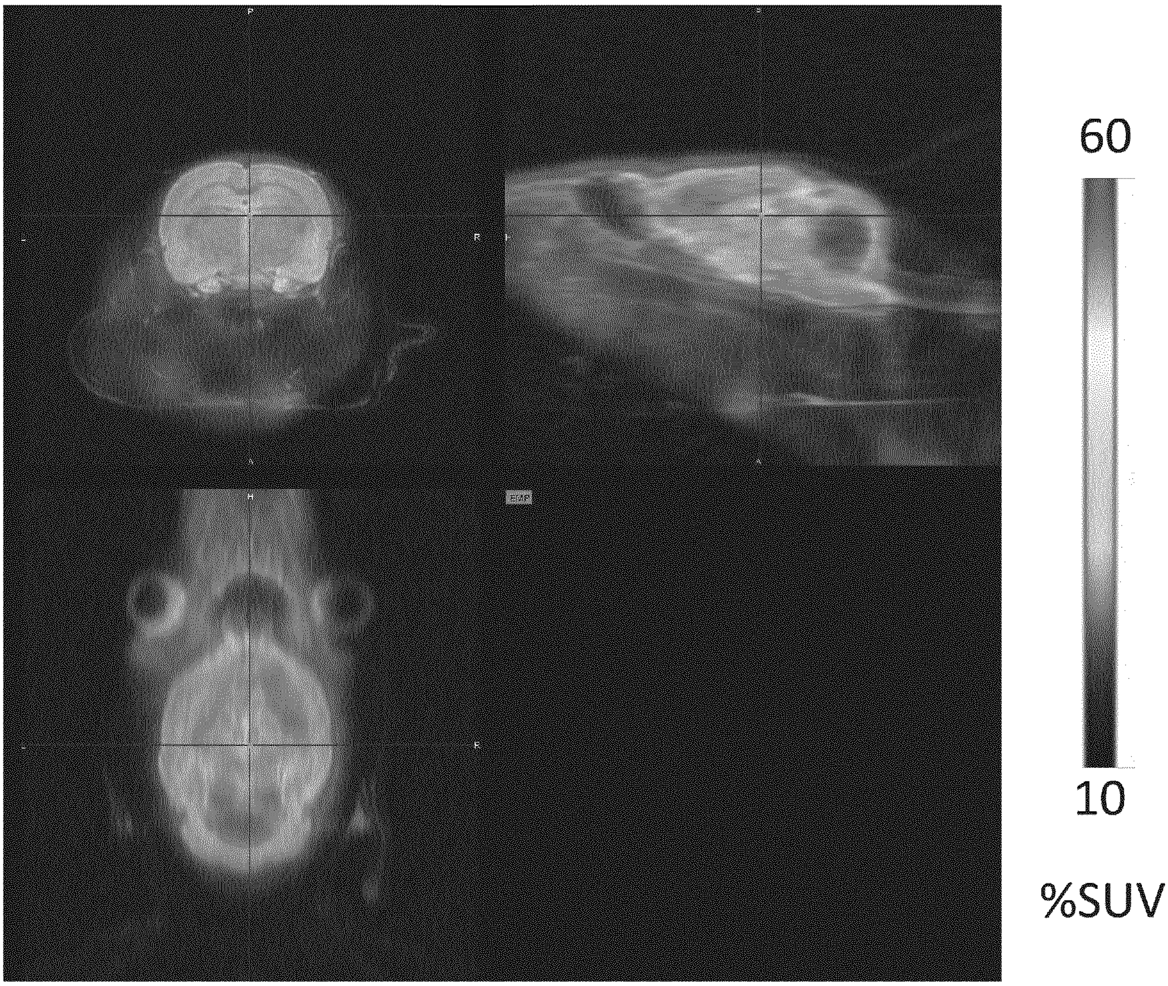

FIG. 8

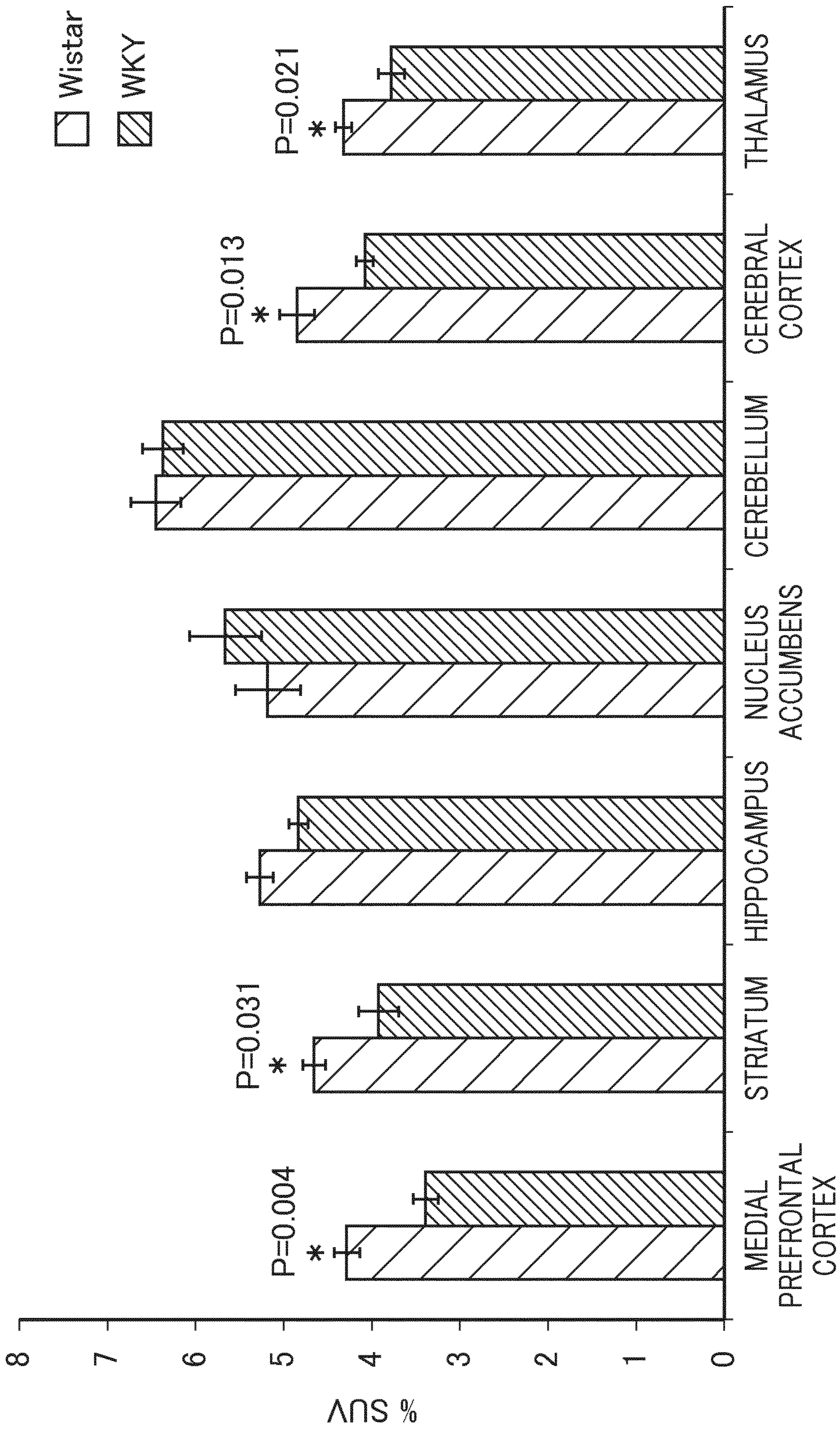
Wistar
WKY
% SUV
0
1
2
3
4
5
6
7
8
P=0.004
P=0.031
P=0.013
P=0.021
MEDIAL PREFRONTAL CORTEX
STRIATUM
HIPPOCAMPUS
NUCLEUS ACCUMBENS
CEREBELLUM
CEREBRAL CORTEX
THALAMUS

FIG. 9

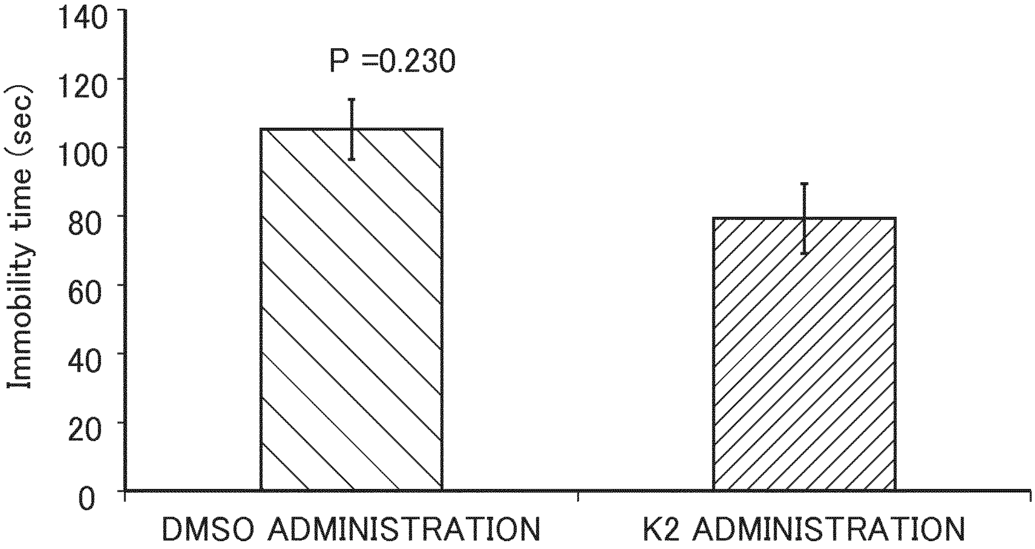
P =0.230
Immobility time (sec)
140
120
100
80
60
40
20
0
DMSO ADMINISTRATION
K2 ADMINISTRATION

FIG. 10

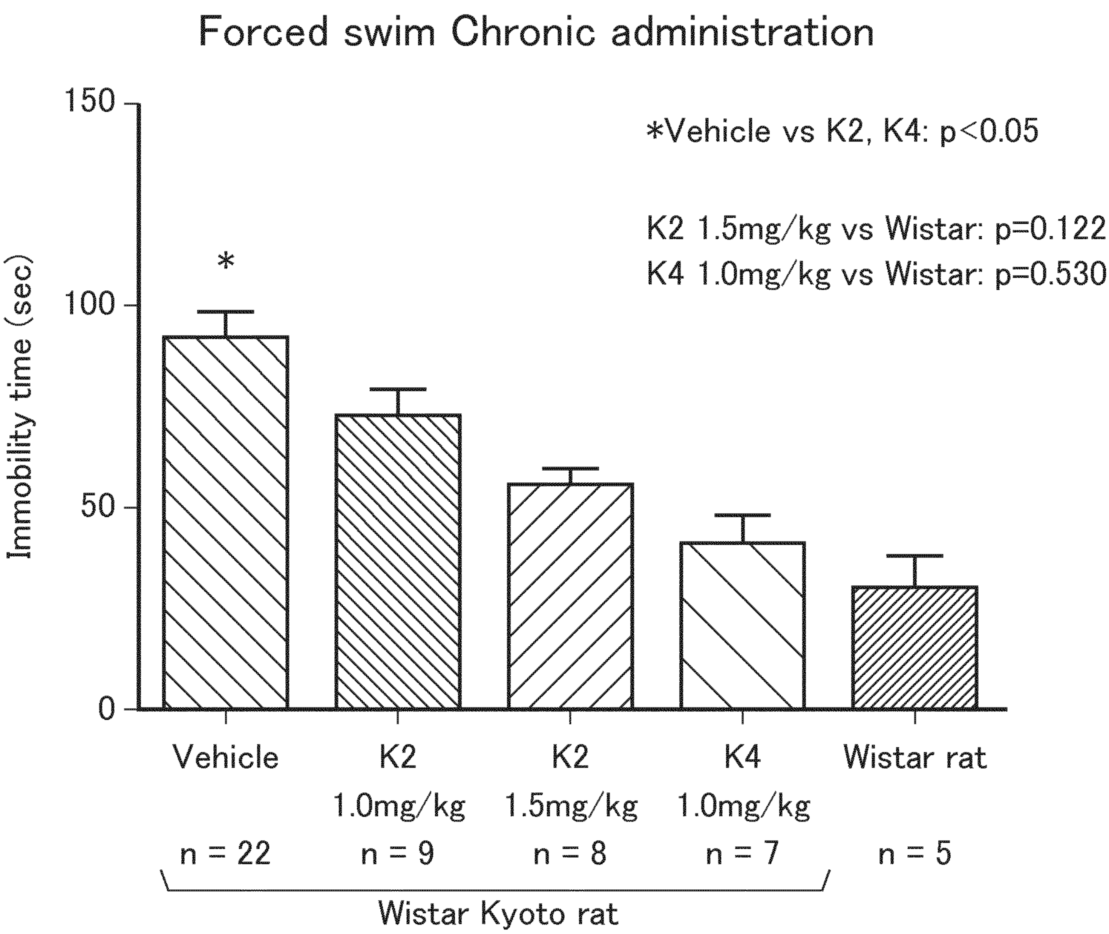
Forced swim Chronic administration
*Vehicle vs K2, K4: p<0.05
K2 1.5mg/kg vs Wistar: p=0.122
K4 1.0mg/kg vs Wistar: p=0.530
*
Immobility time (sec)
150
100
50
0
Vehicle
K2 1.0mg/kg
K2 1.5mg/kg
K4 1.0mg/kg
Wistar rat
n = 22
n = 9
n = 8
n = 7
n = 5
Wistar Kyoto rat

FIG. 11

AVERAGE IMAGES 0-30 MINUTES AFTER DRUG ADMINISTRATION

AVERAGE IMAGES 60-120 MINUTES AFTER DRUG ADMINISTRATION

HUMAN SUBJECT A
Hippo campus

HUMAN SUBJECT B
Hippo campus

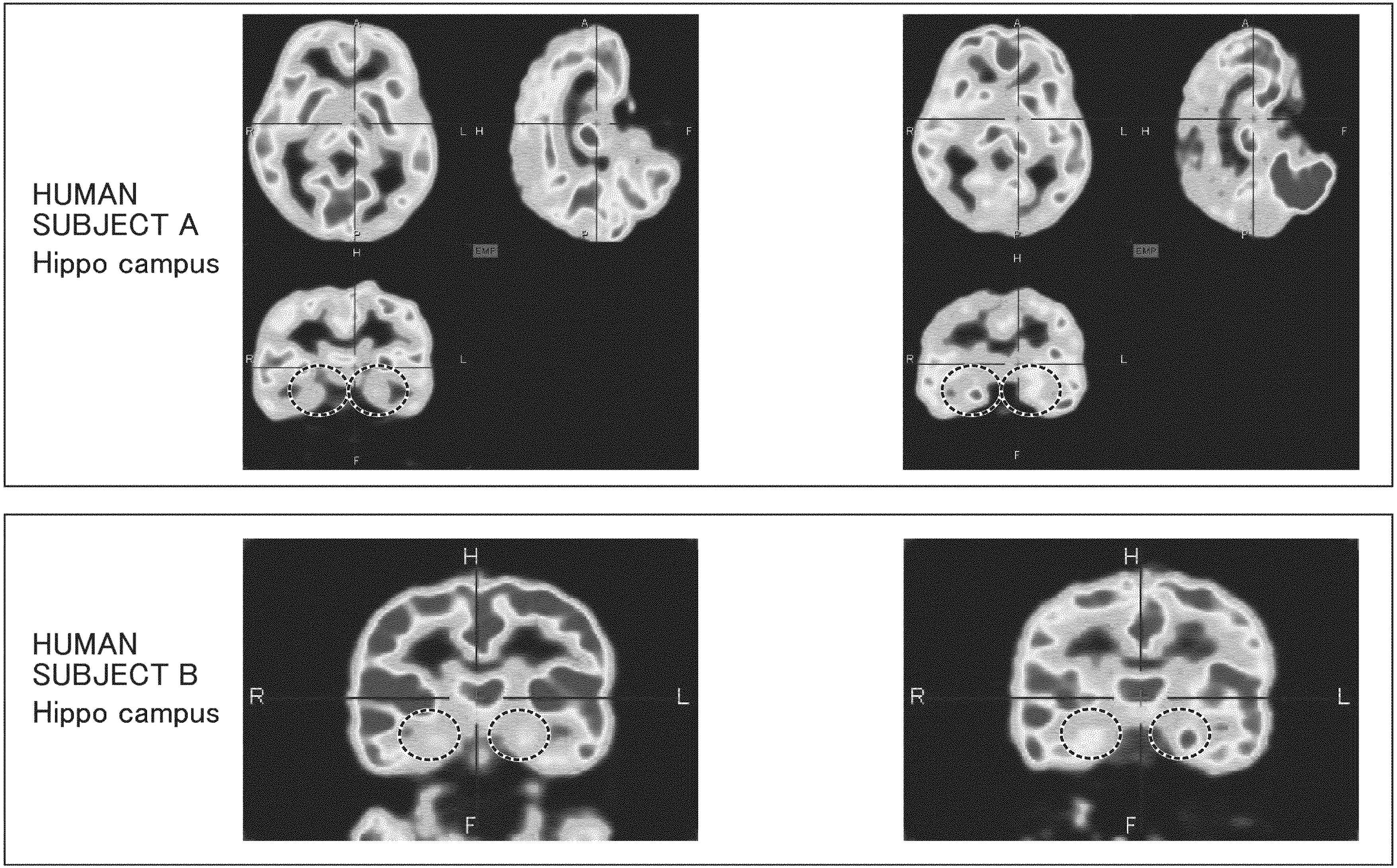

FIG. 12

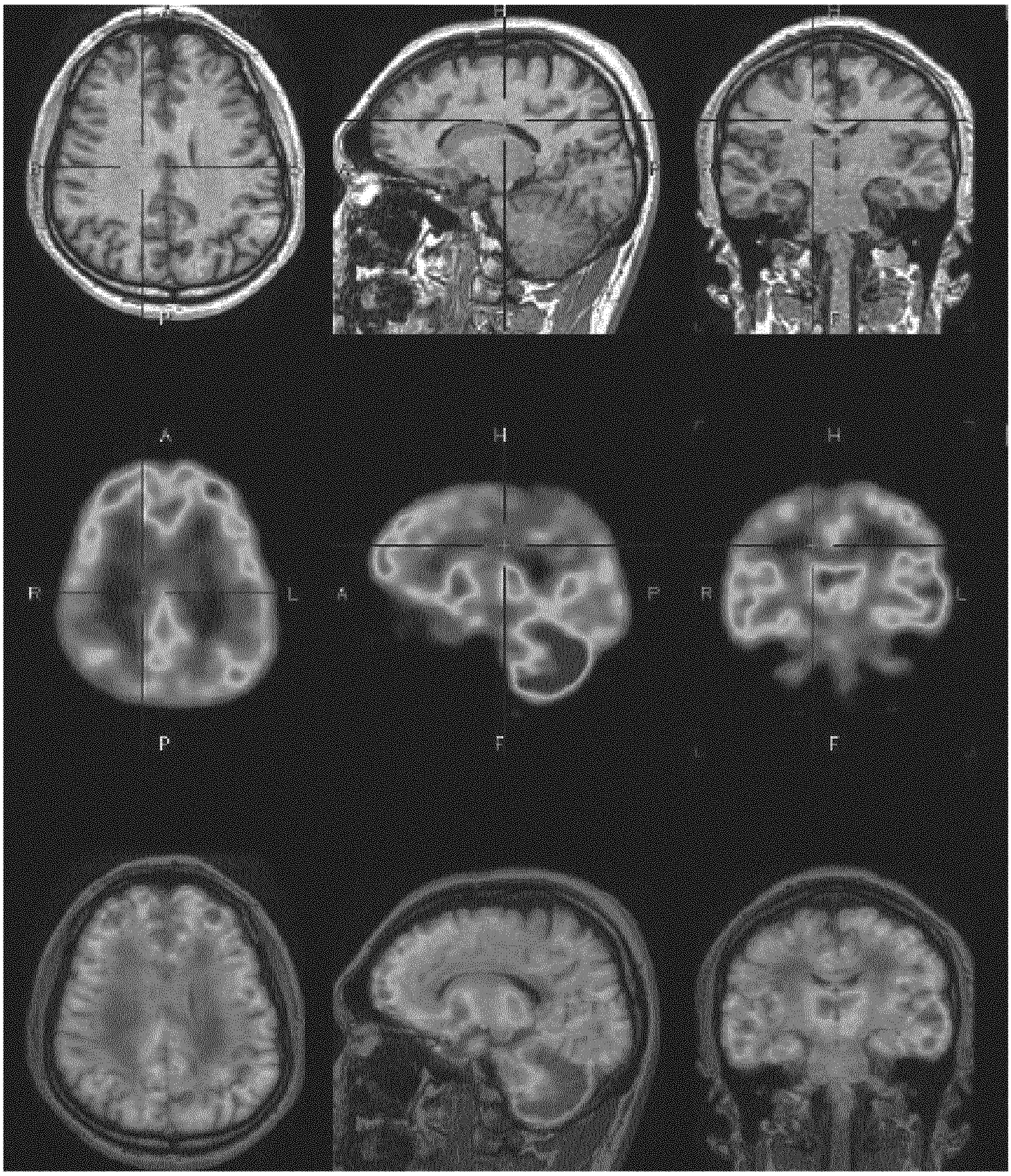

FIG. 13

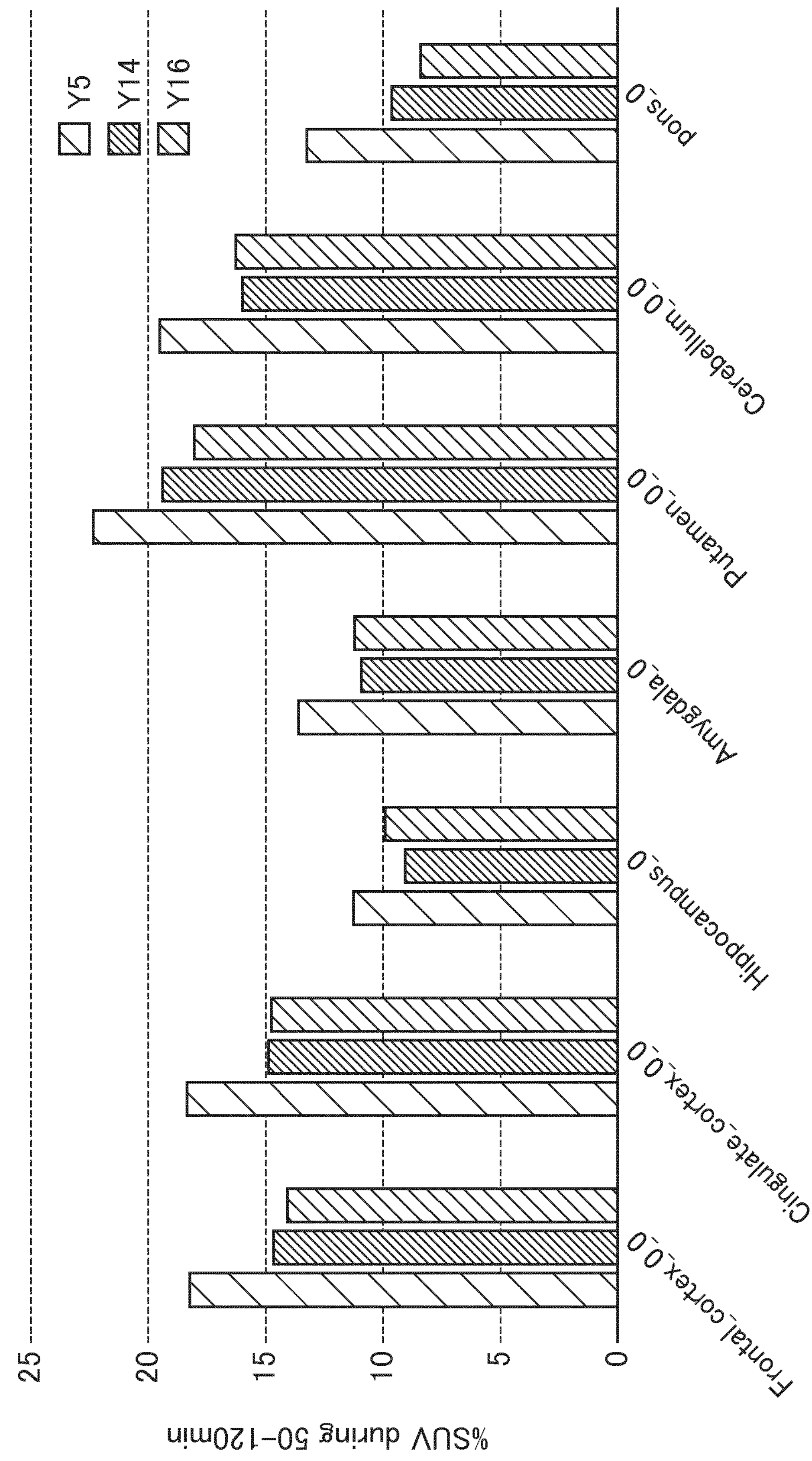

%SUV during 50−120min
0
5
10
15
20
25
Y5
Y14
Y16
Frontal_cortex_0_0
Cingulate_cortex_0_0
Hippocampus_0
Amygdala_0
Putamen_0_0
Cerebellum_0_0
pons_0

FIG. 14

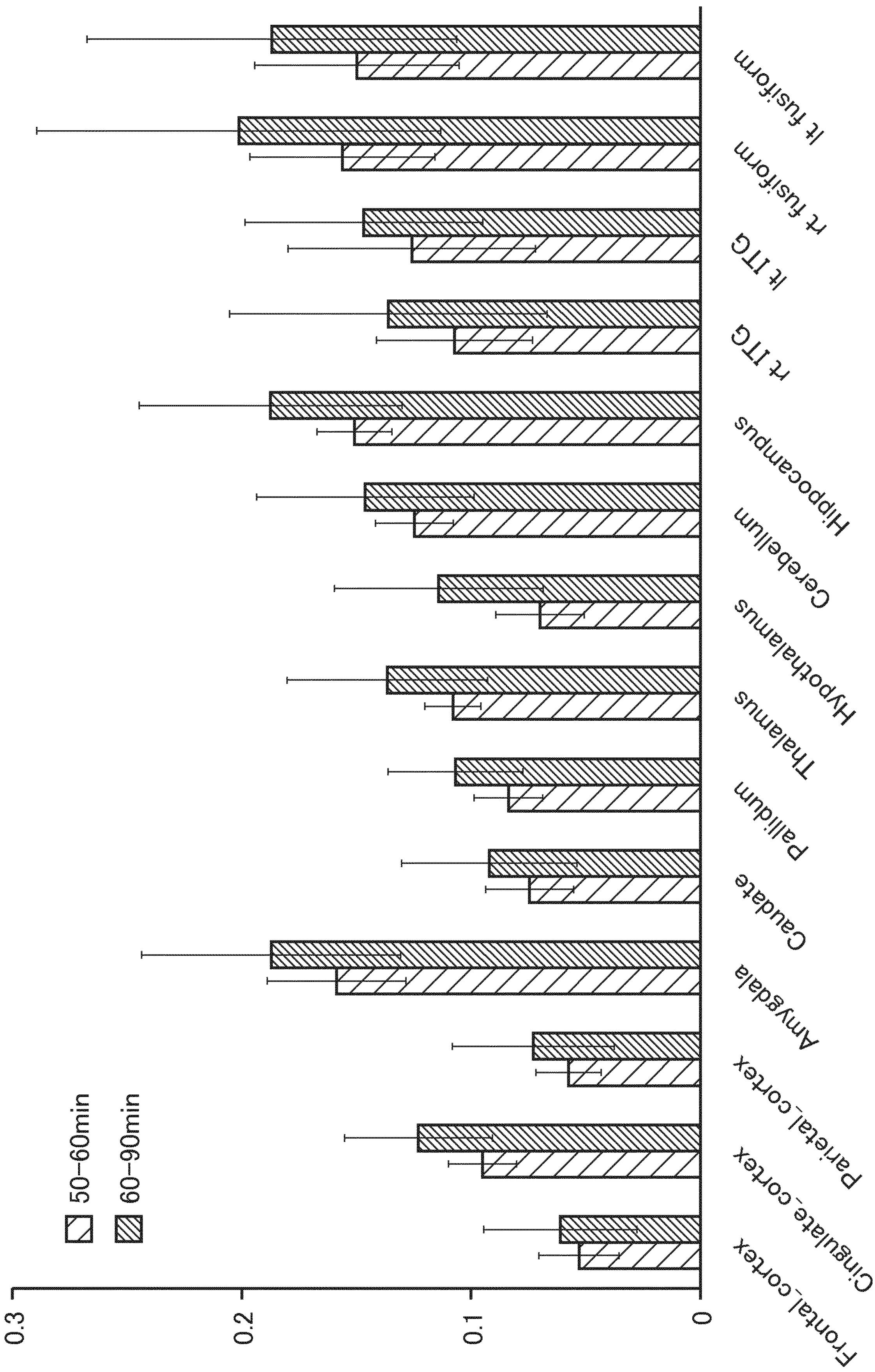
50–60min
60–90min
0.3
0.2
0.1
0
Frontal_cortex
Cingulate_cortex
Parietal_cortex
Amygdala
Caudate
Pallidum
Thalamus
Hypothalamus
Cerebellum
Hippocampus
rt ITG
lt ITG
rt fusiform
lt fusiform

FIG. 15

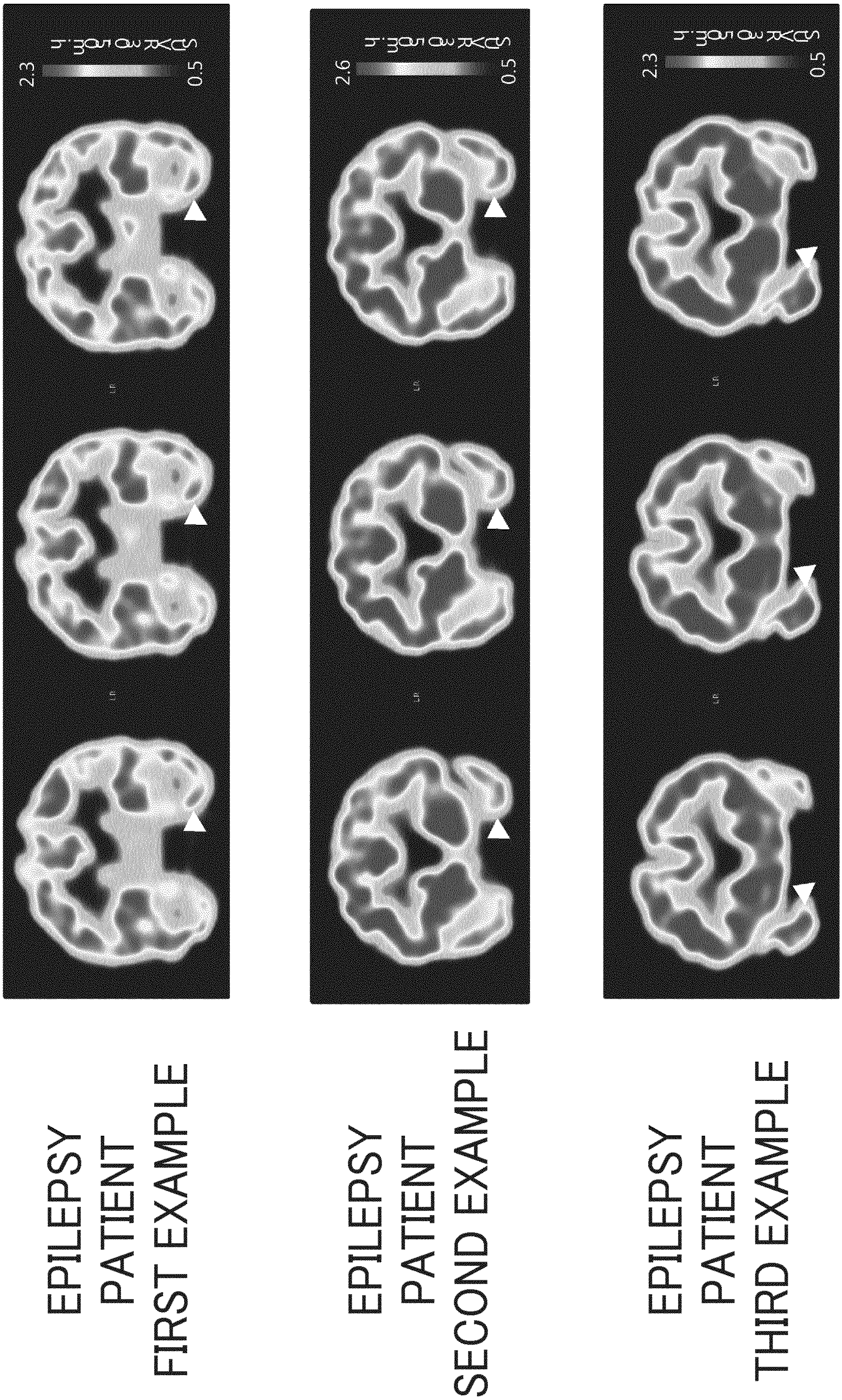
EPILEPSY PATIENT FIRST EXAMPLE
2.3
0.5
EPILEPSY PATIENT SECOND EXAMPLE
2.6
0.5
EPILEPSY PATIENT THIRD EXAMPLE
2.3
0.5

FIG. 16

EPILEPSY
PATIENT
FIRST EXAMPLE

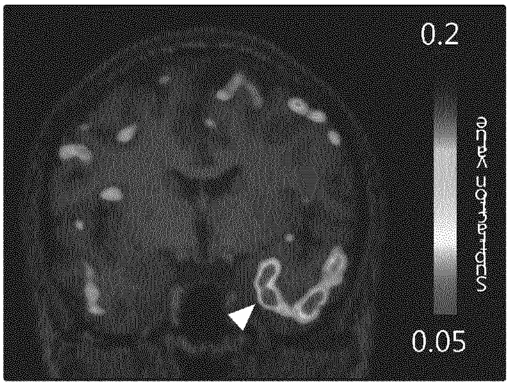


EPILEPSY
PATIENT
SECOND EXAMPLE

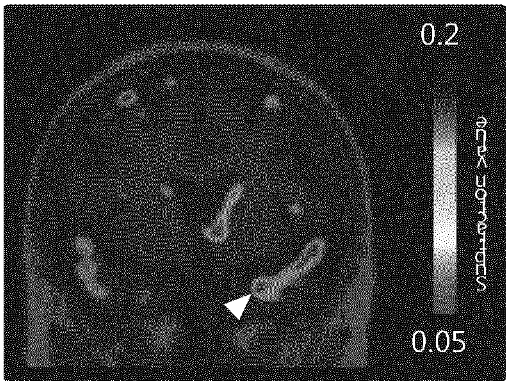


EPILEPSY
PATIENT
THIRD EXAMPLE

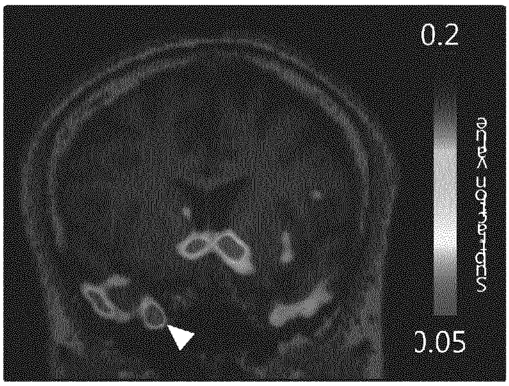

FIG. 17

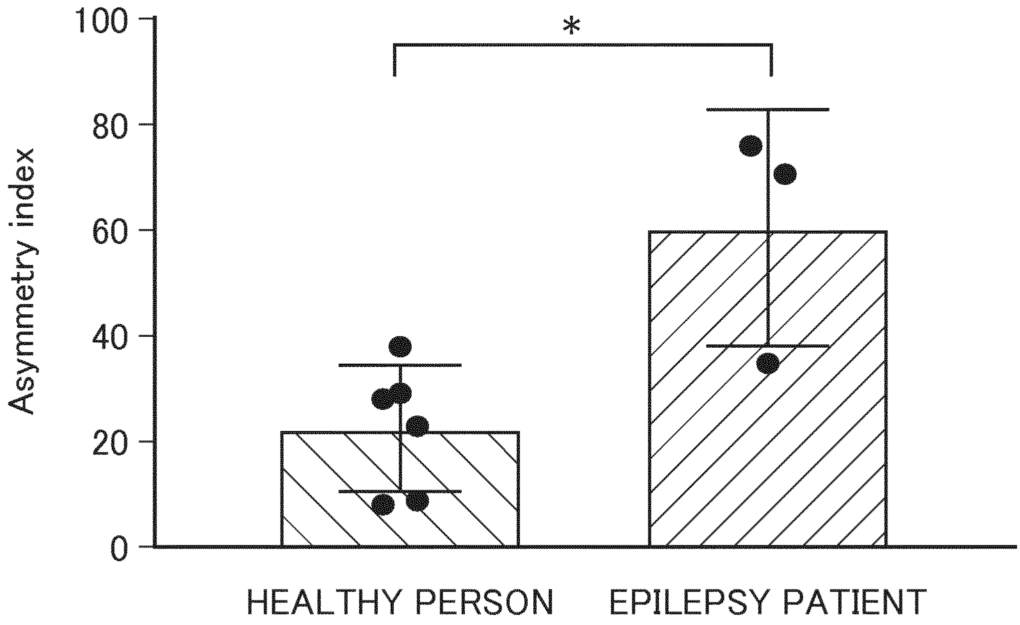
100
80
60
40
20
0
Asymmetry index
*
HEALTHY PERSON
EPILEPSY PATIENT

FIG. 18

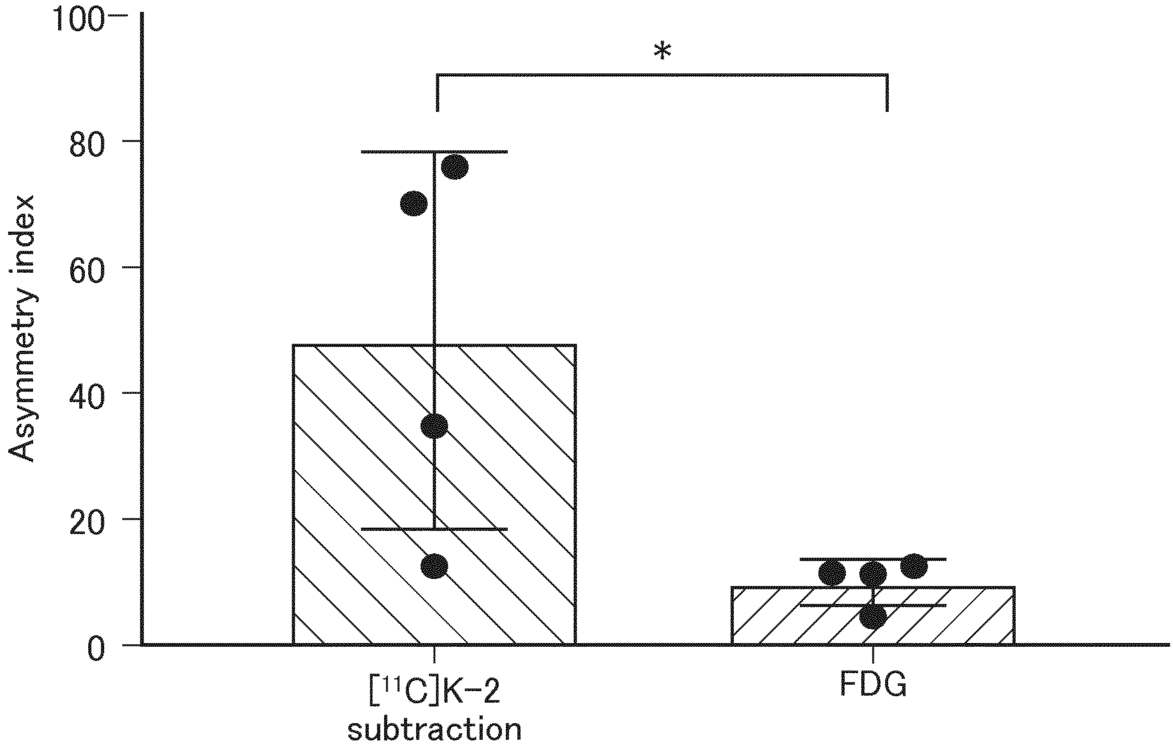
100
80
60
40
20
0
Asymmetry index
*
[11C]K-2
subtraction
FDG

FIG. 19

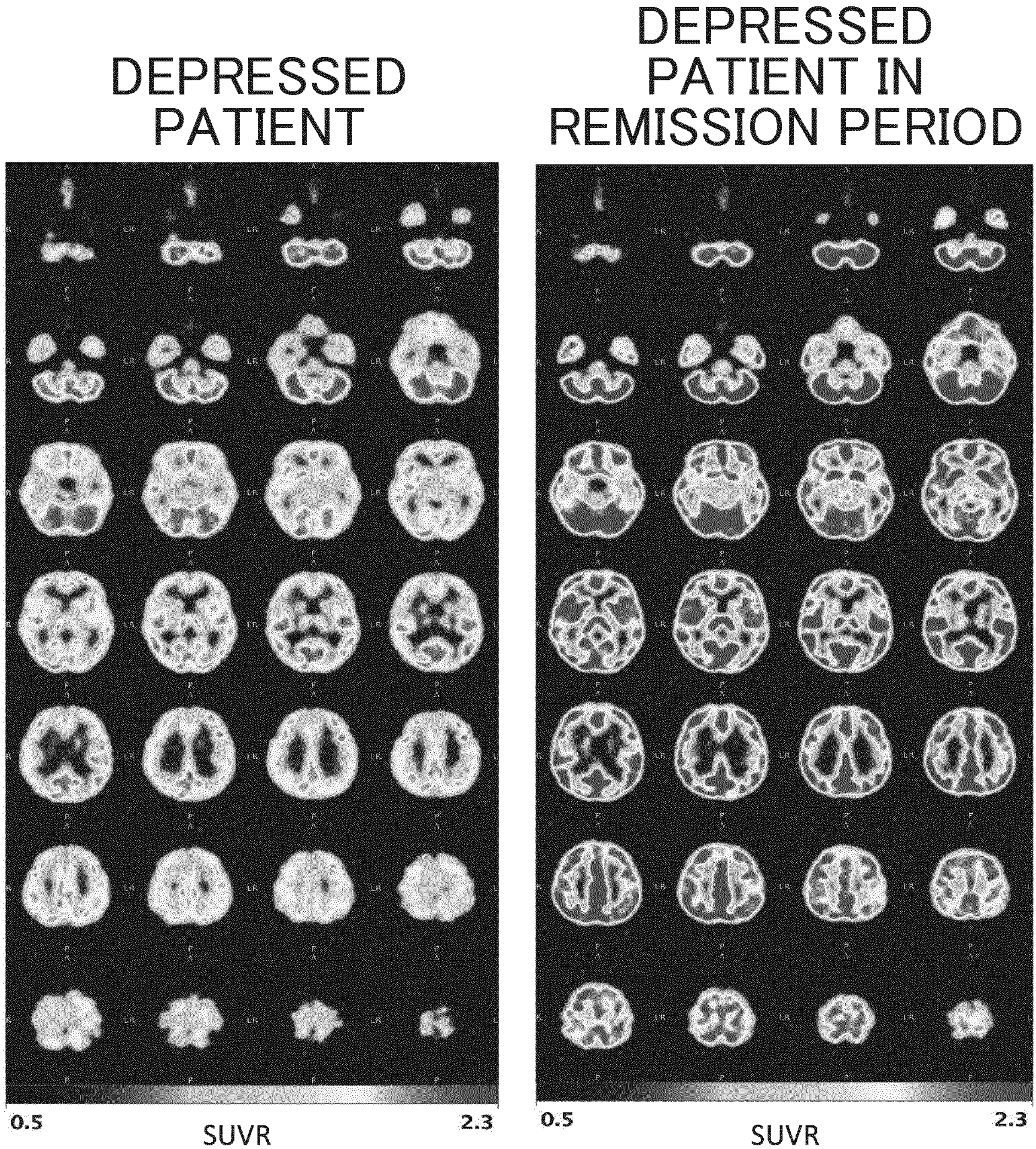
DEPRESSED PATIENT
DEPRESSED PATIENT IN REMISSION PERIOD
0.5
SUVR
2.3
0.5
SUVR
2.3

**INTERNATIONAL SEARCH REPORT**

International application No. PCT/JP2018/000532

A. CLASSIFICATION OF SUBJECT MATTER
Int.Cl. A61K49/00(2006.01)i

According to International Patent Classification (IPC) or to both national classification and IPC

B. FIELDS SEARCHED

Minimum documentation searched (classification system followed by classification symbols)
Int.Cl. A61K49/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | OI, N. et al., "Development of Novel PET Probes for Central 2-Amino-3-(3-hydroxy-5-methyl-4-isoxazolyl) propionic Acid Receptors", Journal of Medicinal Chemistry, 2015, vol. 58, pp. 8444-8462, ISSN 0022-2623, in particular, abstract, tables 1, 2, fig. 1-6, page 8444, right column, paragraph [0002] to page 8445, left column, paragraph [0001] | 1-3, 5-9<br>4, 10-13 |
| X<br>Y | JP 2016-522786 A (EISAI R&D MANAGEMENT CO., LTD.) 04 August 2016, claims 1-15, test examples 1-6, fig. 1-4 & US 2016/0045625 A1, claims 1-11, paragraph [0006], test examples 1-6, fig. 1-4 & WO 2014/163210 A11 & EP 2981531 A1 | 1-3, 5-9<br>4, 10-13 |

☒ Further documents are listed in the continuation of Box C.　　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 March 2018 (14.03.2018) | 27 March 2018 (27.03.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2018/000532

C (Continuation). DOCUMENTS CONSIDERED TO BE RELEVANT

| Category* | Citation of document, with indication, where appropriate, of the relevant passages | Relevant to claim No. |
|---|---|---|
| Y | WO 1996/25926 A1 (NIPPON SUISAN KAISHA, LTD.) 29 August 1996, preparation example 15, examples, table 1 & US 5955505 A, examples 15, 16, table 1 & EP 811375 A1 | 4-9 |
| Y | AHMED, A. H. et al., "Molecular Mechanism of Flop Selectivity and Subsite Recognition for an AMPA Receptor Allosteric Modulator: Structures of GluA2 and GluA3 in Complexes with PEPA", BIOCHEMISTRY, 2010, vol. 49, no. 13, pp. 2843-2850, ISSN 0006-2960, in particular, abstract, fig. 4 | 4-9 |
| Y | WO 2011/002096 A1 (RIKEN, JAPAN) 06 January 2011, claims 1-11, examples & US 2012/0230914 A1, claims 1-13, examples & EP 2450354 A1 | 4-9 |
| Y | WO 2015/066456 A1 (BRISTOL-MYERS SQUIBB COMPANY) 07 May 2015, claims 1-14, examples 1-10 & US 2016/0256577 A1 & EP 3062827 A1 | 4-9 |
| X | JP 2003-159220 A (THE NEW INDUSTRY RESEARCH ORGANIZATION) 03 June 2003, paragraph [0014], fig. 1 (Family: none) | 10 |
| Y | COSTES, N. et al., "A 18F-MPPF PET Normative Database of 5-HT1A Receptor Binding in Men and Women Over Aging", The Journal of Nuclear Medicine, 2005, vol. 46, no. 12, pp. 1980-1989, ISSN 2159-662X, in particular, page 1981, left column, paragraph [0002] | 10-13 |
| Y | JP 2011-520195 A (KONINKLIJKE PHILIPS ELECTRONICS N. V.) 14 July 2011, claims 1-22, paragraphs [0027]-[0031] & US 2011/0046979 A1, claims 1-22, paragraphs [0029]-[0033] & WO 2009/136354 A1 & EP 2283442 A1 & CN 102016859 A | 10-13 |
| P, X | WO 2017/006931 A1 (YOKOHAMA CITY UNIVERSITY) 12 January 2017, claims 1-31, examples 1-6 & TW 201706242 A | 1-9 |

Form PCT/ISA/210 (continuation of second sheet) (January 2015)

## INTERNATIONAL SEARCH REPORT

International application No.
PCT/JP2018/000532

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☒ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (January 2015)

**INTERNATIONAL SEARCH REPORT**

International application No. PCT/JP2018/000532

(Invention 1) Claims 1-9

Documents 1 and 2 indicate that a substance, which is selectively coupled to an intracranial AMPA receptor and radiolabeled, is injected, and then PET scanning is performed for 50 minutes to image the intracranial AMPA receptor. Claims 1-3 lack novelty in light of documents 1 and 2, and thus do not have a special technical feature. However, claim 4, which is dependent on claim 1, has the special technical feature of "a method for imaging an intracranial AMPA receptor of a primate organism, wherein a compound of formula (I) or a pharmaceutically acceptable salt or solvate thereof is migrated to the inside of the brain", and claim 5 also has the same special technical feature as claim 4. Thus, claims 1-5 are classified as invention 1.

In addition, claims 6-9 are substantially identical or equivalent to claims 1-3, and thus are classified as invention 1.

Document 1: OI, N. et al., Development of Novel PET Probes for Central 2-Amino-3-(3-hydroxy-5-methyl-4-isoxazolyl)propionic Acid Receptors, Journal of Medicinal Chemistry, 2015, Vol. 58, pages 8444-8462, ISSN 0022-2623, particularly abstract, table 1, 2, figures 1-6

Document 2: JP 2016-522786 A (EISAI R&D MANAGEMENT CO., LTD.) 04 August 2016, claims 1-15, test examples 1-6, figures 1-4 & US 2016/0045625 A1, claims 1-11, test examples 1-6, figures 1-4 & WO 2014/163210 A1 & EP 2981531 A1

(Invention 2) claim 10

Claim 10 cannot be said to have a special technical feature identical or corresponding to claim 4.

In addition, claim 10 is not dependent on claim 4 and is not substantially identical or equivalent to any of the claims classified as invention 1.

Thus, claim 10 cannot be classified as invention 1.

In addition, claim 10 has the special technical feature of an "input terminal transmitting, to a server, information about the distribution and/or expression level of an intracranial AMPA receptor of a primate organism", and thus is classified as invention 2.

(Invention 3) Claims 11-13

Claim 11 cannot be said to have a special technical feature identical or corresponding to claim 10 classified as invention 2.

In addition, claim 11 is not dependent on claim 4 or 10, and is not substantially identical or equivalent to any of the claims classified as invention 1 or 2.

Thus, claims 11 and 12 cannot be classified as invention 1 or 2.

In addition, claim 11 has the special technical feature of a "server provided with a database storing data associated with the distribution and/or expression level of an intracranial AMPA receptor of a primate organism, ···, and a means for transmitting, to an output terminal, information about the distribution and/or expression level of an intracranial AMPA receptor of a primate organism, and information about ···, and thus is classified as invention 3.

In addition, claims 12 and 13 are substantially identical or equivalent to claim 11, and thus are classified as invention 3.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012207021 A
- JP 2010202525 A
- JP 2006525292 W
- JP 2016522786 PCT

### Non-patent literature cited in the description


- **JINGLI ZHANG et al.** *Rev. Neurosci.,* 2013, vol. 24 (5), 499-505
- **SIMON E WARD et al.** *British Journal of Pharmacology,* 2010, vol. 160, 181-190